# EUROPEAN PATENT APPLICATION

(11) **EP 4 186 527 A1**
(43) Date of publication of application: **31.05.2023**
(21) Application number: 21825999.2
(22) Date of filing: 18.06.2021
(51) Int. Cl.: A61K 45/06, A61K 38/17, A61K 39/395, A61P 35/00, A61P 37/02, A61P 43/00, C07K 14/705, C07K 16/22, C07K 19/00, C12N 9/99

(54) **ANTI-T CELL ANTIGEN-BINDING MOLECULE FOR USE IN COMBINATION WITH ANGIOGENESIS INHIBITOR**

(30) Priority: 19.06.2020 JP 2020106501
(71) Applicant: Chugai Seiyaku Kabushiki Kaisha, Kita-ku Tokyo 115-8543 (JP)
(72) Inventor: TANAKA, Takayoshi, Tokyo 103-8324 (JP); SANO, Yuji, Kamakura-shi, Kanagawa 247-8530 (JP); KAWAI, Yumiko, Kamakura-shi, Kanagawa 247-8530 (JP)
(74) Representative: Vossius & Partner Patentanwälte Rechtsanwälte mbB
(86) International application number: PCT/JP2021/023149
(87) International publication number: WO 2021/256555

(57) **Abstract**

The present disclosure provides methods for preventing, alleviating, or treating cytokine release resulting from administration of a VEGF inhibitor or side effects resulting from the cytokine release. To prevent, alleviate, or treat cytokine release or its side effects, the disclosure also provides combination therapies that use a lymphocyte-stimulating pharmaceutical agent, represented by an anti-T cell antigen-binding molecule, with a VEGF inhibitor. Among the anti-T cell antigen-binding molecules, for example, antibodies that recruit T cells as effector cells into tumor tissues are called T cell redirecting antibodies, and are known as means for treating tumors. On the other hand, when systemic cytokine production is stimulated by binding of antibodies to T cells, it is feared that this systemic action will lead to aberrations such as CRS. The present disclosure provides means for alleviating systemic cytokine production, and will enable safer use of anti-T cell antigen-binding molecules in tumor treatment.

## Description

### [Technical Field]

The present disclosure relates to anti-T cell antigen-binding molecules for use in combination with angiogenesis inhibitors. Furthermore, the present disclosure relates to pharmaceutical compositions and methods for preventing, alleviating, or treating adverse reactions caused by cytokine production associated with administration of anti-T cell antigen-binding molecules.

### [Background Art]

Antibodies are drawing attention as pharmaceuticals because of their high stability in plasma and few adverse reactions (NPL 1 and NPL 2). Antibodies are known to induce not only an antigen-binding action, an agonistic action, and an antagonistic action, but also effector cell-mediated cytotoxic activities (also called effector functions) such as antibody-dependent cytotoxicity (ADCC), antibody dependent cell phagocytosis (ADCP), and complement-dependent cytotoxicity (CDC), and exhibit antitumor effects against cancer cells (NPL 3). A number of therapeutic antibodies showing excellent anti-tumor effects have been developed as pharmaceuticals aimed for cancer treatment (NPL 4); and while existing therapeutic antibodies have shown excellent actions, the therapeutic outcome achieved by administration of these antibodies is still not satisfactory.

Antibodies (bispecific antibodies) that bind to two or more types of antigens with one molecule are being studied as molecules that inhibit multiple targets. If all antigens recognized by a bispecific antibody are antigens specifically expressed in cancer, the bispecific antibody exhibits cytotoxic activity to cancer cells when it binds to any of the antigens; therefore, in comparison to a conventional antibody pharmaceutical that recognizes one antigen, a more efficient antitumor effect can be expected from such an antibody.

As one of the bispecific antibodies, a T cell-redirecting antibody, which is an antibody whose mechanisms of antitumor effect is cytotoxicity mediated by the recruitment of T cells as effector cells, has been known from the 1980s (NPLs 5, 6, and 7). Unlike antibodies that employ ADCC, which is mediated by the recruitment of NK cells or macrophages as effector cells, as the mechanism for their antitumor effects, a T cell-redirecting antibody is a bispecific antibody comprising an antibody against any one of the subunits constituting the T-cell receptor (TCR) complex on T cells, in particular, an antibody that binds to the CD3 epsilon chain; and an antibody that binds to an antigen on the target cancer cell. T cells come close to cancer cells via simultaneous binding of the T cell-redirecting antibody to the CD3 epsilon chain and a cancer antigen. As a result, antitumor effects against cancer cells are considered to be exerted through the cytotoxic activity possessed by T cells. For example, in recent years, novel T cell-redirecting antibodies have been provided, which are antibodies whose two Fabs respectively bind to a cancer antigen (GPC3) and the CD3ε chain expressed on T cells, and have an Fc region with reduced FcyR-binding activity (PTL 1 and 2).

On the other hand, occurrence of toxicity associated with exertion of high antitumor activity by the T cell-redirecting antibody, for example, development of cytokine release syndrome (CRS), has been reported (NPL 8).

### [Citation List]

### [Patent Literature]

[PTL 1] WO2012/073985 (TRAB concept patent)
[PTL 2] WO2016/047722 (ERY974 product patent)

### [Non-Patent Literature]

[NPL 1] Nat. Biotechnol. (2005) 23, 1073-1078
[NPL 2] Eur J Pharm Biopharm. (2005) 59 (3), 389-396
[NPL 3] Drug Des Devel Ther (2009) 3, 7-16
[NPL 4] Clin Cancer Res. (2010) 16 (1), 11-20
[NPL 5] Nature (1985) 314 (6012), 628-31
[NPL 6] Int J Cancer (1988) 41 (4), 609-15.
[NPL 7] Proc Natl Acad Sci USA (1986) 83 (5), 1453-7
[NPL 8] Cancer J. 2014 Mar-Apr; 20(2): 119-122.

### [Summary of Invention]

### [Technical Problem]

In a non-limiting embodiment, the present disclosure relates to combination therapies comprising an anti-T cell antigen-binding molecule and an angiogenesis inhibitor, as well as pharmaceutical compositions and such for use in the combination therapies.

### [Solution to Problem]

In a non-limiting embodiment, the present inventors found that administration of a vascular epithelial cell growth factor (Vascular Endothelial Growth Factor (VEGF)) inhibitor, which is a representative angiogenesis inhibitor, can prevent, alleviate, or treat cytokine release syndrome (CRS) associated with administration of an anti-T cell antigen-binding molecule in an individual.

In a non-limiting embodiment, the present disclosure relates to the following:
(A1) a pharmaceutical composition comprising an anti-T cell antigen-binding molecule, wherein its use in combination with a vascular epithelial cell growth factor (VEGF) inhibitor prevents and/or alleviates and/or treats cytokine release syndrome and/or cytokine release;
(A1-1) the pharmaceutical composition of (A1), wherein the VEGF inhibitor is administered before, simultaneously with, or after administration of the pharmaceutical composition;
(A1-2) the pharmaceutical composition of (A1), wherein the VEGF inhibitor is administered before or simultaneously with administration of the pharmaceutical composition;
(A1-3) the pharmaceutical composition of any one of (A1) to (A1-2), wherein the VEGF inhibitor is administered 6 days, 5 days, 4 days, 3 days, 2 days, or 1 day before the administration of the pharmaceutical composition, or on the same day as but before administration of the pharmaceutical composition;
(A1-4) the pharmaceutical composition of any one of (A1) to (A1-3), wherein the VEGF inhibitor is one or more VEGF inhibitors selected from an anti-VEGF antigen-binding molecule,
an anti-VEGFR1 antigen-binding molecule, an anti-VEGFR2 antigen-binding molecule, a fusion protein comprising a VEGF receptor or a fragment thereof, and a tyrosine kinase inhibitor;
(A1-5) the pharmaceutical composition of any one of (A1) to (A1-4), wherein the VEGF inhibitor is one or more VEGF inhibitors selected from Bevacizumab, Ramucirumab, and Aflibercept;
(A1-6) the pharmaceutical composition of any one of (A1) to (A1-5), which is further additionally for use in combination with an immune checkpoint inhibitor;
(A1-7) the pharmaceutical composition of (A1-6), wherein the immune checkpoint inhibitor is selected from an anti-PD1 antibody, an anti-PD-L1 antibody, and an anti-PD-L2 antibody;
(A1-8) the pharmaceutical composition of (A1-6) or (A1-7), wherein the immune checkpoint inhibitor is Atezolizumab;
(A1-9) the pharmaceutical composition of any one of (A1) to (A1-8), wherein a corticosteroid is not administered before or simultaneously with the administration of the pharmaceutical composition;
(A1-10) the pharmaceutical composition of any one of (A1) to (A1-8), wherein a corticosteroid is further administered before, simultaneously with, or after the administration of the pharmaceutical composition;
(A1-11) the pharmaceutical composition of (A1-9) or (A1-10), wherein the corticosteroid is dexamethasone, a pharmaceutically acceptable salt thereof, or a derivative thereof;
(A1-12) the pharmaceutical composition of any one of (A1) to (A1-11), wherein the anti-T cell antigen-binding molecule is a bispecific antigen-binding molecule comprising:
   (1) a domain comprising an antibody variable region having T cell receptor complex-binding activity; and
   (2) a domain comprising an antibody variable region having cancer antigen-binding activity;
(A1-13) the pharmaceutical composition of any one of (A1) to (A1-12), wherein the anti-T cell antigen-binding molecule is a bispecific antibody comprising:
   (1) a domain comprising an antibody variable region having T cell receptor complex-binding activity;
   (2) a domain comprising an antibody variable region having glypican 3-binding activity; and
   (3) a domain comprising an Fc region with reduced binding activity towards an Fcγ receptor;
(A1-14) the pharmaceutical composition of any one of (A1) to (A1-13), which is for treatment of cancer;
(A1-15) the pharmaceutical composition of any one of (A1) to (A1-14), which is for treating cancer while preventing or alleviating cytokine release syndrome and/or cytokine release;
(A1-16) the pharmaceutical composition of any one of (A1) to (A1-15), wherein the cytokine release syndrome and/or cytokine release are associated with administration of the anti-T cell antigen-binding molecule; and
(A1-17) the pharmaceutical composition of any one of (A1) to (A1-16), wherein the cytokine release syndrome is caused by cytokine release from either or both of a non-tumor tissue and a tumor tissue.

Alternatively, in a non-limiting embodiment, the present disclosure relates to the following:
(A2) a kit comprising (i) a container, (ii) a pharmaceutical composition in the container, wherein the composition comprises an anti-T cell antigen-binding molecule, and (iii) a document instructing administration of a VEGF inhibitor before, simultaneously with, or after administration of the pharmaceutical composition to prevent and/or alleviate and/or treat cytokine release syndrome and/or cytokine release;
(A2-1) a kit comprising (i) a container, (ii) a pharmaceutical composition in the container, wherein the composition comprises an anti-T cell antigen-binding molecule, and (iii) a document instructing administration of a VEGF inhibitor 6 days, 5 days, 4 days, 3 days, 2 days, or 1 day before the administration of the pharmaceutical composition, or on the same day as but before administration of the pharmaceutical composition to prevent and/or alleviate and/or treat cytokine release syndrome and/or cytokine release;
(A2-2) the kit of (A2) or (A2-1), wherein the VEGF inhibitor is one or more VEGF inhibitors selected from an anti-VEGF antigen-binding molecule, an anti-VEGFR1 antigen-binding molecule, an anti-VEGFR2 antigen-binding molecule, a fusion protein comprising a VEGF receptor or a fragment thereof, and a tyrosine kinase inhibitor;
(A2-3) the kit of any one of (A2) to (A2-2), wherein the VEGF inhibitor is one or more VEGF inhibitors selected from Bevacizumab, Ramucirumab, and Aflibercept;
(A2-4) the kit of any one of (A2) to (A2-3), further comprising a label attached to the container, wherein the label indicates that the pharmaceutical composition can be used for treating cancer;
(A2-5) the kit of any one of (A2) to (A2-4), comprising a document instructing to further additionally use the pharmaceutical composition in combination with an immune checkpoint inhibitor;
(A2-6) the kit of (A2-5), wherein the immune checkpoint inhibitor is selected from an anti-PD1 antibody, an anti-PD-L1 antibody, and an anti-PD-L2 antibody;
(A2-7) the pharmaceutical composition of (A2-5) or (A2-6), wherein the immune checkpoint inhibitor is Atezolizumab;
(A2-8) the kit of any one of (A2) to (A2-7), further comprising a document instructing not to administer a corticosteroid before or simultaneously with administration of the pharmaceutical composition;
(A2-9) the kit of any one of (A2) to (A2-7), further comprising a document instructing further administration of a corticosteroid before, simultaneously with, or after administration of the pharmaceutical composition;
(A2-10) the kit of (A2-8) or (A2-9), wherein the corticosteroid is dexamethasone, a pharmaceutically acceptable salt thereof, or a derivative thereof;
(A2-11) the kit of any one of (A2) to (A2-10), wherein the anti-T cell antigen-binding molecule is a bispecific antigen-binding molecule comprising:
   (1) a domain comprising an antibody variable region having T cell receptor complex-binding activity; and
   (2) a domain comprising an antibody variable region having cancer antigen-binding activity;
(A2-12) the kit of any one of (A2) to (A2-11), wherein the anti-T cell antigen-binding molecule is a bispecific antibody comprising:
   (1) a domain comprising an antibody variable region having T cell receptor complex-binding activity;
   (2) a domain comprising an antibody variable region having glypican 3-binding activity; and
   (3) a domain comprising an Fc region with reduced binding activity towards an Fcy receptor;
(A2-13) the kit of any one of (A2) to (A2-12), wherein prescription of the pharmaceutical composition is performed according to the instructions in the document;
(A2-14) the kit of any one of (A2) to (A2-13), which is for treating cancer by the prescription instructed by the document;
(A2-15) the kit of any one of (A2) to (A2-14), wherein the cytokine release syndrome and/or cytokine release are associated with administration of the anti-T cell antigen-binding molecule;
(A2-16) the kit of any one of (A2) to (A1-15), wherein the cytokine release syndrome is caused by cytokine release from either or both of a non-tumor tissue and a tumor tissue;
(A2-17) the kit of any one of (A2) to (A2-16), wherein prescription of the pharmaceutical composition is performed according to the instructions in the document;
(A3) use of an anti-T cell antigen-binding molecule in the manufacture of a pharmaceutical composition, wherein the use of the pharmaceutical composition in combination with a VEGF inhibitor treats cancer while preventing and/or alleviating and/or treating cytokine release syndrome and/or cytokine release;
(A3-1) the use of (A3), wherein the VEGF inhibitor is administered before, simultaneously with, or after administration of the pharmaceutical composition;
(A3-2) the use of (A3), wherein the VEGF inhibitor is administered before or simultaneously with administration of the pharmaceutical composition;
(A3-3) the use of any one of (A3) to (A3-2), wherein the VEGF inhibitor is administered 6 days, 5 days, 4 days, 3 days, 2 days, or 1 day before the administration of the pharmaceutical composition, or on the same day as but before administration of the pharmaceutical composition;
(A3-4) the use of any one of (A3) to (A3-3), wherein the VEGF inhibitor is one or more VEGF inhibitors selected from an anti-VEGF antigen-binding molecule, an anti-VEGFR1 antigen-binding molecule, an anti-VEGFR2 antigen-binding molecule, a fusion protein comprising a VEGF receptor or a fragment thereof, and a tyrosine kinase inhibitor;
(A3-5) the use of any one of (A3) to (A3-4), wherein the VEGF inhibitor is one or more VEGF inhibitors selected from Bevacizumab, Ramucirumab, and Aflibercept;
(A3-6) the use of any one of (A3) to (A3-5), wherein the pharmaceutical composition is further additionally used in combination with an immune checkpoint inhibitor;
(A3-7) the use of (A3-6), wherein the immune checkpoint inhibitor is selected from an anti-PD1 antibody, an anti-PD-L1 antibody, and an anti-PD-L2 antibody;
(A3-8) the use of (A3-6) or (A3-7), wherein the immune checkpoint inhibitor is Atezolizumab;
(A3-9) the use of any one of (A3) to (A3-8), wherein a corticosteroid is not administered before or simultaneously with the administration of the pharmaceutical composition;
(A3-10) the use of any one of (A3) to (A3-8), wherein a corticosteroid is further administered before, simultaneously with, or after the administration of the pharmaceutical composition;
(A3-11) the use of (A3-9) or (A3-10) wherein the corticosteroid is dexamethasone, a pharmaceutically acceptable salt thereof, or a derivative thereof;
(A3-12) the use of any one of (A3) to (A3-11), wherein the anti-T cell antigen-binding molecule is a bispecific antigen-binding molecule comprising:
   (1) a domain comprising an antibody variable region having T cell receptor complex-binding activity; and
   (2) a domain comprising an antibody variable region having cancer antigen-binding activity;
(A3-13) the use of any one of (A3) to (A3-12), wherein the anti-T cell antigen-binding molecule is a bispecific antibody comprising:
   (1) a domain comprising an antibody variable region having T cell receptor complex-binding activity;
   (2) a domain comprising an antibody variable region having glypican 3-binding activity; and
   (3) a domain comprising an Fc region with reduced binding activity towards an Fcy receptor;
(A3-14) the use of any one of (A3) to (A3-13), wherein the cytokine release syndrome and/or cytokine release are associated with administration of the anti-T cell antigen-binding molecule;
(A3-15) the use of any one of (A3) to (A3-14), wherein the cytokine release syndrome is caused by cytokine release from either or both of a non-tumor tissue and a tumor tissue;
(A4) use of an anti-T cell antigen-binding molecule in a combination therapy with a VEGF inhibitor, wherein the combination therapy is for treating cancer while preventing and/or alleviating and/or treating cytokine release syndrome and/or cytokine release;
(A4-1) the use of (A4), wherein in the combination therapy, the VEGF inhibitor is administered before, simultaneously with, or after administration of the T cell antigen-binding molecule;
(A4-2) the use of (A4), wherein in the combination therapy, the VEGF inhibitor is administered before or simultaneously with administration of the T cell antigen-binding molecule;
(A4-3) the use of any one of (A4) to (A4-2), wherein in the combination therapy, the VEGF inhibitor is administered 6 days, 5 days, 4 days, 3 days, 2 days, or 1 day before the administration of the anti-T cell antigen-binding molecule, or on the same day as but before administration of the anti-T cell antigen-binding molecule;
(A4-4) the use of any one of (A4) to (A4-3), wherein the VEGF inhibitor is one or more VEGF inhibitors selected from an anti-VEGF antigen-binding molecule, an anti-VEGFR1 antigen-binding molecule, an anti-VEGFR2 antigen-binding molecule, a fusion protein comprising a VEGF receptor or a fragment thereof, and a tyrosine kinase inhibitor;
(A4-5) the use of any one of (A4) to (A4-4), wherein the VEGF inhibitor is one or more VEGF inhibitors selected from Bevacizumab, Ramucirumab, and Aflibercept;
(A4-6) the use of any one of (A4) to (A4-5), wherein the combination therapy involves further additionally administering an immune checkpoint inhibitor;
(A4-7) the use of (A4-6), wherein the immune checkpoint inhibitor is selected from an anti-PD1 antibody, an anti-PD-L1 antibody, and an anti-PD-L2 antibody;
(A4-8) the use of (A4-6) or (A4-7), wherein the immune checkpoint inhibitor is Atezolizumab;
(A4-9) the use of any one of (A4) to (A4-8), wherein in the combination therapy, a corticosteroid is not administered before or simultaneously with the administration of the anti-T cell antigen-binding molecule;
(A4-10) the use of any one of (A4) to (A4-8), wherein in the combination therapy, a corticosteroid is further administered before, simultaneously with, or after the administration of the anti-T cell antigen-binding molecule;
(A4-11) the use of (A4-9) or (A4-10), wherein the corticosteroid is dexamethasone, a pharmaceutically acceptable salt thereof, or a derivative thereof;
(A4-12) the use of any one of (A4) to (A4-11), wherein the anti-T cell antigen-binding molecule is a bispecific antigen-binding molecule comprising:
   (1) a domain comprising an antibody variable region having T cell receptor complex-binding activity; and
   (2) a domain comprising an antibody variable region having cancer antigen-binding activity;
(A4-13) the use of any one of (A4) to (A4-12), wherein the anti-T cell antigen-binding molecule is a bispecific antibody comprising:
   (1) a domain comprising an antibody variable region having T cell receptor complex-binding activity;
   (2) a domain comprising an antibody variable region having glypican 3-binding activity; and
   (3) a domain comprising an Fc region with reduced binding activity to an Fcy receptor;
(A4-14) the use of any one of (A4) to (A4-13), wherein the cytokine release syndrome and/or cytokine release are associated with administration of the anti-T cell antigen-binding molecule; and
(A4-15) the use of any one of (A4) to (A4-14), wherein the cytokine release syndrome is caused by cytokine release from either or both of a non-tumor tissue and a tumor tissue.

Alternatively, in a non-limiting embodiment, the present disclosure relates to the following:
(A5) a method for treating cancer while preventing and/or alleviating and/or treating cytokine release syndrome and/or cytokine release by administering an anti-T cell antigen-binding molecule to a subject, wherein the method comprises use of a VEGF inhibitor in combination;
(A5-1) the method of (A5), wherein the VEGF inhibitor is administered before, simultaneously with, or after administration of the anti-T cell antigen-binding molecule;
(A5-2) the method of (A5), wherein the VEGF inhibitor is administered before or simultaneously with administration of the anti-T cell antigen-binding molecule;
(A5-3) the method of any one of (A5) to (A5-2), wherein the VEGF inhibitor is administered 6 days, 5 days, 4 days, 3 days, 2 days, or 1 day before the administration of the anti-T cell antigen-binding molecule, or on the same day as but before administration of the anti-T cell antigen-binding molecule;
(A5-4) the method of any one of (A5) to (A5-3), wherein the VEGF inhibitor is one or more VEGF inhibitors selected from an anti-VEGF antigen-binding molecule, an anti-VEGFR1 antigen-binding molecule, an anti-VEGFR2 antigen-binding molecule, a fusion protein comprising a VEGF receptor or a fragment thereof, and a tyrosine kinase inhibitor;
(A5-5) the method of any one of (A5) to (A5-4), wherein the VEGF inhibitor is one or more VEGF inhibitors selected from Bevacizumab, Ramucirumab, and Aflibercept;
(A5-6) the method of any one of (A5) to (A5-5), which comprises further using an immune checkpoint inhibitor in combination;
(A5-7) the method of (A5-6), wherein the immune checkpoint inhibitor is selected from an anti-PD1 antibody, an anti-PD-L1 antibody, and an anti-PD-L2 antibody;
(A5-8) the method of (A5-6) or (A5-7), wherein the immune checkpoint inhibitor is Atezolizumab;
(A5-9) the method of any one of (A5) to (A5-8), wherein a corticosteroid is not administered before or simultaneously with the administration of the anti-T cell antigen-binding molecule;
(A5-10) the method of any one of (A5) to (A5-8), wherein a corticosteroid is further administered before, simultaneously with, or after the administration of the anti-T cell antigen-binding molecule;
(A5-11) the method of (A5-9) or (A5-10), wherein the corticosteroid is dexamethasone, a pharmaceutically acceptable salt thereof, or a derivative thereof;
(A5-12) the method of any one of (A5) to (A5-11), wherein the anti-T cell antigen-binding molecule is a bispecific antigen-binding molecule comprising:
   (1) a domain comprising an antibody variable region having T cell receptor complex-binding activity; and
   (2) a domain comprising an antibody variable region having cancer antigen-binding activity;
(A5-13) the method of any one of (A5) to (A5-12), wherein the anti-T cell antigen-binding molecule is a bispecific antibody comprising:
   (1) a domain comprising an antibody variable region having T cell receptor complex-binding activity;
   (2) a domain comprising an antibody variable region having glypican 3-binding activity; and
   (3) a domain comprising an Fc region with reduced binding activity towards an Fcy receptor;
(A5-14) the method of any one of (A5) to (A5-13), wherein the cytokine release syndrome and/or cytokine release are associated with administration of the anti-T cell antigen-binding molecule; and
(A5-15) (A, wherein the cytokine release syndrome is caused by cytokine release from either or both of a non-tumor tissue and a tumor tissue.

Alternatively, the present disclosure includes the following embodiments:
(B1) a pharmaceutical composition comprising a VEGF inhibitor, wherein the composition is for preventing and/or alleviating and/or treating cytokine release syndrome and/or cytokine release;
(B1-1) the pharmaceutical composition of (B 1), wherein the VEGF inhibitor is one or more VEGF inhibitors selected from an anti-VEGF antigen-binding molecule, an anti-VEGFR1 antigen-binding molecule, an anti-VEGFR2 antigen-binding molecule, a fusion protein comprising a VEGF receptor or a fragment thereof, and a tyrosine kinase inhibitor;
(B1-2) the pharmaceutical composition of (B 1) or (B 1-1), wherein the VEGF inhibitor is selected from the group consisting of Bevacizumab, Ramucirumab, and Aflibercept;
(B1-3) the pharmaceutical composition of any one of (B1) to (B1-2), which is for its use in combination with an immune checkpoint inhibitor;
(B1-4) the pharmaceutical composition of (B1-3), wherein the immune checkpoint inhibitor is selected from an anti-PD1 antibody, an anti-PD-L1 antibody, and an anti-PD-L2 antibody;
(B1-5) the pharmaceutical composition of (B1-3) or (B 1-4), wherein the immune checkpoint inhibitor is Atezolizumab;
(B1-6) the pharmaceutical composition of any one of (B1) to (B1-5), which is for use in its combination therapy with an anti-T cell antigen-binding molecule;
(B1-7) the pharmaceutical composition of (B1-6), which is for administration before, simultaneously with, or after administration of the anti-T cell antigen-binding molecule;
(B1-8) the pharmaceutical composition of (B1-6), which is for administration before or simultaneously with administration of the anti-T cell antigen-binding molecule;
(B1-9) the pharmaceutical composition of any one of (B1-6) to (B 1-8), which is for administration 6 days, 5 days, 4 days, 3 days, 2 days, or 1 day before the administration of the anti-T cell antigen-binding molecule, or on the same day as but before administration of the anti-T cell antigen-binding molecule;
(B1-10) the pharmaceutical composition of any one of (B1-6) to (B1-9), wherein a corticosteroid is not administered before or simultaneously with the administration of the anti-T cell antigen-binding molecule;
(B1-11) the pharmaceutical composition of any one of (B1-6) to (B1-9), wherein a corticosteroid is further administered before, simultaneously with, or after the administration of the anti-T cell antigen-binding molecule;
(B1-12) the pharmaceutical composition of (B1-10) or (B1-11), wherein the corticosteroid is dexamethasone, a pharmaceutically acceptable salt thereof, or a derivative thereof;
(B1-13) the pharmaceutical composition of any one of (B1-6) to (B1-12), wherein the anti-T cell antigen-binding molecule is a bispecific antigen-binding molecule comprising:
   (1) a domain comprising an antibody variable region having T cell receptor complex-binding activity; and
   (2) a domain comprising an antibody variable region having cancer antigen-binding activity;
(B1-14) the pharmaceutical composition of any one of (B1-6) to (B1-13), wherein the anti-T cell antigen-binding molecule is a bispecific antibody comprising:
   (1) a domain comprising an antibody variable region having T cell receptor complex-binding activity;
   (2) a domain comprising an antibody variable region having glypican 3-binding activity; and
   (3) a domain comprising an Fc region with reduced binding activity towards an Fcy receptor;
(B1-15) the pharmaceutical composition of any one of (B1-6) to (B1-14), wherein its use in combination with the anti-T cell antigen-binding molecule is for treating cancer while preventing and/or alleviating and/or treating cytokine release syndrome and/or cytokine release;
(B1-16) the pharmaceutical composition of any one of (B1-6) to (B1-15), wherein the cytokine release syndrome and/or cytokine release are associated with administration of the anti-T cell antigen-binding molecule; and
(B1-17) the pharmaceutical composition of any one of (B1) to (B1-16), wherein the cytokine release syndrome is caused by cytokine release from either or both of a non-tumor tissue and a tumor tissue.

Alternatively, in a non-limiting embodiment, the present disclosure relates to the following:
(B2) a kit comprising (i) a container, (ii) a pharmaceutical composition in the container, wherein the composition comprises a VEGF inhibitor, and (iii) a document instructing administration of the
   pharmaceutical composition to prevent and/or alleviate and/or treat cytokine release syndrome and/or cytokine release;
(B2-1) the kit of (B2), wherein the VEGF inhibitor is one or more cytokine inhibitors selected from an anti-VEGF antigen-binding molecule, an anti-VEGFR1 antigen-binding molecule, an anti-VEGFR2 antigen-binding molecule, a fusion protein comprising a VEGF receptor or a fragment thereof, and a tyrosine kinase inhibitor;
(B2-2) the kit of (B2) or (B2-1), wherein the VEGF inhibitor is one or more VEGF inhibitors selected from Bevacizumab, Ramucirumab, and Aflibercept;
(B2-3) the kit of (B2) to (B2-2), further comprising a document instructing to use the pharmaceutical composition in combination with an immune checkpoint inhibitor;
(B2-4) the kit of (B2-3), wherein the immune checkpoint inhibitor is selected from an anti-PD1 antibody, an anti-PD-L1 antibody, and an anti-PD-L2 antibody;
(B2-5) the pharmaceutical composition of (B2-3) or (B2-4), wherein the immune checkpoint inhibitor is Atezolizumab;
(B2-6) the kit of any one of (B2) to (B2-5), further comprising a document instructing to use the pharmaceutical composition in combination with an anti-T cell antigen-binding molecule;
(B2-7) the kit of (B2-6), further comprising a document instructing to administer the pharmaceutical composition before, simultaneously with, or after administration of the anti-T cell antigen-binding molecule;
(B2-8) the kit of (B2-6), further comprising a document instructing to administer the pharmaceutical composition before or simultaneously with administration of the anti-T cell antigen-binding molecule;
(B2-9) the kit of any one of (B2-6) to (B2-8), further comprising a document instructing to administer the pharmaceutical composition 6 days, 5 days, 4 days, 3 days, 2 days, or 1 day before the administration of the anti-T cell antigen-binding molecule, or on the same day as but before administration of the anti-T cell antigen-binding molecule;
(B2-10) the kit of any one of (B2-6) to (B2-9), further comprising a document instructing not to administer a corticosteroid before or simultaneously with administration of the anti-T cell antigen-binding molecule;
(B2-11) the kit of any one of (B2-6) to (B2-9), further comprising a document instructing to further administer a corticosteroid before, simultaneously with, or after administration of the anti-T cell antigen-binding molecule;
(B2-12) the kit of any one of (B2-10) to (B2-11), wherein the corticosteroid is dexamethasone, a pharmaceutically acceptable salt thereof, or a derivative thereof;
(B2-13) the kit of any one of (B2-6) to (B2-12), wherein the anti-T cell antigen-binding molecule is a bispecific antigen-binding molecule comprising:
   (1) a domain comprising an antibody variable region having T cell receptor complex-binding activity; and
   (2) a domain comprising an antibody variable region having cancer antigen-binding activity;
(B2-14) the kit of any one of (B2-6) to (B2-13), wherein the anti-T cell antigen-binding molecule is a bispecific antibody comprising:
   (1) a domain comprising an antibody variable region having T cell receptor complex-binding activity;
   (2) a domain comprising an antibody variable region having glypican 3-binding activity; and
   (3) a domain comprising an Fc region with reduced binding activity towards an Fcy receptor;
(B2-15) the kit of any one of (B2-6) to (B2-14), wherein combined use of the pharmaceutical composition and the anti-T cell antigen-binding molecule is for treating cancer while preventing and/or alleviating and/or treating cytokine release syndrome and/or cytokine release;
(B2-16) the kit of any one of (B2-6) to (B2-15), wherein the cytokine release syndrome and/or cytokine release are associated with administration of the anti-T cell antigen-binding molecule;
(B2-17) the kit of any one of (B2) to (B2-16), wherein the cytokine release syndrome is caused by cytokine release from either or both of a non-tumor tissue and a tumor tissue;
(B2-18) the kit of any one of (B2) to (B2-17), wherein prescription of the pharmaceutical composition is performed according to the instructions in the document;
(B3) use of a VEGF inhibitor in the manufacture of a pharmaceutical composition for preventing and/or alleviating and/or treating cytokine release syndrome and/or cytokine release;
(B3-1) the use of (B3), wherein the VEGF inhibitor is one or more cytokine inhibitors selected from an anti-VEGF antigen-binding molecule, an anti-VEGFR1 antigen-binding molecule, an anti-VEGFR2 antigen-binding molecule, a fusion protein comprising a VEGF receptor or a fragment thereof, and a tyrosine kinase inhibitor;
(B3-2) the use of (B3) or (B3-1), wherein the VEGF inhibitor is one or more VEGF inhibitors selected from Bevacizumab, Ramucirumab, and Aflibercept;
(B3-3) the use of any one of (B3) to (B3-2), wherein the pharmaceutical composition is used in combination with an immune checkpoint inhibitor;
(B3-4) the use of (B3-3), wherein the immune checkpoint inhibitor is selected from an anti-PD1 antibody, an anti-PD-L1 antibody, and an anti-PD-L2 antibody;
(B3-5) the use of (B3-3) or (B3-4), wherein the immune checkpoint inhibitor is Atezolizumab;
(B3-6) the use of any one of (B3) to (B3-5), wherein the pharmaceutical composition is for use in its combination therapy with an anti-T cell antigen-binding molecule;
(B3-7) the use of (B3-6), wherein the pharmaceutical composition is administered before, simultaneously with, or after administration of the anti-T cell antigen-binding molecule;
(B3-8) the use of (B3-6), wherein the pharmaceutical composition is administered before or simultaneously with administration of the anti-T cell antigen-binding molecule;
(B3-9) the use of any one of (B3-6) to (B3-8), wherein the pharmaceutical composition is administered 6 days, 5 days, 4 days, 3 days, 2 days, or 1 day before the administration of the anti-T cell antigen-binding molecule, or on the same day as but before administration of the anti-T cell antigen-binding molecule;
(B3-10) the use of any one of (B3-6) to (B3-9), wherein a corticosteroid is not administered before or simultaneously with the administration of the anti-T cell antigen-binding molecule;
(B3-11) the use of any one of (B3-6) to (B3-9), wherein a corticosteroid is further administered before, simultaneously with, or after the administration of the anti-T cell antigen-binding molecule;
(B3-12) the use of (B3-10) or (B3-11), wherein the corticosteroid is dexamethasone, a pharmaceutically acceptable salt thereof, or a derivative thereof;
(B3-13) the use of any one of (B3-6) to (B3-12), wherein the anti-T cell antigen-binding molecule is a bispecific antigen-binding molecule comprising:
   (1) a domain comprising an antibody variable region having T cell receptor complex-binding activity; and
   (2) a domain comprising an antibody variable region having cancer antigen-binding activity;
(B3-14) the use of any one of (B3-6) to (B3-13), wherein the anti-T cell antigen-binding molecule is a bispecific antibody comprising:
   (1) a domain comprising an antibody variable region having T cell receptor complex-binding activity;
   (2) a domain comprising an antibody variable region having glypican 3-binding activity; and
   (3) a domain comprising an Fc region with reduced binding activity towards an Fcy receptor;
(B3-15) the use of any one of (B3-6) to (B3-14), wherein the combination therapy using the pharmaceutical composition and the anti-T cell antigen-binding molecule is for treating cancer while preventing and/or alleviating and/or treating cytokine release syndrome and/or cytokine release;
(B3-16) the use of any one of (B3-6) to (B3-15), wherein the cytokine release syndrome and/or cytokine release are associated with administration of the anti-T cell antigen-binding molecule;
(B3-17) the use of any one of (B3) to (B3-16), wherein the cytokine release syndrome is caused by cytokine release from either or both of a non-tumor tissue and a tumor tissue;
(B4) use of a VEGF inhibitor in preventing and/or alleviating and/or treating cytokine release syndrome and/or cytokine release;
(B4-1) the use of (B4), wherein the VEGF inhibitor is one or more VEGF inhibitors selected from an anti-VEGF antigen-binding molecule, an anti-VEGFR1 antigen-binding molecule, an anti-VEGFR2 antigen-binding molecule, a fusion protein comprising a VEGF receptor or a fragment thereof, and a tyrosine kinase inhibitor;
(B4-2) the use of (B4) or (B4-1), wherein the VEGF inhibitor is one or more VEGF inhibitors selected from Bevacizumab, Ramucirumab, and Aflibercept;
(B4-3) the use of any one of (B4) to (B4-2), wherein the VEGF inhibitor is used in combination with an immune checkpoint inhibitor;
(B4-4) the use of (B4-3), wherein the immune checkpoint inhibitor is selected from an anti-PD1 antibody, an anti-PD-L1 antibody, and an anti-PD-L2 antibody;
(B4-5) the use of (B4-3) or (B4-4), wherein the immune checkpoint inhibitor is Atezolizumab;
(B4-6) the use of any one of (B4) to (B4-5), wherein the VEGF inhibitor is for use in combination with an anti-T cell antigen-binding molecule;
(B4-7) the use of (B4-6), wherein the VEGF inhibitor is administered before, simultaneously with, or after administration of the anti-T cell antigen-binding molecule;
(B4-8) the use of (B4-6), wherein the VEGF inhibitor is administered before or simultaneously with administration of the anti-T cell antigen-binding molecule;
(B4-9) the use of any one of (B4-6) to (B4-8), wherein the VEGF inhibitor is administered 6 days, 5 days, 4 days, 3 days, 2 days, or 1 day before the administration of the anti-T cell antigen-binding molecule, or on the same day as but before administration of the anti-T cell antigen-binding molecule;
(B4-10) the use of any one of (B4-6) to (B4-9), wherein a corticosteroid is not administered before or simultaneously with the administration of the anti-T cell antigen-binding molecule;
(B4-11) the use of any one of (B4-6) to (B4-9), wherein a corticosteroid is further administered before, simultaneously with, or after the administration of the anti-T cell antigen-binding molecule;
(B4-12) the use of (B4-10) or (B4-11), wherein the corticosteroid is dexamethasone, a pharmaceutically acceptable salt thereof, or a derivative thereof;
(B4-13) the use of any one of (B4-6) to (B4-12), wherein the anti-T cell antigen-binding molecule is a bispecific antigen-binding molecule comprising:
   (1) a domain comprising an antibody variable region having T cell receptor complex-binding activity; and
   (2) a domain comprising an antibody variable region having cancer antigen-binding activity;
(B4-14) the use of any one of (B4-6) to (B4-13), wherein the anti-T cell antigen-binding molecule is a bispecific antibody comprising:
   (1) a domain comprising an antibody variable region having T cell receptor complex-binding activity;
   (2) a domain comprising an antibody variable region having glypican 3-binding activity; and
   (3) a domain comprising an Fc region with reduced binding activity towards an Fcy receptor;
(B4-15) the use of any one of (B4-6) to (B4-14), wherein use of the VEGF inhibitor in combination with the anti-T cell antigen-binding molecule is for treating cancer while preventing and/or alleviating and/or treating cytokine release syndrome and/or cytokine release;
(B4-16) the use of any one of (B4-6) to (B4-15), wherein the cytokine release syndrome and/or cytokine release are associated with administration of the anti-T cell antigen-binding molecule; and
(B4-17) the use of any one of (B4) to (B4-16), wherein the cytokine release syndrome is caused by cytokine release from either or both of a non-tumor tissue and a tumor tissue.

Alternatively, in a non-limiting embodiment, the present disclosure relates to the following:
(B5) a method of preventing and/or alleviating and/or treating cytokine release syndrome and/or cytokine release, wherein the method comprises administering a VEGF inhibitor to a subject;
(B5-1) the method of (B5), wherein the VEGF inhibitor is one or more VEGF inhibitors selected from an anti-VEGF antigen-binding molecule, an anti-VEGFR1 antigen-binding molecule, an anti-VEGFR2 antigen-binding molecule, a fusion protein comprising a VEGF receptor or a fragment thereof, and a tyrosine kinase inhibitor;
(B5-2) the method of (B5) or (B5-1), wherein the VEGF inhibitor is one or more VEGF inhibitors selected from Bevacizumab, Ramucirumab, and Aflibercept;
(B5-3) the method of any one of (B5) to (B5-2), wherein the cytokine release syndrome and/or cytokine release are associated with administration of an anti-T cell antigen-binding molecule;
(B5-4) the method of (B5-3), wherein the VEGF inhibitor is administered to a subject before, simultaneously with, or after administration of the anti-T cell antigen-binding molecule;
(B5-5) the method of (B5-3), wherein the VEGF inhibitor is administered to a subject before or simultaneously with administration of the anti-T cell antigen-binding molecule;
(B5-6) the method of any one of (B5-3) to (B5-5), wherein the VEGF inhibitor is administered to a subject 6 days, 5 days, 4 days, 3 days, 2 days, or 1 day before the administration of the anti-T cell antigen-binding molecule, or on the same day as but before administration of the anti-T cell antigen-binding molecule;
(B5-7) the method of any one of (B5-3) to (B5-6), wherein the anti-T cell antigen-binding molecule is a bispecific antigen-binding molecule comprising:
   (1) a domain comprising an antibody variable region having T cell receptor complex-binding activity; and
   (2) a domain comprising an antibody variable region having cancer antigen-binding activity;
(B5-8) the method of any one of (B5-3) to (B5-7), wherein the anti-T cell antigen-binding molecule is a bispecific antibody comprising:
   (1) a domain comprising an antibody variable region having T cell receptor complex-binding activity;
   (2) a domain comprising an antibody variable region having glypican 3-binding activity; and
   (3) a domain comprising an Fc region with reduced binding activity towards an Fcy receptor;
(B5-9) the method of any one of (B5) to (B5-8), which is for treating cancer while preventing and/or alleviating and/or treating cytokine release syndrome and/or cytokine release; and
(B5-10) the method of (B5-9), wherein the cytokine release syndrome is caused by cytokine release from either or both of a non-tumor tissue and a tumor tissue;

Alternatively, in a non-limiting embodiment, the present disclosure relates to the following:
(C1) a method of treating cancer while preventing and/or alleviating and/or treating cytokine release syndrome and/or cytokine release, wherein the method comprises administering an anti-T cell antigen-binding molecule and a VEGF inhibitor to a subject;
(C1-1) a method of combination therapy for treating cancer while preventing and/or alleviating and/or treating cytokine release syndrome and/or cytokine release, wherein the method comprises administering an anti-T cell antigen-binding molecule, and administering a VEGF inhibitor, to a subject;
(C1-2) the method of (C1) or (C1-1), wherein the VEGF inhibitor is administered before, simultaneously with, or after administration of the T cell antigen-binding molecule to a subject;
(C1-3) the method of any one of (C1) to (C1-2), wherein the VEGF inhibitor is administered before or simultaneously with administration of the T cell antigen-binding molecule to a subject;
(C1-4) the method of any one of (C1) to (C1-3), wherein the VEGF inhibitor is administered 6 days, 5 days, 4 days, 3 days, 2 days, or 1 day before the administration of the anti-T cell antigen-binding molecule to a subject, or on the same day as but before administration of the anti-T cell antigen-binding molecule;
(C1-5) the method of any one of (C1) to (C1-4), wherein the VEGF inhibitor is one or more VEGF inhibitors selected from an anti-VEGF antigen-binding molecule, an anti-VEGFR1 antigen-binding molecule, an anti-VEGFR2 antigen-binding molecule, a fusion protein comprising a VEGF receptor or a fragment thereof, and a tyrosine kinase inhibitor;
(C1-6) the method of any one of (C1) to (C1-5), wherein the VEGF inhibitor is one or more VEGF inhibitors selected from Bevacizumab, Ramucirumab, and Aflibercept;
(C1-7) the method of any one of (C1) to (C1-6), which comprises further administering an immune checkpoint inhibitor to a subject;
(C1-8) the method of (C1-7), wherein the immune checkpoint inhibitor is selected from an anti-PD1 antibody, an anti-PD-L1 antibody, and an anti-PD-L2 antibody;
(C1-9) the method of (C1-7) or (C1-8), wherein the immune checkpoint inhibitor is Atezolizumab;
(C1-10) the method of any one of (C1) to (C1-9), wherein a corticosteroid is not administered to a subject before or simultaneously with the administration of the anti-T cell antigen-binding molecule;
(C1-11) the method of any one of (C1) to (C1-9), which comprises further administering a corticosteroid to a subject before, simultaneously with, or after the administration of the anti-T cell antigen-binding molecule;
(C1-12) the method of (C1-10) or (C1-11), wherein the corticosteroid is dexamethasone, a pharmaceutically acceptable salt thereof, or a derivative thereof;
(C1-13) the method of any one of (C1) to (C1-12), wherein the anti-T cell antigen-binding molecule is a bispecific antigen-binding molecule comprising:
   (1) a domain comprising an antibody variable region having T cell receptor complex-binding activity; and
   (2) a domain comprising an antibody variable region having cancer antigen-binding activity;
(C1-14) the method of any one of (C1) to (C1-13), wherein the anti-T cell antigen-binding molecule is a bispecific antibody comprising:
   (1) a domain comprising an antibody variable region having T cell receptor complex-binding activity;
   (2) a domain comprising an antibody variable region having glypican 3-binding activity; and
   (3) a domain comprising an Fc region with reduced binding activity towards an Fcγ receptor;
(C1-15) the method of any one of (C1) to (C1-14), wherein the cytokine release syndrome and/or cytokine release are associated with administration of the anti-T cell antigen-binding molecule; and
(C1-16) the method of any one of (C1) to (C1-15), wherein the cytokine release syndrome is caused by cytokine release from either or both of a non-tumor tissue and a tumor tissue.

In a non-limiting embodiment, the present disclosure relates to combination therapies of various embodiments disclosed herein, which comprise administering an anti-T cell antigen-binding molecule and a VEGF inhibitor; methods for treating cancer by the combination therapies; and methods aimed at any one or a combination selected from prevention, alleviation, and treatment, by the combination therapies, of cytokine release syndrome associated with administration of the anti-T cell antigen-binding molecule.

Alternatively, in a non-limiting embodiment, the present disclosure relates to the following:
(C2) a combination therapy for treating cancer, while preventing and/or alleviating and/or treating cytokine release syndrome and/or cytokine release, wherein the therapy comprises an anti-T cell antigen-binding molecule and a VEGF inhibitor;
(C2-1) the combination therapy of (C2), wherein the VEGF inhibitor is administered before, simultaneously with, or after administration of the anti-T cell antigen-binding molecule;
(C2-2) the combination therapy of (C2) or (C2-1), wherein the VEGF inhibitor is administered before or simultaneously with administration of the anti-T cell antigen-binding molecule;
(C2-3) the combination therapy of (C2) or (C2-1), wherein the VEGF inhibitor is administered 6 days, 5 days, 4 days, 3 days, 2 days, or 1 day before the administration of the anti-T cell antigen-binding molecule, or on the same day as but before administration of the anti-T cell antigen-binding molecule;
(C2-4) the combination therapy of any one of (C2) to (C2-3), wherein the VEGF inhibitor is one or more cytokine inhibitors selected from an anti-VEGF antigen-binding molecule, an anti-VEGFR1 antigen-binding molecule, an anti-VEGFR2 antigen-binding molecule, a fusion protein comprising a VEGF receptor or a fragment thereof, and a tyrosine kinase inhibitor;
(C2-5) the combination therapy of any one of (C2) to (C2-4), wherein the VEGF inhibitor is one or more VEGF inhibitors selected from Bevacizumab, Ramucirumab, and Aflibercept;
(C2-6) the combination therapy of any one of (C2) to (C2-5), which comprises further using an immune checkpoint inhibitor in combination;
(C2-7) the combination therapy of (C2-6), wherein the immune checkpoint inhibitor is selected from an anti-PD1 antibody, an anti-PD-L1 antibody, and an anti-PD-L2 antibody;
(C2-8) the combination therapy of (C2-6) or (C2-7), wherein the immune checkpoint inhibitor is Atezolizumab;
(C2-9) the combination therapy of any one of (C2) to (C2-8), wherein a corticosteroid is not administered before or simultaneously with the administration of the anti-T cell antigen-binding molecule;
(C2-10) the combination therapy of any one of (C2) to (C2-8), wherein a corticosteroid is further administered before, simultaneously with, or after the administration of the anti-T cell antigen-binding molecule;
(C2-11) the combination therapy of (C2-9) or (C2-10), wherein the corticosteroid is dexamethasone, a pharmaceutically acceptable salt thereof, or a derivative thereof;
(C2-12) the combination therapy of any one of (C2) to (C2-11), wherein the anti-T cell antigen-binding molecule is a bispecific antigen-binding molecule comprising:
   (1) a domain comprising an antibody variable region having T cell receptor complex-binding activity; and
   (2) a domain comprising an antibody variable region having cancer antigen-binding activity;
(C2-13) the combination therapy of any one of (C2) to (C2-12), wherein the anti-T cell antigen-binding molecule is a bispecific antibody comprising:
   (1) a domain comprising an antibody variable region having T cell receptor complex-binding activity;
   (2) a domain comprising an antibody variable region having glypican 3-binding activity; and
   (3) a domain comprising an Fc region with reduced binding activity towards an Fcγ receptor;
(C2-14) the combination therapy of any one of (C2) to (C2-13), wherein the cytokine release syndrome and/or cytokine release are associated with administration of the anti-T cell antigen-binding molecule;
(C2-15) the combination therapy of any one of (C2) to (C2-14), wherein the cytokine release syndrome is caused by cytokine release from either or both of a non-tumor tissue and a tumor tissue;
(C3) a pharmaceutical composition comprising an anti-T cell antigen-binding molecule and a VEGF inhibitor, wherein the composition is for treating cancer while preventing and/or alleviating and/or treating cytokine release syndrome and/or cytokine release;
(C3-1) the pharmaceutical composition of (C3), wherein the VEGF inhibitor is one or more cytokine inhibitors selected from an anti-VEGF antigen-binding molecule, an anti-VEGFR1 antigen-binding molecule, an anti-VEGFR2 antigen-binding molecule, a fusion protein comprising a VEGF receptor or a fragment thereof, and a tyrosine kinase inhibitor;
(C3-2) the pharmaceutical composition of (C3) or (C3-1), wherein the VEGF inhibitor is one or more VEGF inhibitors selected from Bevacizumab, Ramucirumab, and Aflibercept;
(C3-3) the pharmaceutical composition of any one of (C3) to (C3-2), wherein an immune checkpoint inhibitor is further used in combination;
(C3-4) the pharmaceutical composition of (C3-3), wherein the immune checkpoint inhibitor is selected from an anti-PD1 antibody, an anti-PD-L1 antibody, and an anti-PD-L2 antibody;
(C3-5) the pharmaceutical composition of (C3-3) or (C3-4), wherein the immune checkpoint inhibitor is Atezolizumab;
(C3-6) the pharmaceutical composition of any one of (C3) to (C3-5), wherein a corticosteroid is not administered before or simultaneously with the administration of the pharmaceutical composition;
(C3-7) the pharmaceutical composition of any one of (C3) to (C3-5), wherein a corticosteroid is further administered before, simultaneously with, or after the administration of the pharmaceutical composition;
(C3-8) the pharmaceutical composition of (C3-6) or (C3-7), wherein the corticosteroid is dexamethasone, a pharmaceutically acceptable salt thereof, or a derivative thereof;
(C3-9) the pharmaceutical composition of any one of (C3) to (C3-8), wherein the anti-T cell antigen-binding molecule is a bispecific antigen-binding molecule comprising:
   (1) a domain comprising an antibody variable region having T cell receptor complex-binding activity; and
   (2) a domain comprising an antibody variable region having cancer antigen-binding activity;
(C3-10) the pharmaceutical composition of any one of (C3) to (C3-9), wherein the anti-T cell antigen-binding molecule is a bispecific antibody comprising:
   (1) a domain comprising an antibody variable region having T cell receptor complex-binding activity;
   (2) a domain comprising an antibody variable region having glypican 3-binding activity; and
   (3) a domain comprising an Fc region with reduced binding activity towards an Fcγ receptor;
(C3-11) the pharmaceutical composition of any one of (C3) to (C3-10), wherein the cytokine release syndrome and/or cytokine release are associated with administration of the anti-T cell antigen-binding molecule;
(C3-12) the pharmaceutical composition of any one of (C3) to (C3-11), wherein the cytokine release syndrome is caused by cytokine release from either or both of a non-tumor tissue and a tumor tissue; and
(C4) a combination of an anti-T cell antigen-binding molecule and a VEGF inhibitor.

### [Effects of the Invention]

Anti-T cell antigen-binding molecules which bind to T cell antigens such as CD3 are drawing attention as novel means for treating cancer that utilizes the antitumor effects of T cells possessed by living bodies. However, since they target T cells that produce cytokines, the possibility of causing adverse reactions due to cytokine production (for example, cytokine release syndrome; CRS) has been pointed out. Therefore, concerns of unexpected adverse reactions caused by cytokine production had existed for administration of anti-T cell antigen-binding molecules. In the present disclosure, an anti-T cell antigen-binding molecule is administered in combination with a VEGF inhibitor in a preplanned manner; therefore, unexpected adverse reactions caused by cytokines can be prevented or alleviated.

### [Brief Description of Drawings]

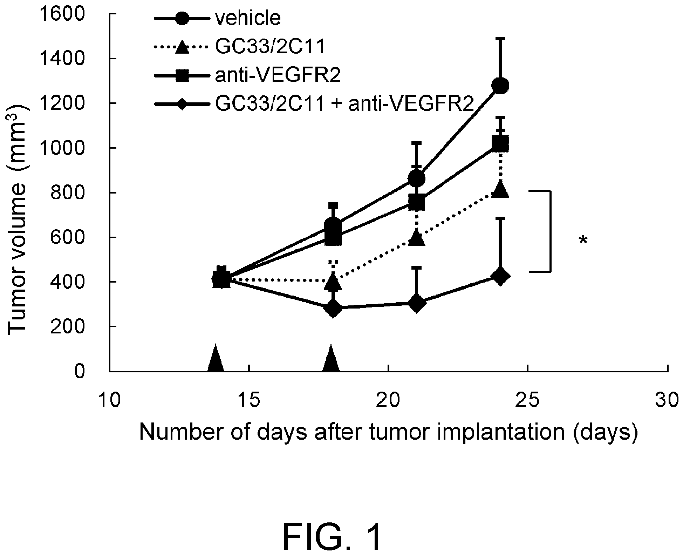

Fig. 1 shows the effect on drug efficacy of the ERY974 surrogate antibody (GC33/2C11) by the combined use of the anti-mouse VEGFR2 antibody (DC101). In the figure, the vertical axis represents the tumor volume (mm³), and the horizontal axis represents the number of days after tumor transplantation when the day of tumor transplantation is defined as Day 0.

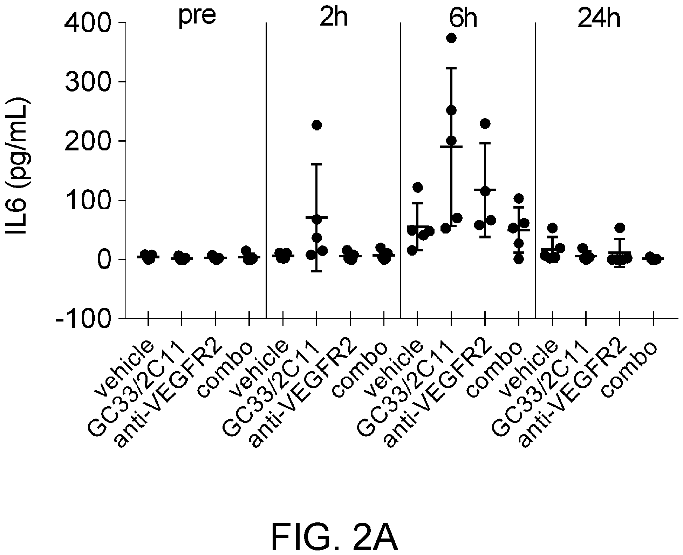

Fig. 2-A shows the effect on tumor-derived cytokine (IL-6) production caused by the ERY974 surrogate antibody (GC33/2C11) by the combined use of the anti-mouse VEGFR2 antibody (DC101). In the figure, the vertical axis represents the plasma IL-6 concentration (pg/mL), the horizontal axis indicates the ingredients administered to the mice, and "combo" refers to combined use of the two ingredients. Indicated at the top of the graph is the time elapsed after administration when the time of the administration of the active ingredients is defined as 0.

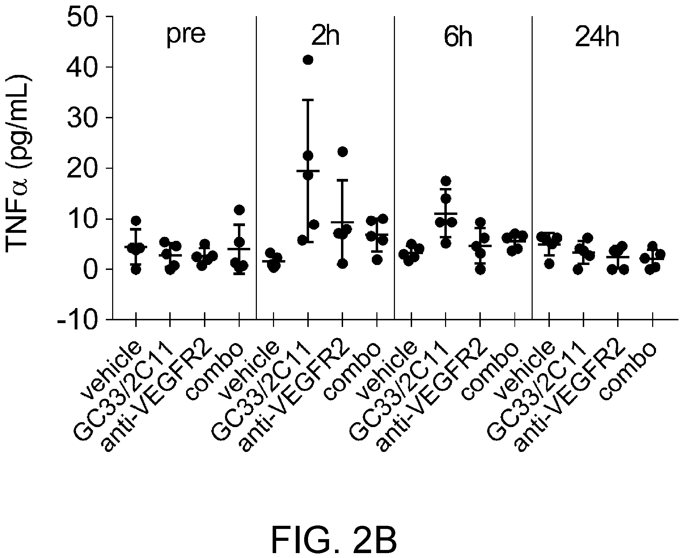

Fig. 2-B shows the effect on tumor-derived cytokine (TNF) production caused by the ERY974 surrogate antibody (GC33/2C11) by the combined use of the anti-mouse VEGFR2 antibody (DC101). In the figure, the vertical axis represents the plasma TNF concentration (pg/mL), the horizontal axis indicates the ingredients administered to the mice, and "combo" refers to combined use of the two ingredients. Indicated at the top of the graph is the time elapsed after administration when the time of the administration of the active ingredients is defined as 0.

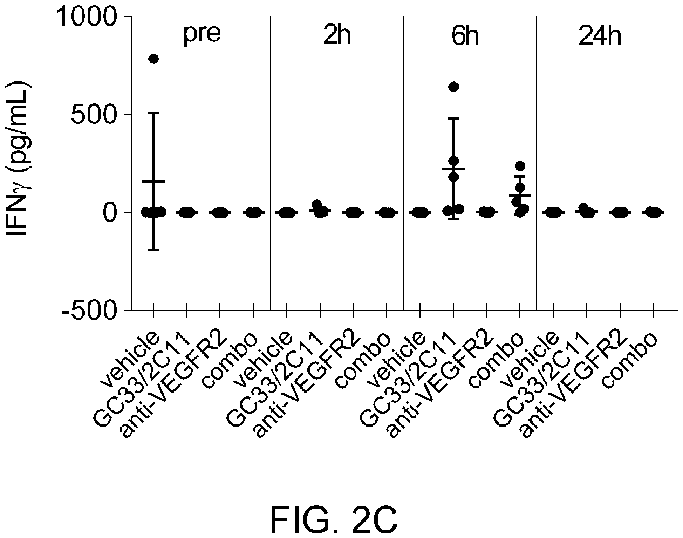

Fig. 2-C shows the effect on tumor-derived cytokine (IFNgamma) production caused by the ERY974 surrogate antibody (GC33/2C11) by the combined use of the anti-mouse VEGFR2 antibody (DC101). In the figure, the vertical axis represents the plasma IFNy concentration (pg/mL), the horizontal axis indicates the ingredients administered to the mice, and "combo" refers to combined use of the two ingredients. Indicated at the top of the graph is the time elapsed after administration when the time of the administration of the active ingredients is defined as 0.

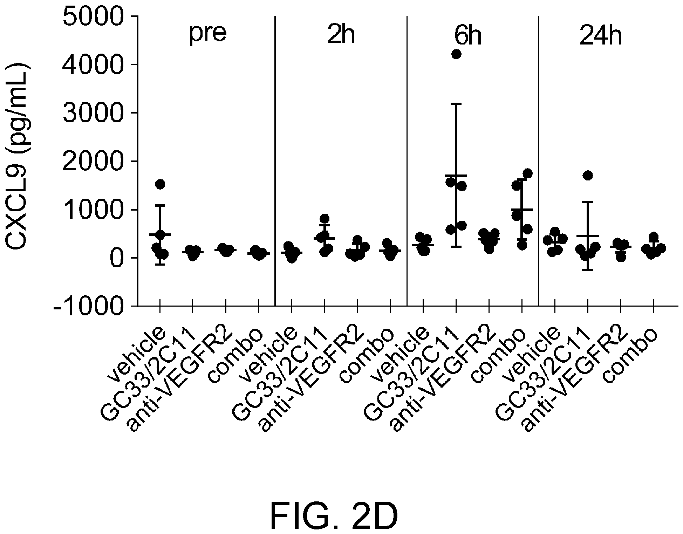

Fig. 2-D shows the effect on tumor-derived cytokine (CXCL9) production caused by the ERY974 surrogate antibody (GC33/2C11) by the combined use of the anti-mouse VEGFR2 antibody (DC101). In the figure, the vertical axis represents the plasma CXC ligand 9 concentration (pg/mL), the horizontal axis indicates the ingredients administered to the mice, and "combo" refers to combined use of the two ingredients. Indicated at the top of the graph is the time elapsed after administration when the time of the administration of the active ingredients is defined as 0.

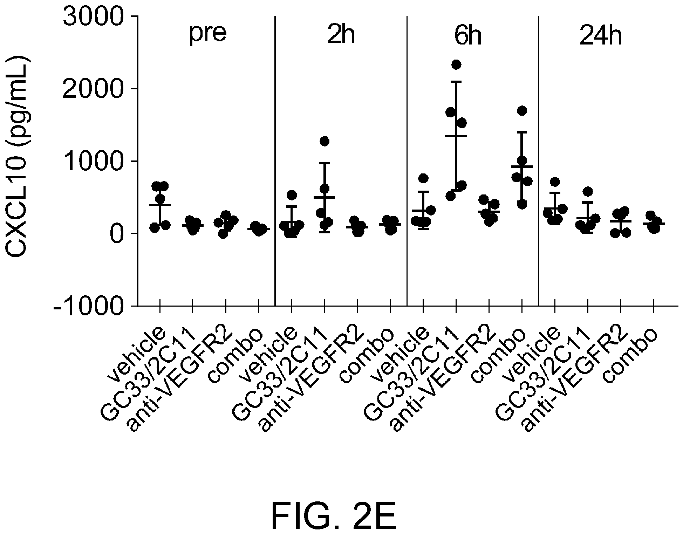

Fig. 2-E shows the effect on tumor-derived cytokine (CXCL10) production caused by the ERY974 surrogate antibody (GC33/2C11) by the combined use of the anti-mouse VEGFR2 antibody (DC101). In the figure, the vertical axis represents the plasma CXCL10 concentration (pg/mL), the horizontal axis indicates the ingredients administered to the mice, and "combo" refers to combined use of the two ingredients. Indicated at the top of the graph is the time elapsed after administration when the time of the administration of the active ingredients is defined as 0.

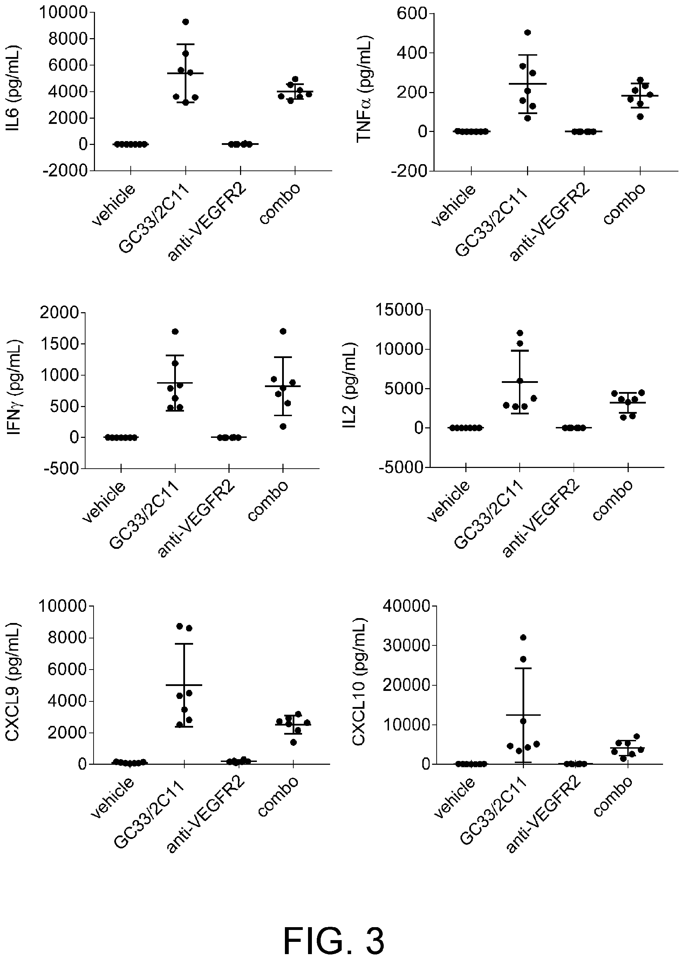

Fig. 3 shows the effect on normal tissue-derived cytokine production caused by the ERY974 surrogate antibody (GC33/2C11) by the combined use of the anti-mouse VEGFR2 antibody (DC101). In the figure, the vertical axis represents the plasma concentration (pg/mL) of IL-6 (top left), TNFα (top right), IFNy (middle left), IL-2 (middle right), CXCL9 (bottom left), or CXCL10 (bottom right) two hours after administration, and the horizontal axis indicates the active ingredients administered.

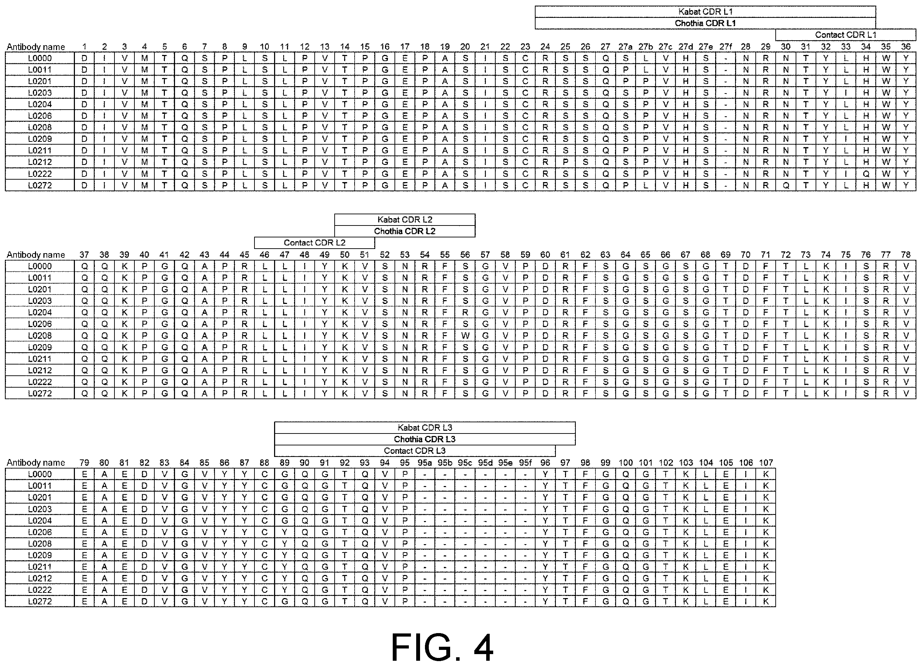

Fig. 4 shows the light chain variable region sequences and their various numbering according to Kabat and such.

### [Description of Embodiments]

### I. Definition

The definitions below are provided to help understanding of the present invention illustrated herein.

### Specific

The term "specific" means that one of the molecules involved in specific binding does not show any significant binding to molecules other than a single or a number of binding partner molecules. Furthermore, the term is also used when a domain containing an antibody variable region is specific to a particular epitope among the multiple epitopes in an antigen. When an epitope bound by a domain containing an antibody variable region is included in a number of different antigens, antigen-binding molecules comprising the antibody variable region-containing domain can bind to various antigens that have the epitope.

### Binding activity

The term "binding activity" refers to the strength of the sum total of noncovalent interactions between one or more binding sites of a molecule (e.g., an antibody) and its binding partner (e.g., an antigen). Herein, "binding activity" is not strictly limited to a 1:1 interaction between members of a binding pair (e.g., antibody and antigen). For example, when the members of a binding pair reflect a monovalent 1: 1 interaction, the binding activity is particularly called the intrinsic binding affinity (affinity). When a member of a binding pair is capable of both monovalent binding and multivalent binding, the binding activity is the sum of each binding strength. The binding activity of a molecule X for its partner Y can generally be represented by the dissociation constant (KD) or "binding amount of analyte per unit amount of ligand" (hereinbelow, may be referred to as "binding amount"). Those skilled in the art would understand that, generally, lower value of dissociation constant (KD) means higher binding activity; and higher value of "binding amount of analyte per unit amount of ligand" or "binding amount" means higher binding activity. Binding activity can be measured by common methods known in the art, including those described herein. Specific illustrative and exemplary embodiments for measuring binding activity (including affinity) are described in the following. Specific illustrative and exemplary embodiments for measuring binding affinity are described in the following.

### Anti-"target antigen" -binding antigen-binding molecule

The terms "anti-'target antigen'-binding antigen-binding molecule" (herein 'target antigen' is any antigen protein that can be targeted, and examples include a T cell antigen, cancer antigen, cytokine, and cytokine receptor) and "an antigen-binding molecule that binds to a target antigen" refer to an antigen-binding molecule that is capable of binding a target antigen with sufficient affinity such that the antigen-binding molecule is useful as a diagnostic and/or therapeutic agent when the antigen-binding molecule targets the antigen. In one embodiment, the extent of binding of an antigen-binding molecule to an unrelated, non-target antigen protein is less than about 10% of the binding of the antigen-binding molecule to the target antigen as measured, e.g., by a radioimmunoassay (RIA). In certain embodiments, an antigen-binding molecule that binds to the target protein has a dissociation constant (Kd) of 1 µM or less, 100 nM or less, 10 nM or less, 1 nM or less, 0.1 nM or less, 0.01 nM or less, or 0.001 nM or less (e.g. 10⁻⁸ M or less, e.g. from 10⁻⁸ M to 10⁻¹³ M, e.g., from 10⁻⁹ M to 10⁻¹³ M). In certain embodiments, an antigen-binding molecule binds to an epitope of the target antigen that is conserved among the target antigen from different species.

### Antigen-binding molecule "that binds to the same epitope" as a reference antigen-binding molecule

An antigen-binding molecule "that binds to the same epitope" as a reference antigen-binding molecule refers to an antigen-binding molecule that blocks binding of the reference antigen-binding molecule to its antigen in a competition assay by 50% or more, and conversely, the reference antigen-binding molecule blocks binding of the antigen-binding molecule to its antigen in a competition assay by 50% or more. An exemplary competition assay is provided herein.

In an exemplary competition assay, an immobilized antigen (for example, GPC3, CD3, and/or VEGF) is incubated in a solution comprising a first labeled antigen-binding molecule that binds to the antigen and a second unlabeled antigen-binding molecule that is being tested for its ability to compete with the first antigen-binding molecule for binding to the antigen. The second antigen-binding molecule may be present in a hybridoma supernatant. As a control, the immobilized antigen is incubated in a solution comprising the first labeled antigen-binding molecule but not the second unlabeled antigen-binding molecule. After incubation under conditions permissive for binding of the first antigen-binding molecule to the antigen, excess unbound antigen-binding molecule is removed, and the amount of label associated with the immobilized antigen is measured. If the amount of label associated with the immobilized antigen is substantially reduced in the test sample relative to the control sample, then that indicates that the second antigen-binding molecule is competing with the first antigen-binding molecule for binding to the antigen. See Harlow and Lane (1988) Antibodies: A Laboratory Manual ch. 14 (Cold Spring Harbor Laboratory, Cold Spring Harbor, NY).

### Antigen-binding molecule

The term "antigen-binding molecule" herein is used in the broadest sense and encompasses various antibody structures, including but not limited to monoclonal antibodies, polyclonal antibodies, multispecific antibodies (e.g., bispecific antibodies), antibody derivatives, and antibody fragments so long as they exhibit the desired antigen-binding activity.

In certain embodiments, an antibody provided herein may be further modified to contain additional nonproteinaceous moieties that are known in the art and readily available (this is called "antibody derivatives"). The moieties suitable for derivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, polypropylene glycol homopolymers, polypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer are attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

### Antibody fragment

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')2; diabodies; linear antibodies; single-chain antibody molecules (e.g. scFv); and multispecific antibodies formed from antibody fragments.

### Antibody that binds to the same epitope as a reference antibody

An "antibody that binds to the same epitope" as a reference antibody refers to an antibody that blocks binding of the reference antibody to its antigen in a competition assay by 50% or more, and conversely, the reference antibody blocks binding of the antibody to its antigen in a competition assay by 50% or more. An exemplary competition assay is provided herein.

### Chimeric antibody

The term "chimeric" antibody refers to an antibody in which a portion of the heavy and/or light chain is derived from a particular source or species, while the remainder of the heavy and/or light chain is derived from a different source or species.

### Antibody class

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG1, IgG2, IgG3, IgG4, IgA1, and IgA2. The heavy chain constant domains that correspond to the different classes of immunoglobulins are called alpha, delta, epsilon, gamma, and mu, respectively.

Constant regions of the isotypes IgG1, IgG2, IgG3, and IgG4 are called Cγ1, Cy2, Cy3, and Cy4, respectively. The amino acid sequences of Fc region polypeptides forming human Cγ1, Cy2, Cy3, and Cy4 are exemplified in SEQ ID NO: 23, 24, 25, and 26, respectively. The relationship between amino acid residues forming each amino acid sequence and Kabat's EU numbering (herein also referred to as EU INDEX) is shown in Fig. 1.

### Fc region

The term "Fc region" herein is used to define a C-terminal region of an immunoglobulin heavy chain that contains at least a portion of the constant region. The term includes native sequence Fc regions and variant Fc regions. In one embodiment, a human IgG heavy chain Fc region extends from Cys226, or from Pro230, to the carboxyl-terminus of the heavy chain. However, the C-terminal lysine (Lys447) or glycine-lysine (Gly446-Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

### Fc receptor

The term "Fc receptor" or "FcR" refers to a receptor that binds to the Fc region of an antibody. In some embodiments, an FcR is a native human FcR. In some embodiments, an FcR is one which binds an IgG antibody (a gamma receptor) and includes receptors of the Fc gamma RI, Fc gamma RII, and Fc gamma RIII subclasses, including allelic variants and alternatively spliced forms of those receptors. Fc gamma RII receptors include Fc gamma RIIA (an "activating receptor") and Fc gamma RIIB (an "inhibiting receptor"), which have similar amino acid sequences that differ primarily in the cytoplasmic domains thereof. Activating receptor Fc gamma RIIA contains an immunoreceptor tyrosine-based activation motif (ITAM) in its cytoplasmic domain. Inhibiting receptor Fc gamma RIIB contains an immunoreceptor tyrosine-based inhibition motif (ITIM) in its cytoplasmic domain. (see, *e.g.,* Daëron, Annu. Rev. Immunol. 15:203-234 (1997)). FcRs are reviewed, for example, in Ravetch and Kinet, Annu. Rev. Immunol 9:457-92 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein.

The term "Fc receptor" or "FcR" also includes the neonatal receptor, FcRn, which is responsible for the transfer of maternal IgGs to the fetus (Guyer et al., J. Immunol. 117:587 (1976) and Kim etal., J. Immunol. 24:249 (1994)) and regulation of homeostasis of immunoglobulins. Methods of measuring binding to FcRn are known (see, *e.g.,* Ghetie and Ward., Immunol. Today 18(12):592-598 (1997); Ghetie et al., Nature Biotechnology, 15(7):637-640 (1997); Hinton et al., J. Biol. Chem. 279(8):6213-6216 (2004); WO 2004/92219 (Hinton et al.).

Binding to human FcRn *in vivo* and plasma half life of human FcRn high affinity binding polypeptides can be assayed, *e*.*g*., in transgenic mice or transfected human cell lines expressing human FcRn, or in primates to which the polypeptides with a variant Fc region are administered. WO 2000/42072 (Presta) describes antibody variants with increased or decreased binding to FcRs. See also, *e.g.,* Shields et al. J. Biol. Chem. 9(2):6591-6604 (2001).

### Fc region-comprising antibody

The term "Fc region-comprising antibody" refers to an antibody that comprises an Fc region. The C-terminal lysine (residue 447 according to the EU numbering system) or C-terminal glycine-lysine (residues 446-447) of the Fc region may be removed, for example, during purification of the antibody or by recombinant engineering of the nucleic acid encoding the antibody. Accordingly, a composition comprising an antibody having an Fc region according to this invention can comprise an antibody with G446-K447, with G446 and without K447, with all G446-K447 removed, or a mixture of three types of antibodies described above.

Fc regions with reduced Fc receptor-binding activity include Fc regions with reduced binding activity to any of Fcγ receptors FcγI, FcyIIA, FcyIIB, FcyIIIA, and/or FcyIIIB. The reduced binding activity to any of the Fcy receptors FcγI, FcyIIA, FcyIIB, FcyIIIA, and/or FcγIIIB can be assessed by using FACS and ELISA formats known to those skilled in the art as well as ALPHA screen (Amplified Luminescent Proximity Homogeneous Assay) and surface plasmon resonance (SPR)-based BIACORE method (Proc. Natl. Acad. Sci. USA (2006) 103(11), 4005-4010).

Antigen-binding molecules or antibodies comprising an Fc region with reduced Fc receptor-binding activity include antigen-binding molecules or antibodies with reduced effector function. Antigen-binding molecules or antibodies with reduced effector function include those with substitution of one or more of Fc region residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Certain antibody variants with increased or decreased binding to FcRs are described. (See, e.g., U.S. Patent No. 6,737,056; WO 2004/056312, and Shields et al., J. Biol. Chem. 9(2): 6591-6604 (2001).)

In certain embodiments, an antibody variant comprises an Fc region with one or more amino acid substitutions which improve ADCC, e.g., substitutions at positions 298, 333, and/or 334 of the Fc region (EU numbering of residues).

In some embodiments, alterations are made in the Fc region that result in altered (*i.e.,* either increased or decreased) C1q binding and/or Complement Dependent Cytotoxicity (CDC), e.g., as described in US Patent No. 6,194,551, WO 99/51642, and Idusogie et al. J. Immunol. 164: 4178-4184 (2000).

In some embodiments, Fc regions with reduced Fc receptor-binding activity include Fc mutants of the Fc regions illustrated herein.

### Framework

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

### Full length antibody

The terms "full length antibody," "intact antibody," and "whole antibody" are used herein interchangeably to refer to an antibody having a structure substantially similar to a native antibody structure or having heavy chains that contain an Fc region as defined herein.

### Host cell

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

### Human antibody

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

### Human consensus framework

A "human consensus framework" is a framework which represents the most commonly occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as in Kabat et al., Sequences of Proteins of Immunological Interest, Fifth Edition, NIH Publication 91-3242, Bethesda MD (1991), vols. 1-3. In one embodiment, for the VL, the subgroup is subgroup kappa I as in Kabat et al., *supra.* In one embodiment, for the VH, the subgroup is subgroup III as in Kabat et al., *supra.*

### Humanized antibody

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

### Hypervariable region

The term "hypervariable region," "HVR," or "CDR" as used herein refers to each of the regions of an antibody variable domain which are hypervariable in sequence ("complementarity determining regions" or "complementarity determining regions") and/or form structurally defined loops ("hypervariable loops") and/or contain the antigen-contacting residues ("antigen contacts"). Generally, antibodies comprise six CDRs: three in the VH (H1, H2, H3), and three in the VL (L1, L2, L3). Exemplary CDRs herein include:
(a) hypervariable loops occurring at amino acid residues 26-32 (L1), 50-52 (L2), 91-96 (L3), 26-32 (H1), 53-55 (H2), and 96-101 (H3) (Chothia and Lesk, J. Mol. Biol. 196:901-917 (1987));
(b) CDRs occurring at amino acid residues 24-34 (L1), 50-56 (L2), 89-97 (L3), 31-35b (H1), 50-65 (H2), and 95-102 (H3) (Kabat et al., Sequences of Proteins of Immunological Interest, 5th Ed. Public Health Service, National Institutes of Health, Bethesda, MD (1991));
(c) antigen contacts occurring at amino acid residues 27c-36 (L1), 46-55 (L2), 89-96 (L3), 30-35b (H1), 47-58 (H2), and 93-101 (H3) (MacCallum et al. J. Mol. Biol. 262: 732-745 (1996)); and
(d) combinations of (a), (b), and/or (c), including amino acid residues 46-56 (L2), 47-56 (L2), 48-56 (L2), 49-56 (L2), 26-35 (H1), 26-35b (H1), 49-65 (H2), 93-102 (H3), and 94-102 (H3).

Unless otherwise indicated, CDR residues and other residues in the variable domain (e.g., FR residues) are numbered herein according to Kabat et al., *supra.*

### Individual

An "individual," "subject," or "patient" is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In certain embodiments, the individual or subject is a human.

### Isolated antigen-binding molecule

An "isolated" antigen-binding molecule is one which has been separated from a component of its natural environment. In some embodiments, an antigen-binding molecule is purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC). For review of methods for assessment of antibody purity, see, e.g., Flatman et al., J. Chromatogr. B 848:79-87 (2007).

### Isolated nucleic acid

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

### Isolated nucleic acid encoding an antigen-binding molecule

"Isolated nucleic acid encoding an antigen-binding molecule" refers to one or more nucleic acid molecules encoding one or two or more polypeptide chains or fragments thereof of an antigen-binding molecule (in case of an antibody, antibody heavy and light chains or fragments thereof), including such nucleic acid molecule(s) in a single vector or separate vectors, and such nucleic acid molecule(s) present at one or more locations in a host cell.

### Monoclonal antibody

The term "monoclonal antibody" as used herein refers to an antibody obtained from a population of substantially homogeneous antibodies, i.e., the individual antibodies composing the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

### Package insert

The term "package insert" is used to refer to instructions customarily included in commercial packages of therapeutic products, that contain information about the indications, usage, dosage, administration, combination therapy, contraindications and/or warnings concerning the use of such therapeutic products.

### Amino acid sequence identity

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN, Megalign (DNASTAR) software, or GENETYX (registered trademark) (Genetyx Co., Ltd.). Those skilled in the art can determine appropriate parameters for aligning sequences, including any algorithms needed to achieve maximal alignment over the full length of the sequences being compared.

The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system (UNIX is a registered trademark), including digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y
where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

### Pharmaceutical formulation

The term "pharmaceutical formulation" refers to a preparation which is in such form as to permit the biological activity of an active ingredient contained therein to be effective, and which contains no additional components which are unacceptably toxic to a subject to which the formulation would be administered.

### Pharmaceutically acceptable carrier

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

### Treatment

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies of the invention are used to delay development of a disease or to slow the progression of a disease.

### Variable region

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (See, e.g., Kindt et al. Kuby Immunology, 6th ed., W.H. Freeman and Co., page 91 (2007).) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, e.g., Portolano et al., J. Immunol. 150:880-887 (1993); Clarkson et al., Nature 352:624-628 (1991).

### Vector

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

### Multispecific antibody

In certain embodiments, an antigen-binding molecule (or antibody) provided herein is a multispecific antibody, e.g. a bispecific antibody. Multispecific antibodies are monoclonal antibodies that have binding specificities for at least two different sites. Multispecific antibodies (e.g., bispecific antibodies) can be prepared as full length antibodies or antibody fragments.

Techniques for making multispecific antibodies include, but are not limited to, recombinant co-expression of two immunoglobulin heavy chain-light chain pairs having different specificities (see Milstein and Cuello, Nature 305: 537 (1983)), WO 93/08829, and Traunecker et al., EMBO J. 10: 3655 (1991)), and "knob-in-hole" engineering (see, e.g., U.S. Patent No. 5,731,168). Multi-specific antibodies may also be made by engineering electrostatic steering effects for making antibody Fc-heterodimeric molecules (WO 2009/089004A1); crosslinking two or more antibodies or fragments (see, US Patent No. 4,676,980, and Brennan et al., Science, 229: 81 (1985)); using leucine zippers to produce bi-specific antibodies (see, Kostelny et al., J. Immunol., 148(5): 1547-1553 (1992)); using "diabody" technology for making bispecific antibody fragments (see, Hollinger et al., Proc. Natl. Acad. Sci. USA, 90:6444-6448 (1993)); and using single-chain Fv (scFv) dimers (see, Gruber et al., J. Immunol., 152:5368 (1994)); and preparing trispecific antibodies as described, e.g., in Tutt et al. J. Immunol. 147: 60 (1991).

Engineered antibodies with three or more functional antigen binding sites, including "Octopus antibodies," are also included herein (see, e.g. US 2006/0025576A1).

The antigen-binding molecule or fragment herein also includes a "Dual Acting Fab" or "DAF" comprising an antigen binding site that binds to a specific antigen as well as another, different antigen (see, US 2008/0069820, for example).

### II. Anti-T cell antigen-binding molecules and pharmaceutical compositions

In one embodiment, "anti-T cell antigen-binding molecules" refer to antigen-binding molecules that bind to antigens on T cells, and include antigen-binding molecules that bind to T cell receptor complexes. In one embodiment, anti-T cell antigen-binding molecules are multispecific antigen-binding molecules. In one embodiment, anti-T cell antigen-binding molecules are bispecific antigen-binding molecules, preferably bispecific antibodies comprising "a domain comprising an antibody variable region having T cell receptor complex-binding activity" and "a domain comprising an antibody variable region having cancer antigen-binding activity". In one embodiment, the bispecific antibodies may have a structure of a single chain antibody, such as a structure in which antibody variable regions are linked by linkers. In one embodiment, anti-T cell antigen-binding molecules further comprise an Fc region with reduced Fcγ receptor-binding activity.

Furthermore, in one embodiment, a domain comprising an antibody variable region having T cell receptor complex-binding activity is preferably a domain comprising an antibody variable region having T cell receptor-binding activity, and more preferably a domain comprising an antibody variable region having CD3-binding activity. Furthermore, in one embodiment, the above "domain comprising an antibody variable region" is provided by one or more variable domains of an antibody, and preferably the domain comprising an antibody variable region comprises an antibody light chain variable region (VL) and an antibody heavy chain variable region (VH). Suitable examples of such domains comprising antibody variable regions include various antibody fragments such as "scFv (single chain Fv)", "single chain antibody", "Fv", "single chain Fv 2 (scFv2)", "Fab", and "F(ab')2".

### Domain comprising an antibody variable region having cancer antigen-binding activity

Herein, the "domain comprising an antibody variable region having cancer antigen-binding activity" refers to a portion of an antibody comprising a region that specifically binds to and is complementary to all or a part of a cancer antigen.

### Cancer-specific antigen

Herein, a "cancer-specific antigen" refers to an antigen expressed by cancer cells, which enables one to distinguish between cancer cells and healthy cells; and for example, it includes antigens that are expressed as cells become malignant, or abnormal sugar chains that appear on protein molecules or cell surface when cells become cancerous. Specific examples include GPC3; ALK receptor (pleiotrophin receptor); pleiotrophin; KS 1/4 pancreas carcinoma antigen; ovarian carcinoma antigen (CA125); prostatic acid phosphate; prostate-specific antigen (PSA); melanoma-associated antigen p97; melanoma antigen gp75; high molecular weight melanoma antigen (HMW-MAA); prostate-specific membrane antigen; carcinoembryonic antigen (CEA); polymorphic epithelial mucin antigen; human milk fat globule antigen; colorectal tumor-associated antigens such as CEA, TAG-72, CO17-1A, GICA 19-9, CTA-1, and LEA; Burkitt's lymphoma antigen-38.13; CD19; human B-lymphoma antigen-CD20; CD33; melanoma-specific antigens such as ganglioside GD2, ganglioside GD3, ganglioside GM2, and ganglioside GM3; tumor-specific transplantation type cell-surface antigen (TSTA); virus-induced tumor antigens including T antigen and envelope antigens of DNA tumor viruses and RNA tumor viruses; CEA of colon; oncofetal antigens such as 5T4 oncofetal trophoblast glycoprotein and bladder tumor oncofetal antigen; α-fetoprotein; differentiation antigens such as human lung carcinoma antigens L6 and L20; antigens of fibrosarcoma; human leukemia T cell antigen-Gp37; neoglycoprotein; sphingolipids; breast cancer antigens such as EGFR (epidermal growth factor receptor); NY-BR-16; NY-BR-16 and HER2 antigen (p185HER2); polymorphic epithelial mucin (PEM); malignant human lymphocyte antigen-APO-1; differentiation antigens such as I antigen found in fetal erythrocytes; primary endoderm I antigen found in adult erythrocytes; preimplantation embryos; I(Ma) found in gastric cancer; M18 and M39 found in mammary epithelium; SSEA-1, VEP8, VEP9, Myl, and VIM-D5 found in myeloid cells; D156-22 found in colorectal cancer; TRA-1-85 (blood group H); SCP-1 found in testis and ovarian cancer; C14 found in colon cancer; F3 found in lung cancer; AH6 found in gastric cancer; Y hapten; Ley found in embryonal carcinoma cells; TL5 (blood group A); EGF receptor found in A431 cells; E1 series (blood group B) found in pancreatic cancer; FC10.2 found in embryonal carcinoma cells; gastric cancer antigen; CO-514 (blood group Lea) found in adenocarcinomas; NS-10 found in adenocarcinomas; CO-43 (blood group Leb); G49 found in EGF receptor of A431 cells; MH2 (blood group ALeb/Ley) found in colon cancer; 19.9 found in colon cancer; gastric cancer mucins; T5A7 found in myeloid cells; R24 found in melanoma; 4.2, GD3, D1.1, OFA-1, GM2, OFA-2, GD2, and M1:22:25:8 found in embryonal carcinoma cells as well as SSEA-3 and SSEA-4 found in 4 to 8-cell stage embryos; subcutaneous T cell lymphoma antigen; MART-1 antigen; sialyl Tn (STn) antigen; colon cancer antigen NY-CO-45; lung cancer antigen NY-LU-12 variant A; adenocarcinoma antigen ART1; paraneoplastic associated brain-testis-cancer antigen (onconeuronal antigen MA2; paraneoplastic neuronal antigen); Neuro-oncological ventral antigen 2 (NOVA2); hemocyte carcinoma antigen gene 520; tumor-associated antigen CO-029; tumor-associated antigens MAGE-C1 (cancer/testis antigen CT7), MAGE-B 1 (MAGE-XP antigen), MAGE-B2 (DAM6), MAGE-2, MAGE-4a, MAGE-4b and MAGE-X2; Cancer-Testis Antigen (NY-EOS-1); YKL-40, fragments of any of the aforementioned polypeptides, or structures produced by modification thereof (for example, the above-mentioned modified phosphate group or sugar chain); EpCAM; EREG; CA19-9; CA15-3; sialyl SSEA-1(SLX); HER2; PSMA; CEA; and CLEC12A. Cancer-specific antigens which become targets of the cancer-specific antigen-binding domains of the present invention are, in particular, preferably those expressed on cell surface, and examples of such cancer-specific antigens include CD19, CD20, EGFR, HER2, EpCAM, and EREG.

### A domain comprising an antibody variable region having glypican 3 (GPC3)-binding activity

Herein, the phrase "a domain comprising an antibody variable region having glypican 3 (GPC3)-binding activity" refers to an antibody portion that comprises a region that specifically binds to the above-mentioned GPC3 protein, or to all or a portion of a partial peptide of the GPC3 protein, and is also complementary thereto.

Herein, glypican 3 (GPC3) is a protein that belongs to the glypican family, i.e., a group of heparan sulfate proteoglycans bound to cell surface *via* glycosylphosphatidylinositol (Filmus, J. Clin. Invest., 2001, 108, 497-501). Glypicans play an important role in cell proliferation, differentiation, and migration. GPC3 is expressed in 70% or more of hepatoma tissues obtained by surgical excision or biopsy, and is hardly or not at all expressed in neighboring nonneoplastic hepatic lesions and most adult tissues (Zhu-Zu-W, Gut, 2001, 48, 558-564, Yamauchi, Mod. Pathol., 2005, 18, 1591-1598).

### A domain comprising an antibody variable region having T-cell receptor complex-binding activity

Herein, the phrase "a domain comprising an antibody variable region having T-cell receptor complex-binding activity" refers to a T-cell receptor complex-binding antibody portion that comprises a region that specifically binds to all or a portion of a T-cell receptor complex and is also complementary thereto. The T-cell receptor complex may be a T-cell receptor itself, or an adaptor molecule constituting a T-cell receptor complex along with a T-cell receptor. CD3 is suitable as an adaptor molecule.

### A domain comprising an antibody variable region having T-cell receptor-binding activity

Herein, the phrase "a domain comprising an antibody variable region having T-cell receptor-binding activity" refers to a T-cell receptor-binding antibody portion that comprises a region that specifically binds to all or a portion of a T-cell receptor and is also complementary thereto.

The portion of a T cell receptor to which the domain of the present invention binds may be a variable region or a constant region, but an epitope present in the constant region is preferred. Examples of the constant region sequence include the T cell receptor α chain of RefSeq Accession No. CAA26636.1 (SEQ ID NO: 9), the T cell receptor β chain of RefSeq Accession No. C25777 (SEQ ID NO: 10), the T cell receptor γ1 chain of RefSeq Accession No. A26659 (SEQ ID NO: 11), the T cell receptor γ2 chain of RefSeq Accession No. AAB63312.1 (SEQ ID NO: 12), and the T cell receptor δ chain of RefSeq Accession No. AAA61033.1 (SEQ ID NO: 13).

### A domain comprising an antibody variable region having CD3-binding activity

Herein, the phrase "a domain comprising an antibody variable region having CD3-binding activity" refers to a CD3-binding antibody portion that comprises a region that specifically binds to all or a portion of CD3 and is also complementary thereto.

The domain comprising an antibody variable region having CD3-binding activity of the present invention may be any epitope-binding domain as long as the epitope exists in the γ-chain, δ-chain, or ε-chain sequence that constitutes human CD3. In the present invention, preferably, a domain comprising an anti-CD3 antibody light-chain variable region (VL) and an anti-CD3 antibody heavy-chain variable region (VH) that bind to an epitope present in the extracellular region of the ε chain of the human CD3 complex is suitably used. Besides the anti-CD3 antibody light chain variable region (VL) and anti-CD3 antibody heavy chain variable region (VH) described in the Examples, various known CD3-binding domains containing a CD3-binding antibody light chain variable region (VL) and a CD3-binding antibody heavy chain variable region (VH), and those of the OKT3 antibody (Proc. Natl. Acad. Sci. USA (1980) 77, 4914-4917) are suitably used as such domains. One may appropriately use an antibody variable region-containing domain derived from the anti-CD3 antibody having desired properties, which is obtained by immunizing a desired animal by the above-mentioned method using the γ-chain, δ-chain, or ε-chain constituting the human CD3. Human antibodies and properly humanized antibodies as described above may be appropriately used as the anti-CD3 antibody to give rise to the domain containing the antibody variable region having CD3-binding activity. Regarding the structure of the γ-chain, δ-chain, or ε-chain constituting CD3, their polynucleotide sequences are shown in SEQ ID NOs: 9 (NM_000073.2), 10 (NM_000732.4), and 11 (NM_000733.3), and their polypeptide sequences are shown in SEQ ID NOs: 12 (NP_000064.1), 13 (NP_000723.1), and 14 (NP_000724.1) (the RefSeq accession number is shown in parentheses).

Antibody variable region-containing domains in antigen binding molecules of the present invention may bind to the same epitope. Herein, the same epitope may be present in a protein comprising the amino acid sequence of SEQ ID NO: 2 or 14. Alternatively, antibody variable region-containing domains in antigen binding molecules of the present invention may bind to different epitopes, respectively. Herein, the different epitopes may be present in a protein comprising the amino acid sequence of SEQ ID NO: 2 or 14.

### Fc region with reduced Fcγ receptor-binding activity

In one embodiment, "Fcy receptor-binding activity is reduced" means, for example, that the binding activity of a test antigen-binding molecule is 50% or less, preferably 45% or less, 40% or less, 35% or less, 30% or less, 20% or less, or 15% or less, and particularly preferably 10% or less, 9% or less, 8% or less, 7% or less, 6% or less, 5% or less, 4% or less, 3% or less, 2% or less, or 1% or less than the binding activity of a control antigen-binding molecule.

Antigen-binding molecules comprising the Fc region of a monoclonal IgG1, IgG2, IgG3, or IgG4 antibody can be appropriately used as control antigen-binding molecules. Furthermore, when an antigen-binding molecule comprising an Fc region mutant of an antibody of a particular isotype is used as a test substance, the effect of the mutation of the mutant on the Fcy receptor-binding activity is assessed using as a control an antigen-binding molecule comprising an Fc region of the same isotype. As described above, antigen-binding molecules comprising an Fc region mutant whose Fcy receptor-binding activity has been judged to be reduced are appropriately prepared.

Such known mutants include, for example, mutants having a deletion of amino acids 231A-238S (EU numbering) (WO 2009/011941), as well as mutants C226S, C229S, P238S, (C220S) (J. Rheumatol (2007) 34, 11); C226S and C229S (Hum. Antibod. Hybridomas (1990) 1(1), 47-54); C226S, C229S, E233P, L234V, and L235A (Blood (2007) 109, 1185-1192).

Specifically, the preferred antigen-binding molecules include those comprising an Fc region with a substitution of the amino acid at position 220, 226, 229, 231, 232, 233, 234, 235, 236, 237, 238, 239, 240, 264, 265, 266, 267, 269, 270, 295, 296, 297, 298, 299, 300, 325, 327, 328, 329, 330, 331, or 332 (EU numbering) in the amino acids forming the Fc region of an antibody of a particular isotype. The isotype of antibody from which the Fc region originates is not particularly limited, and it is possible to use an appropriate Fc region derived from a monoclonal IgG1, IgG2, IgG3, or IgG4 antibody. It is preferable to use Fc regions derived from IgG1 antibodies.

Suitably used antigen-binding molecules include, for example, those comprising an Fc region which has any one of the substitutions shown below, whose positions are specified according to EU numbering (each number represents the position of an amino acid residue in the EU numbering; and the one-letter amino acid symbol before the number represents the amino acid residue before substitution, while the one-letter amino acid symbol after the number represents the amino acid residue before the substitution) in the amino acids forming the Fc region of IgG1 antibody:
(a) L234F, L235E, P331S;
(b) C226S, C229S, P238S;
(c) C226S, C229S;
(d) C226S, C229S, E233P, L234V, L235A;
(e) L234A, L235A or L235R, N297A;
(f) L235A or L235R, S239K, N297A
as well as those having an Fc region which has a deletion of the amino acid sequence at positions 231 to 238.

Furthermore, suitably used antigen-binding molecules also include those comprising an Fc region that has any one of the substitutions shown below, whose positions are specified according to EU numbering in the amino acids forming the Fc region of an IgG2 antibody:
(g) H268Q, V309L, A330S, and P331S;
(h) V234A;
(i) G237A;
(j) V234A and G237A;
(k) A235E and G237A;
(l) V234A, A235E, and G237A (each number represents the position of an amino acid residue in EU numbering; and the one-letter amino acid symbol before the number represents the amino acid residue before substitution, while the one-letter amino acid symbol after the number represents the amino acid residue before the substitution).

Furthermore, suitably used antigen-binding molecules also include those comprising an Fc region that has any one of the substitutions shown below, whose positions are specified according to EU numbering in the amino acids forming the Fc region of an IgG3 antibody:
(m) F241A;
(n) D265A;
(o) V264A (each number represents the position of an amino acid residue in EU numbering; and the one-letter amino acid symbol before the number represents the amino acid residue before substitution, while the one-letter amino acid symbol after the number represents the amino acid residue before the substitution).

Furthermore, suitably used antigen-binding molecules also include those comprising an Fc region that has any one of the substitutions shown below, whose positions are specified according to EU numbering in the amino acids forming the Fc region of an IgG4 antibody:
(p) L235A, G237A, and E318A;
(q) L235E;
(r) F234A and L235A (each number represents the position of an amino acid residue in EU numbering; and the one-letter amino acid symbol before the number represents the amino acid residue before substitution, while the one-letter amino acid symbol after the number represents the amino acid residue before the substitution).

Other preferred antigen-binding molecules include, for example, those comprising an Fc region in which any amino acid at position 233, 234, 235, 236, 237, 327, 330, or 331 (EU numbering) in the amino acids forming the Fc region of an IgG1 antibody is substituted with an amino acid of the corresponding position in EU numbering in the corresponding IgG2 or IgG4.

Other preferred antigen-binding molecules also include, for example, those comprising an Fc region in which any one or more of the amino acids at positions 234, 235, and 297 (EU numbering) in the amino acids forming the Fc region of an IgG1 antibody is substituted with other amino acids. The type of amino acid after substitution is not particularly limited; however, antigen-binding molecules comprising an Fc region in which any one or more of the amino acids at positions 234, 235, and 297 are substituted with alanine are particularly preferred.

Other preferred antigen-binding molecules also include, for example, those comprising an Fc region in which an amino acid at position 265 (EU numbering) in the amino acids forming the Fc region of an IgG1 antibody is substituted with another amino acid. The type of amino acid after substitution is not particularly limited; however, antigen-binding molecules comprising an Fc region in which the amino acid at position 265 is substituted with alanine are particularly preferred.

### Anti-glypican 3 (GPC3)/T-cell receptor complex bispecific antibody

In one embodiment, an anti-T cell antigen-binding molecule is a bispecific antibody comprising:
(1) a domain comprising an antibody variable region having glypican 3-binding activity;
(2) a domain comprising an antibody variable region having T-cell receptor complex-binding activity; and
(3) a domain comprising an Fc region with reduced Fcy receptor-binding activity.
In one embodiment, for the antibody L-chain variable regions contained in the antibody variable region having glypican 3-binding activity and the antibody variable region having T-cell receptor complex-binding activity of the present invention, it is preferable to obtain a common L chain that may provide a binding ability both to the H chain having glypican3-binding activity and to the H chain having T-cell receptor complex-binding activity, and to use this as the common L-chain variable region of the bispecific antigen-binding molecule.

Examples of a preferred antibody H-chain variable region of the present disclosure contained in the antibody variable region having glypican 3-binding activity comprises the antibody H-chain variable regions of Table 1, or antibody H-chain variable regions having CDR sequences whose CDR1, CDR2, and CDR3 amino acid sequences are the same as the CDR1, CDR2, and CDR3 amino acid sequences contained in the antibody H-chain variable regions of Table 1, or antibody H-chain variable regions which are functionally equivalent to the above-mentioned variable regions.

**[Table 1]**

| Sequence Name | SEQ ID NO: |
|---|---|
| H0000 | 40 |
| GCH003 | 170 |
| GCH005 | 171 |
| GCH006 | 172 |
| GCH007 | 173 |
| GCH008 | 174 |
| GCH010 | 175 |
| GCH012 | 176 |
| GCH013 | 177 |
| GCH014 | 178 |
| GCH015 | 179 |
| GCH016 | 180 |
| GCH019 | 181 |
| GCH022 | 182 |
| GCH023 | 183 |
| GCH025 | 184 |
| GCH026 | 185 |
| GCH027 | 186 |
| GCH029 | 187 |
| GCH032 | 188 |
| GCH034 | 189 |
| GCH035 | 190 |
| GCH039 | 191 |
| GCH040 | 192 |
| GCH042 | 193 |
| GCH043 | 194 |
| GCH045 | 195 |
| GCH053 | 196 |
| GCH054 | 197 |
| GCH055 | 198 |
| GCH056 | 199 |
| GCH057 | 200 |
| GCH059 | 201 |
| GCH060 | 202 |
| GCH061 | 203 |
| GCH062 | 204 |
| GCH064 | 205 |
| GCH065 | 206 |
| GCH066 | 207 |
| GCH067 | 208 |
| GCH068 | 209 |
| GCH073 | 210 |
| GCH094 | 211 |
| GCH098 | 212 |
| GCH099 | 213 |
| GCH100 | 214 |
| H0610 | 215 |

Examples of a preferred antibody variable region having T-cell receptor complex-binding activity in the present disclosure include antibody variable regions having T-cell receptor-binding activity. Of the T-cell receptors, CD3 is preferred, and CD3ε is particularly preferred. Examples of an antibody H-chain variable region contained in such antibody variable regions include the antibody H-chain variable regions of Table 2, antibody H-chain variable regions having CDR sequences whose CDR1, CDR2, and CDR3 amino acid sequences are the same as the CDR1, CDR2, and CDR3 amino acid sequences contained in the antibody H-chain variable regions of Table 2, and antibody H-chain variable regions that are functionally equivalent to the above-mentioned variable regions.

**[Table 2]**

| Sequence Name | SEQ ID NO |
|---|---|
| hCE115HA | 52 |
| CE115HA177 | 64 |
| CE115HA178 | 65 |
| CE115HA179 | 66 |
| CE115HA180 | 67 |
| hCE115HAa | 68 |
| TR01H006 | 69 |
| TR01H007 | 70 |
| TR01H008 | 71 |
| TR01H009 | 72 |
| TR01H010 | 73 |
| TR01H011 | 74 |
| TR01H012 | 75 |
| TR01H013 | 76 |
| TR01H014 | 77 |
| TR01H015 | 78 |
| TR01H016 | 79 |
| TR01H017 | 80 |
| TR01H018 | 81 |
| TR01H019 | 82 |
| TR01H020 | 83 |
| TR01H021 | 84 |
| TR01H022 | 85 |
| TR01H023 | 86 |
| TR01H024 | 87 |
| TR01H025 | 88 |
| TR01H026 | 89 |
| TR01H027 | 90 |
| TR01H028 | 91 |
| TR01H029 | 92 |
| TR01H030 | 93 |
| TR01H031 | 94 |
| TR01H032 | 95 |
| TR01H033 | 96 |
| TR01H034 | 97 |
| TR01H035 | 98 |

| Sequence Name | SEQ ID NO: |
|---|---|
| TR01H036 | 99 |
| TR01H037 | 100 |
| TR01H038 | 101 |
| TR01H039 | 102 |
| TR01H040 | 103 |
| TR01H041 | 104 |
| TR01H042 | 105 |
| TR01H043 | 106 |
| TR01H044 | 107 |
| TR01H045 | 108 |
| TR01H046 | 109 |
| TR01H047 | 110 |
| TR01H048 | 111 |
| TR01H049 | 112 |
| TR01H050 | 113 |
| TR01H051 | 114 |
| TR01H052 | 115 |
| TR01H053 | 116 |
| TR01H054 | 117 |
| TR01H055 | 118 |
| TR01H056 | 119 |
| TR01H057 | 120 |
| TR01H058 | 121 |
| TR01H061 | 122 |
| TR01H062 | 123 |
| TR01H063 | 124 |
| TR01H064 | 125 |
| TR01H065 | 126 |
| TR01H066 | 127 |
| TR01H067 | 128 |
| TR01H068 | 129 |
| TR01H069 | 130 |
| TR01H070 | 131 |
| TR01H071 | 132 |
| TR01H072 | 133 |
| TR01H073 | 134 |
| TR01H074 | 135 |
| TR01H075 | 136 |
| TR01H076 | 137 |
| TR01H077 | 138 |
| TR01H079 | 139 |
| TR01H080 | 140 |
| TR01H081 | 141 |
| TR01H082 | 142 |
| TR01H083 | 143 |
| TR01H084 | 144 |
| TR01H090 | 145 |
| TR01H091 | 146 |
| TR01H092 | 147 |
| TR01H093 | 148 |
| TR01H094 | 149 |
| TR01H095 | 150 |
| TR01H096 | 151 |
| TR01H097 | 152 |
| TR01H098 | 153 |
| TR01H099 | 154 |
| TR01H100 | 155 |
| TR01H101 | 156 |
| TR01H102 | 157 |
| TR01H103 | 158 |
| TR01H104 | 159 |
| TR01H105 | 160 |
| TR01H106 | 161 |
| TR01H107 | 162 |
| TR01H108 | 163 |
| TR01H109 | 164 |
| TR01H110 | 165 |
| TR01H111 | 166 |
| TR01H112 | 167 |
| TR01H113 | 168 |
| TR01H114 | 169 |
| TR01H001 | 420 |
| TR01H002 | 421 |
| TR01H003 | 422 |
| TR01H004 | 423 |
| rCE115H | 424 |
| CE115HA121 | 425 |
| CE115HA122 | 426 |
| CE115HA124 | 427 |
| CE115HA192 | 428 |
| CE115HA236 | 429 |
| CE115HA251 | 430 |
| CE115HA252 | 431 |

The relationship between the CDR regions of the amino acid residues constituting the antibody H chain amino acid sequence and Kabat numbering is as shown in Fig. 2.

Examples of the common L-chain variable region to be used in the present invention include the L-chain variable regions of Table 3, antibody L-chain variable regions having CDR sequences whose CDR1, CDR2, and CDR3 amino acid sequences are the same as the CDR1, CDR2, and CDR3 amino acid sequences contained in the antibody L-chain variable regions of Table 3, and antibody L-chain variable regions that are functionally equivalent to the above-mentioned variable regions.

**[Table 3]**

| Sequence Name | SEQ ID NO: |
|---|---|
| L0000 | 53 |
| L0002 | 217 |
| L0003 | 218 |
| L0006 | 219 |
| L0007 | 220 |
| L0008 | 221 |
| L0009 | 222 |
| L0011 | 223 |
| L0012 | 224 |
| L0013 | 225 |
| L0014 | 226 |
| L0015 | 227 |
| L0016 | 228 |
| L0032 | 229 |
| L0038 | 230 |
| L0039 | 231 |
| L0041 | 232 |
| L0042 | 233 |
| L0043 | 234 |
| L0044 | 235 |
| L0045 | 236 |
| L0046 | 237 |
| L0047 | 238 |
| L0062 | 239 |
| L0063 | 240 |
| L0064 | 241 |
| L0065 | 242 |
| L0066 | 243 |
| L0069 | 244 |
| L0075 | 245 |
| L0079 | 246 |
| L0082 | 247 |
| L0085 | 248 |
| L0089 | 249 |
| L0090 | 250 |
| L0091 | 251 |
| L0093 | 252 |
| L0104 | 253 |
| L0106 | 254 |
| L0107 | 255 |
| L0109 | 256 |
| L0113 | 257 |
| L0115 | 258 |
| L0117 | 259 |
| L0120 | 260 |
| L0122 | 261 |
| L0123 | 262 |
| L0124 | 263 |
| L0125 | 264 |
| L0126 | 265 |
| L0127 | 266 |
| L0129 | 267 |
| L0132 | 268 |
| L0134 | 269 |
| L0136 | 270 |
| L0137 | 271 |
| L0138 | 272 |
| L0139 | 273 |
| L0140 | 274 |
| L0141 | 275 |
| L0143 | 276 |
| L0144 | 277 |
| L0145 | 278 |
| L0147 | 279 |
| L0148 | 280 |
| L0149 | 281 |
| L0151 | 282 |
| L0152 | 283 |
| L0154 | 284 |
| L0155 | 285 |
| L0157 | 286 |
| L0160 | 287 |
| L0161 | 288 |
| L0163 | 289 |
| L0167 | 290 |
| L0168 | 291 |
| L0173 | 292 |
| L0175 | 293 |
| L0180 | 294 |
| L0181 | 295 |
| L0186 | 296 |
| L0187 | 297 |
| L0200 | 298 |
| L0201 | 299 |
| L0202 | 300 |
| L0203 | 301 |
| L0204 | 302 |
| L0205 | 303 |
| L0206 | 304 |
| L0207 | 305 |
| L0208 | 306 |
| L0209 | 307 |
| L0210 | 308 |
| L0211 | 309 |
| L0212 | 310 |
| L0213 | 311 |
| L0214 | 312 |
| L0215 | 313 |
| L0216 | 314 |
| L0217 | 315 |
| L0218 | 316 |
| L0219 | 317 |
| L0220 | 318 |
| L0222 | 319 |
| L0223 | 320 |
| L0224 | 321 |
| L0226 | 322 |
| L0227 | 323 |
| L0228 | 324 |
| L0229 | 325 |
| L0230 | 326 |
| L0231 | 327 |
| L0232 | 328 |
| L0233 | 329 |
| L0234 | 330 |
| L0235 | 331 |
| L0236 | 332 |
| L0237 | 333 |
| L0238 | 334 |
| L0239 | 335 |
| L0240 | 336 |
| L0241 | 337 |
| L0242 | 338 |
| L0243 | 339 |
| L0246 | 340 |
| L0247 | 341 |
| L0248 | 342 |
| L0249 | 343 |
| L0250 | 344 |
| L0258 | 345 |
| L0259 | 346 |
| L0260 | 347 |
| L0261 | 348 |
| L0262 | 349 |
| L0263 | 350 |
| L0264 | 351 |
| L0265 | 352 |
| L0266 | 353 |
| L0267 | 354 |
| L0268 | 355 |
| L0269 | 356 |
| L0270 | 357 |
| L0271 | 358 |
| L0272 | 359 |

The relationship between the CDR regions of the amino acid residues constituting the antibody L-chain amino acid sequence and Kabat numbering is as shown in Fig. 4.

In a preferred embodiment, examples of a combination of the antibody variable region having glypican 3-binding activity and the antibody variable region having T-cell receptor complex binding activity include the combinations of antibody H-chain variable regions shown in Table 4, combinations of antibody H-chain variable regions having CDR sequences whose CDR1, CDR2, and CDR3 amino acid sequences are the same as the CDR1, CDR2, and CDR3 amino acid sequences contained in the antibody H-chain variable regions of Table 4, and combinations of antibody H-chain variable regions functionally equivalent to these variable regions.

**[Table 4]**

| GPC3 side / T cell receptor complex side | SEQ ID NO: |
|---|---|
| H0000/hCE115HA | 40/52 |
| H0000/CE115HA251 | 40/430 |
| H0000/CE115HA236 | 40/429 |
| H0000/TR01 H002 | 40/421 |
| H0000/CE115HA122 | 40/426 |
| H0610/rCE115H | 215/424 |
| H0610/TR01 H040 | 215/103 |
| H0610/TR01H061 | 215/122 |
| H0610/TR01 H068 | 215/129 |
| H0610/TR01H071 | 215/132 |
| GCH054/TR01H067 | 197/128 |
| GCH094/TR01H082 | 211/142 |
| GCH094/TR01H084 | 211/144 |
| GCH065/TR01H084 | 206/144 |
| GCH065/TR01H082 | 206/142 |
| GCH094/TR01H109 | 211/164 |
| GCH065/TR01H109 | 206/164 |
| GCH094/TR01H113 | 211/168 |
| GCH065/TR01H113 | 206/168 |

A preferred common L chain for the above combinations of an antibody variable region having glypican 3-binding activity and an antibody variable region having T-cell receptor complex binding activity includes, for example, L0000, L0011, L0201, L0203, L0204, L0206, L0208, L0209, L0211, L0212, L0222, and common L chains having CDR sequences whose CDR1, CDR2, and CDR3 amino acid sequences are identical to the CDR1, CDR2, and CDR3 amino acid sequences of these common L chains. Specific preferred combinations include, for example, the combinations of antibody H-chain variable regions and a common L chain shown in Table 5, combinations of antibody variable regions having CDR sequences whose CDR1, CDR2, and CDR3 amino acid sequences are identical to the amino acid sequences of CDR1, CDR2, and CDR3 carried by the antibody variable regions and a common L chain of Table 5, and combinations of antibody H-chain variable regions and a common L chain functionally equivalent to these variable regions.

**[Table 5]**

| GPC3 side / T cell receptor complex side / common L chain | SEQ ID NO: |
|---|---|
| H0610/rCE115H/L0000 | 215/424/53 |
| H0610/TR01H040/L0000 | 215/103/53 |
| H0610/TR01H040/L0201 | 215/103/299 |
| H0610/TR01H040/L0203 | 215/103/301 |
| H0610/TR01H040/L0204 | 215/103/302 |
| H0610/TR01H040/L0206 | 215/103/304 |
| H0610/TR01H040/L0208 | 215/103/306 |
| H0610/TR01H040/L0209 | 215/103/307 |
| H0610/TR01H040/L0211 | 215/103/309 |
| H0610/TR01H061/L0000 | 215/122/53 |
| H0610/TR01H068/L0000 | 215/129/53 |
| H0610/TR01H071/L0000 | 215/132/53 |
| GCH054/TR01H067/L0201 | 197/128/299 |
| GCH054/TR01H067/L0212 | 197/128/310 |
| GCH054/TR01H067/L0222 | 197/128/319 |
| GCH054/TR01H067/L0000 | 197/128/53 |
| GCH094/TR01H082/L0201 | 211/142/299 |
| GCH094/TR01H082/L0011 | 211/142/223 |
| GCH094/TR01H084/L0011 | 211/144/223 |
| GCH065/TR01H084/L0011 | 206/144/223 |
| GCH065/TR01H082/L0011 | 206/142/223 |
| GCH094/TR01H109/L0011 | 211/164/223 |
| GCH065/TR01H109/L0011 | 206/164/223 |
| GCH094/TR01H113/L0011 | 211/168/223 |
| GCH065/TR01H113/L0011 | 206/168/223 |

The Fc region comprised in the anti-T cell antigen-binding molecule (preferably the bispecific antibody) is not particularly limited as long as it is an Fc region having reduced Fcy receptor-binding activity, but examples of a preferred Fc region include a combination of the Fc-region portion of E22Hh and the Fc-region portion of E22Hk, a combination of the Fc-region portion of E2702GsKsc and the Fc-region portion of E2704sEpsc, and a combination of the Fc-region portion of E2702sKsc and the Fc-region portion of E2704sEpsc.

### ERY974

In one embodiment, the anti-T cell antigen-binding molecule of the present disclosure is ERY974. ERY974 is a bispecific antibody comprising (1) a domain comprising an antibody variable region having glypican 3-binding activity, (2) a domain comprising an antibody variable region having T cell receptor complex-binding activity, and (3) a domain comprising an Fc region with reduced Fcγ receptor-binding activity. This bispecific antibody comprises TR01H113 (SEQ ID NO: 168) as the CD3-side heavy chain variable region, E2702sKsc (SEQ ID NO: 62) as the CD3-side heavy chain constant region, GCH065 (SEQ ID NO: 206) as the GPC3-side heavy chain variable region, E2704sEpsc (SEQ ID NO: 61) as the GPC3-side heavy chain constant region, L0011 (SEQ ID NO: 223) as the common light chain variable region, and k0 (SEQ ID NO: 63) as the common light chain constant region. In another embodiment, of ERY974, the CD3-side heavy chain comprises the amino acid sequence of SEQ ID NO: 402, the GPC3-side heavy chain comprises the amino acid sequence of SEQ ID NO: 385, and the common light chain comprises the amino acid sequence of SEQ ID NO: 410, respectively.

In one embodiment, amino acids contained in the amino acid sequences of the present disclosure may be subjected to post-translational modification. Modification well-known to those skilled in the art is, for example, conversion of an N-terminal glutamine residue (Q) to pyroglutamic acid (pGlu) by pyroglutamylation. Even when amino acids are post-translationally modified this way, they are, of course, included in the amino acid sequences described in the present disclosure.

Other preferred anti-T cell antigen-binding molecules in the present disclosure include bispecific antibodies having an antibody variable region having glypican 3-binding activity and an antibody variable region having CD3ε-binding activity. More preferably, the cytotoxic activity is equal to or higher than that of the bispecific antibody GPC3_ERY22_rCE115 (disclosed in Example 1 of WO2015156268). Examples of such bispecific antibodies include the bispecific antibody having an H chain and an L chain described in Example 3 (Table 13) of WO2015156268, or a bispecific antibody that binds to an epitope overlapping with an epitope to which the antibody binds and has an Fc region with reduced Fcγ receptor-binding activity.

In the present disclosure, the phrase "functionally equivalent" means that the binding affinities for an antigen are equivalent, or alternatively, it means that the cytotoxic activities against glypican 3-expressing cells or tissues containing these cells are equivalent when it is used as a bispecific antibody. The binding affinity and cytotoxic activity can be measured based on the description herein. The cells used for measurement of cytotoxic activity may be the desired GPC3-expressing cells or a desired tissue containing these cells, and for example, PC-10 or NCI-H446 which are GPC3-expressing human cancer cell lines can be used. Regarding the antibody constant regions, the phrase may mean that the decreases in Fcy receptor-binding activity are equivalent.

For example, an antibody H-chain variable region functionally equivalent to the antibody H chain variable region described herein (i.e., the original H chain variable region) means that this region has the same binding affinity when it is combined with the antibody L-chain variable region described herein which forms a pair with the original H chain, or alternatively that the region has the same cytotoxic activity towards glypican 3-expressing cells or a tissue containing these cells when used for a bispecific antibody. Furthermore, an antibody L-chain variable region functionally equivalent to the antibody L-chain variable region described herein (i.e., the original L-chain variable region) means that this region has the same binding affinity when it is combined with the antibody H-chain variable region described herein which forms a pair with the original L chain, or alternatively that the region has the same cytotoxic activity towards glypican 3-expressing cells or a tissue containing these cells when used for a bispecific antibody.

The term "equivalent" does not necessarily have to mean the same degree of activity, and the activity may be enhanced. Specifically, for antigen-binding affinity, examples include the case where the value (KD value / parent KD value) obtained by comparison to the binding affinity of the antibody variable region serving as the control (parent KD value) is 1.5 or less. The value of KD value / parent KD value is preferably 1.3 or less, more preferably 1.2 or less, 1.1 or less, 1.0 or less, 0.9 or less, 0.8 or less, 0.7 or less, 0.6 or less, or 0.5 or less. While there is no lower limit, examples include 10⁻¹, 10⁻², 10⁻³, 10⁻⁴, 10⁻⁵, or 10⁻⁶. More specifically, in the present invention, the value of KD value / parent KD value is preferably 10⁻⁶ to 1.5 x 10⁻⁰, more preferably 10⁻⁶ to 10⁻¹, even more preferably 10⁻⁶ to 10⁻², and yet even more preferably 10⁻⁶ to 10⁻³. For cytotoxic activity, examples include the case where the value (cell growth inhibition rate / parent cell growth inhibition rate) obtained by comparison to the cell growth inhibition rate of the bispecific antibody serving as the control (parent cell growth inhibition rate) is 0.7 or more. The concentration of the added multispecific antigen-binding molecule can be determined appropriately, but is preferably, for example, 0.01 nM, 0.05 nM, 0.1 nM, 0.5 nM, or 1 nM; and preferably, measurements are taken at 0.05 nM or 0.1 nM. The value for cell growth inhibition rate / parent cell growth inhibition rate is preferably 0.8 or higher, more preferably 0.9 or higher, 1.0 or higher, 1.2 or higher, 1.5 or higher, 2 or higher, 3 or higher, 5 or higher, 10 or higher, or 20 or higher. While there is no upper limit, the value may be 10, 10², 10³, 10⁴, 10⁵, or 10⁶.

Furthermore, for cytotoxic activity, examples include the case where the value (concentration for 50% inhibition of cell growth / parent concentration for 50% inhibition of cell growth) obtained by comparison to the concentration of the original bispecific antibody for 50% inhibition of cell growth (parent concentration for 50% inhibition of cell growth) is 1.5 or less. Concentration for 50% growth inhibition refers to the concentration of the multispecific antigen-binding molecule necessary for reducing the cell proliferation rate to one half compared to when the multispecific antigen-binding molecule is not added. The value of "concentration for 50% inhibition of cell growth / parent concentration for 50% inhibition of cell growth" is preferably 1.3 or less, more preferably 1.2 or less, 1.1 or less, 1.0 or less, 0.9 or less, 0.8 or less, 0.7 or less, 0.6 or less, or 0.5 or less. While there is no lower limit, the value may be, for example, 10⁻¹, 10⁻², 10⁻³, 10⁻⁴, 10⁻⁵, or 10⁻⁶. Specifically, the value is preferably 10⁻⁶ to 1.5 x 10⁻⁰, more preferably 10⁻⁶ to 10⁻¹, even more preferably 10⁻⁶ to 10⁻², and yet even more preferably 10⁻⁶ to 10⁻³.

Regarding the domain comprising an antibody variable region having GPC3-binding activity, the KD value towards GPC3 (for example, human GPC3) may be, for example, 5 x 10⁻⁹ M or less, preferably 4 x 10⁻⁹ M or less, such as 3 x 10⁻⁹ M or less, 2 x 10⁻⁹ M or less, 1 x 10⁻⁹ M or less, 8 x 10⁻¹⁰ M or less, 5 x 10⁻¹⁰ M or less, 4 x 10⁻¹⁰ M or less, 3 x 10⁻¹⁰ M or less, 2 x 10⁻¹⁰ M or less, 1 x 10⁻¹⁰ M or less, 8 x 10⁻¹¹ M or less, 5 x 10⁻¹¹ M or less, 4 x 10⁻¹¹ M or less, 3 x 10⁻¹¹ M or less, 2 x 10⁻¹¹ M or less, 1 x 10⁻¹¹ M or less, 8 x 10⁻¹² M or less, 5 x 10⁻¹² M or less, 4 x 10⁻¹² M or less, 3 x 10⁻¹² M or less, 2 x 10⁻¹² M or less, 1 x 10⁻¹² M or less, 8 x 10⁻¹³ M or less, 5 x 10⁻¹³ M or less, 4 x 10⁻¹³ M or less, 3 x 10⁻¹³ M or less, 2 x 10⁻¹³ M or less, or 1 x 10⁻¹³ M or less.

Regarding the domain comprising an antibody variable region having T-cell receptor complex-binding activity, the KD value towards a human T-cell receptor complex such as a human T cell receptor, or more specifically for example human CD3ε chain may be, for example, 2 x 10⁻⁷ M or less, preferably 1.5 x 10⁻⁷ M or less, such as 1.4 x 10⁻⁷ M or less, 1.3 x 10⁻⁷ M or less, 1.2 x 10⁻⁷ M or less, 1 × 10⁻⁷ M or less, 3 x 10⁻⁸ M or less, 2 x 10⁻⁸ M or less, 1 × 10⁻⁸ M or less, 8 x 10⁻⁹ M or less, 5 x 10⁻⁹ M or less, 4 x 10⁻⁹ M or less, 3 x 10⁻⁹ M or less, 2 x 10⁻⁹ M or less, 1 × 10⁻⁹ M or less, 8 × 10⁻¹⁰ M or less, 5 × 10⁻¹⁰ M or less, 4 × 10⁻¹⁰ M or less, 3 × 10⁻¹⁰ M or less, 2 × 10⁻¹⁰ M or less, 1 × 10⁻¹⁰ M or less, 8 × 10⁻¹¹ M or less, 5 × 10⁻¹¹ M or less, 4 × 10⁻¹¹ M or less, 3 × 10⁻¹¹ M or less, 2 × 10⁻¹¹ M or less, 1 × 10⁻¹¹ M or less, 8 × 10⁻¹² M or less, 5 × 10⁻¹² M or less, 4 × 10⁻¹² M or less, 3 × 10⁻¹² M or less, 2 × 10⁻¹² M or less, or 1 × 10⁻¹² M or less.

The bispecific antibodies of the present disclosure preferably have KD values toward human GPC3 and human T-cell receptor complex (for example, human CD3ε chain) that are 5 × 10⁻⁹ M or less and 2 × 10⁻⁷ M or less, respectively, and more preferably 1 × 10⁻⁹ M or less and 5 × 10⁻⁸ M or less, respectively.

In the present invention, antibody variable regions that are "functionally equivalent" are not particularly limited as long as they are antibody H-chain and/or antibody L-chain variable regions that satisfy the above-described conditions. Examples of such antibody variable regions include regions produced by introducing substitution, deletion, addition, and/or insertion of one or more amino acids (for example, 1, 2, 3, 4, 5, or 10 amino acids) into the amino acid sequences of the variable regions of Tables 1 to 3 mentioned above. A method well known to those skilled in the art for introducing one or more amino-acid substitutions, deletions, additions, and/or insertions into an amino acid sequence is a method of introducing mutations into proteins. For example, those skilled in the art can prepare variable regions that are functionally equivalent to the antibody variable regions having the above-mentioned functions by appropriately introducing mutations into amino acid sequences using methods such as site-directed mutagenesis (Hashimoto-Gotoh, T., Mizuno, T., Ogasahara, Y., and Nakagawa, M. (1995) An oligodeoxyribonucleotide-directed dual amber method for site-directed mutagenesis. Gene 152, 271-275; Zoller, M.J., and Smith, M. (1983) Oligonucleotide-directed mutagenesis of DNA fragments cloned into M13 vectors. Methods Enzymol. 100, 468-500; Kramer, W., Drutsa, V., Jansen, H.W., Kramer, B., Pflugfelder, M., and Fritz, H.J. (1984) The gapped duplex DNA approach to oligonucleotide-directed mutation construction. Nucleic Acids Res. 12, 9441-9456; Kramer, W., and Fritz, H.J. (1987) Oligonucleotide-directed construction of mutations via gapped duplex DNA Methods. Enzymol. 154, 350-367; and Kunkel, T.A. (1985) Rapid and efficient site-specific mutagenesis without phenotypic selection. Proc Natl Acad. Sci. USA. 82, 488-492).

When an amino acid residue is altered, the amino acid is preferably mutated into a different amino acid(s) that conserves the properties of the amino acid side-chain. Examples of amino-acid side chain properties are: hydrophobic amino acids (A, I, L, M, F, P, W, Y, and V), hydrophilic amino acids (R, D, N, C, E, Q, G, H, K, S, and T), amino acids containing aliphatic side chains (G, A, V, L, I, and P), amino acids containing hydroxyl group-containing side chains (S, T, and Y), amino acids containing sulfur atom-containing side chains (C and M), amino acids containing carboxylic acid- and amide-containing side chains (D, N, E, and Q), amino acids containing basic side chains (R, K, and H), and amino acids containing aromatic side chains (H, F, Y, and W) (amino acids are represented by one-letter codes in parentheses). Amino acid substitutions within each of these groups are called conservative substitutions. It is already known that a polypeptide containing a modified amino acid sequence in which one or more amino acid residues in a given amino acid sequence are deleted, added, and/or substituted with other amino acids can retain the original biological activity (Mark, D. F. et al., Proc. Natl. Acad. Sci. USA; (1984) 81: 5662-6; Zoller, M. J. and Smith, M., Nucleic Acids Res. (1982) 10: 6487-500; Wang, A. et al., Science (1984) 224: 1431-3; Dalbadie-McFarland, G. et al., Proc. Natl. Acad. Sci. USA (1982) 79: 6409-13). Variable regions of the present invention containing such amino acid modifications have an amino acid sequence identity of at least 70%, more preferably at least 75%, even more preferably at least 80%, still more preferably at least 85%, yet more preferably at least 90%, and most preferably at least 95%, with the amino acid sequence of the CDR sequences, FR sequences, or whole variable regions of the variable region prior to modification. Herein, sequence identity is defined as the percentage of residues identical to those in the original amino acid sequence of the H-chain variable region or L-chain variable region determined after the sequences are aligned, and gaps are appropriately introduced to maximize the sequence identity as necessary. The identity of amino acid sequences can be determined by the method described below.

Furthermore, a "functionally equivalent antibody variable region" can be obtained, for example, from nucleic acids that hybridize under stringent conditions with nucleic acids comprising a nucleotide sequence encoding the amino acid sequence of a variable region in Tables 1 to 3 mentioned above. Stringent hybridization conditions for isolating a nucleic acid that hybridizes under stringent conditions with a nucleic acid comprising a nucleotide sequence encoding the amino acid sequence of a variable region include, for example, the conditions of 6 M urea, 0.4% SDS, 0.5x SSC, and 37°C, or hybridization conditions with a stringency equivalent thereto. Isolation of nucleic acids with a much higher homology can be expected with more stringent conditions, for example, the conditions of 6 M urea, 0.4% SDS, 0.1x SSC, and 42°C. The washing conditions following the hybridization are, for example, washing using 0.5x SSC (1x SSC is 0.15 M NaCl and 0.015 M sodium citrate at pH7.0) and 0.1% SDS at 60°C, more preferably washing using 0.2x SSC and 0.1% SDS at 60°C, even more preferably washing using 0.2x SSC and 0.1% SDS at 62°C, yet even more preferably washing using 0.2x SSC and 0.1% SDS at 65°C, and still more preferably washing using 0.1x SSC and 0.1% SDS at 65°C. The sequences of the isolated nucleic acids can be determined by the known methods described below. The overall nucleotide sequence homology of the isolated nucleic acid is at least 50% or higher, preferably 70% or higher, and more preferably 90% or higher (for example, 95%, 96%, 97%, 98%, 99%, or higher) sequence identity.

Nucleic acids that hybridize under stringent conditions to a nucleic acid comprising a nucleotide sequence encoding the amino acid sequence of a variable region can also be isolated by using, instead of the above-described methods using hybridization techniques, gene amplification methods such as polymerase chain reaction (PCR) that uses primers synthesized based on information of the nucleotide sequence encoding the variable-region amino acid sequence.

The identity of one nucleotide sequence or amino acid sequence to another can be determined using the algorithm BLAST, by Karlin and Altschul (Proc. Natl. Acad. Sci. USA (1993) 90: 5873-7). Programs called BLASTN and BLASTX were developed based on this algorithm (Altschul et al., J. Mol. Biol. (1990) 215: 403-10). To analyze nucleotide sequences according to BLASTN based on BLAST, the parameters are set, for example, as score = 100 and wordlength = 12. On the other hand, parameters used for the analysis of amino acid sequences by BLASTX based on BLAST include, for example, score = 50 and wordlength = 3. Default parameters for each program are used when using the BLAST and Gapped BLAST programs. Specific techniques for such analyses are known in the art (see the website of the National Center for Biotechnology Information (NCBI), Basic Local Alignment Search Tool (BLAST); http://www.ncbi.nlm.nih.gov).

### Pharmaceutical compositions comprising an anti-T cell antigen-binding molecule

From another viewpoint, the present disclosure provides pharmaceutical compositions comprising as the active ingredient an anti-T cell antigen-binding molecule, preferably a bispecific antibody that comprises: (1) a domain comprising an antibody variable region having glypican 3-binding activity, (2) a domain comprising an antibody variable region having T-cell receptor complex-binding activity, and (3) a domain comprising an Fc region with reduced binding activity towards an Fcy receptor. Furthermore, the present disclosure relates to pharmaceutical compositions that induce cell injury, which comprise the anti-T cell antigen-binding molecule as an active ingredient. Pharmaceutical compositions of the present disclosure which induce the described cell injury, particularly T-cell-dependent cellular cytotoxicity, are preferably administered to an individual suffering from a disease for which the activities are needed for prevention or treatment, or an individual in which the disease is possible to relapse.

As specifically described above, human glypican 3 (GPC3) is a cancer antigen specifically expressed in cancer cells. Therefore, pharmaceutical compositions comprising as the active ingredient an anti-T cell antigen-binding molecule composed of a domain comprising an antibody variable region having glypican 3-binding activity are preferably administered to an individual suffering from cancer or an individual in which the cancer may relapse with the aim of either or both of treatment and prevention of the cancer. More specifically, the present disclosure provides pharmaceutical compositions comprising as the active ingredient a bispecific antibody that comprises (1) a domain comprising an antibody variable region having glypican 3-binding activity, (2) a domain comprising an antibody variable region having T-cell receptor complex-binding activity, and (3) a domain comprising an Fc region with reduced binding activity towards an Fcy receptor; wherein the pharmaceutical compositions are for administration with a VEGF inhibitor, and are for either or both of the treatment and prevention of cancer; and wherein the pharmaceutical compositions are for the purpose of any one or a combination selected from preventing, alleviating, and treating either of both of CRS and cytokine release resulting from administration of the pharmaceutical compositions.

Furthermore, the present disclosure relates to production of pharmaceutical compositions of a bispecific antibody that comprises (1) a domain comprising an antibody variable region having glypican 3-binding activity, (2) a domain comprising an antibody variable region having T-cell receptor complex-binding activity, and (3) a domain comprising an Fc region with reduced binding activity towards an Fcy receptor, wherein the compositions are administered with a VEGF inhibitor, and are for either or both of the treatment and prevention of cancer, and wherein the pharmaceutical compositions are for the purpose of any one or a combination selected from preventing, alleviating, and treating either or both of CRS and cytokine release resulting from administration of the pharmaceutical compositions.

Alternatively, the present disclosure relates to methods for either or both of treating and preventing cancer by administering, with a VEGF inhibitor, a bispecific antibody that comprises (1) a domain comprising an antibody variable region having glypican 3-binding activity, (2) a domain comprising an antibody variable region having T-cell receptor complex-binding activity, and (3) a domain comprising an Fc region with reduced binding activity towards an Fcy receptor, wherein the methods are for the purpose of any one or a combination selected from preventing, alleviating, and treating either or both of CRS and cytokine release resulting from administration of the pharmaceutical compositions.

Furthermore, the present disclosure provides uses of a bispecific antibody, administered with a VEGF inhibitor, which comprises (1) a domain comprising an antibody variable region having glypican 3-binding activity, (2) a domain comprising an antibody variable region having T-cell receptor complex-binding activity, and (3) a domain comprising an Fc region with reduced binding activity towards an Fcy receptor in either or both of the treatment and prevention of cancer, wherein the VEGF inhibitor is used for the purpose of any one or a combination selected from preventing, alleviating, and treating either or both of CRS and cytokine release resulting from administration of the bispecific antibody.

Accordingly, in a preferred embodiment of the present disclosure, a step of identifying a subject suffering from a GPC3-expressing cancer can be included before administering the pharmaceutical composition. Techniques for sampling cancer tissues from living bodies, identifying cancer cells included in the sampled tissues, and determining their levels of GPC3 expression are well known. If necessary, the level of GPC expression in the cells used as the sample may be compared to the level of GPC3 expression in a normal tissue, and when the level is higher, the GPC3 expression level can be judged to be high. Preferred normal tissues for comparing the expression level are normal cells from the same tissues (organs) as the tissues (organs) from which the cancer cells derive.

More specifically, in certain embodiments of the present disclosure, provided are methods for either or both of treating and preventing cancer, which comprise identifying a subject suffering from cancer with high GPC3 expression level, and administering to the identified subject, together with a VEGF inhibitor, a bispecific antibody which comprises (1) a domain comprising an antibody variable region having glypican 3-binding activity, (2) a domain comprising an antibody variable region having T-cell receptor complex-binding activity, and (3) a domain comprising an Fc region with reduced binding activity towards an Fcy receptor, wherein the methods are for the purpose of any one or a combination selected from preventing, alleviating, and treating either or both of CRS and cytokine release caused by administration of the bispecific antibody.

Furthermore, in the present disclosure, cytotoxicity-inducing agents and cell growth-inhibiting agents comprising as the active ingredient a multispecific antigen-binding molecule that comprises:
(1) a domain comprising an antibody variable region having glypican 3-binding activity,
(2) a domain comprising an antibody variable region having T-cell receptor complex-binding activity, and
(3) a domain comprising an Fc region with reduced binding activity towards an Fcy receptor can be expressed as a method for inducing cell injury which comprises the step of administering the anti-T cell antigen-binding molecule to an individual, or it can be expressed as a use of the anti-T cell antigen-binding molecule in the manufacture of a cytotoxicity-inducing agent and a cell growth-inhibiting agent.

In the present disclosure "comprising as the active ingredient a multispecific antigen-binding molecule that comprises (1) a domain comprising an antibody variable region having glypican 3-binding activity, (2) a domain comprising an antibody variable region having T-cell receptor complex-binding activity, and (3) a domain comprising an Fc region with reduced binding activity towards an Fcy receptor" means comprising the anti-T cell antigen-binding molecule as a major active component, without limitation to the content ratio of the anti-T cell antigen-binding molecule.

If necessary, anti-T cell antigen-binding molecules, preferably bispecific antibodies of the present invention may be encapsulated in microcapsules (e.g., those made of hydroxymethylcellulose, gelatin, and poly(methylmetacrylate)), or incorporated as components of a colloidal drug delivery system (e.g., liposomes, albumin microspheres, microemulsion, nanoparticles, and nanocapsules) (see, for example, "Remington's Pharmaceutical Science 16th edition", Oslo Ed. (1980)). Methods for preparing the pharmaceutical agents as controlled-release pharmaceutical agents are also well known, and such methods may be applied to the anti-T cell antigen-binding molecules of the present invention (J. Biomed. Mater. Res. (1981) 15: 267-277; Chemtech. (1982) 12: 98-105; U.S. Patent No. 3,773,719; European Patent Application Publication Nos. EP 58,481 and EP 133,988; Biopolymers (1983) 22: 547-556).

The pharmaceutical compositions comprising the anti-T cell antigen-binding molecule of the present disclosure may be administered to patients by oral or parenteral administration, and parenteral administration is preferred. Specific examples of the administration method include administration by injection, transnasal administration, transpulmonary administration, and transdermal administration. Examples of administration by injection include intravenous injection, intramuscular injection, intraperitoneal injection, and subcutaneous injection. A pharmaceutical composition of the present invention or a cytotoxicity-inducing agent and a cell growth-inhibiting agent can be administered systemically or locally, for example, through administration by injection. The method of administration can be selected appropriately according to the age and symptoms of the patient. The dose can be selected from the range of 0.0001 mg to 1000 mg per kilogram body weight for a single administration. Alternatively, for example, the dose may be selected from the range of 0.001 mg/body to 100000 mg/body per patient. However, the pharmaceutical compositions of the present invention or a cytotoxicity-inducing agent and cell growth-inhibiting agent are not limited to these doses.

The pharmaceutical compositions comprising the anti-T cell antigen-binding molecule of the present disclosure can be formulated according to conventional methods (for example, Remington's Pharmaceutical Science, latest edition, Mark Publishing Company, Easton, U.S.A), and may also contain pharmaceutically acceptable carriers and additives. Examples include, but are not limited to surfactants, excipients, coloring agents, perfumes, preservatives, stabilizers, buffers, suspending agents, isotonization agents, binders, disintegrants, lubricants, fluidity promoting agents, and flavoring agents; and other commonly used carriers can be suitably used. Specific examples of the carriers include light anhydrous silicic acid, lactose, crystalline cellulose, mannitol, starch, carmellose calcium, carmellose sodium, hydroxypropyl cellulose, hydroxypropyl methyl cellulose, polyvinylacetal diethylaminoacetate, polyvinylpyrrolidone, gelatin, medium chain fatty acid triglyceride, polyoxyethylene hardened castor oil 60, saccharose, carboxymethyl cellulose, corn starch, inorganic salt, and such.

In one embodiment, the present disclosure relates to pharmaceutical compositions for use in a combination therapy with a VEGF inhibitor, which comprise an anti-T cell antigen-binding molecule, for example, a bispecific antibody, preferably a bispecific antibody that binds to a cancer antigen and CD3, wherein the pharmaceutical compositions are for the purpose of any one or a combination selected from preventing, alleviating, and treating either or both of CRS and cytokine release caused by administration of the bispecific antibody. In one embodiment, the VEGF inhibitor can be selected, for example, from the group consisting of an anti-VEGF antigen-binding molecule, an anti-VEGFR1 antigen-binding molecule, an anti-VEGFR2 antigen-binding molecule, a fusion protein comprising a VEGF receptor or a fragment thereof, and a tyrosine kinase inhibitor. In particular, antibodies that bind to VEGF, VEGFR1, or VEGFR2 are preferred VEGF inhibitors. Specifically, Bevacizumab is a humanized anti-VEGF antibody; alternatively, Ramucirumab is a human anti-VEGFR2 antibody. In addition, molecular target drugs, such as Aflibercept, that were developed using VEGF as the target are also preferred examples of a VEGF inhibitor. Details of the combination therapy are disclosed in "V. Combination therapies" herein.

### III. VEGF inhibitors

In one embodiment, a "VEGF inhibitor" or "VEGF antagonist" refers to a substance capable of inhibiting or inactivating VEGF, or reducing the expression level or activity level of VEGF. A VEGF inhibitor is, for example, a compound that binds to VEGF and partially or completely blocks its activity, reduces, prevents, delays activation of, inactivates, or desensitizes it, or down-regulates its activity or expression, such as an antagonist. In another example, a VEGF inhibitor is a compound that binds to VEGF receptors or inhibit the binding of VEGF to receptors to partially or completely block the activity of VEGF, reduce, prevent, delay the activation of, inactivate, or desensitize VEGF, or down-regulate the activity. VEGF inhibitors include polypeptide inhibitors such as antigen-binding molecules, antibodies, antibody derivatives, antibody fragments, and soluble receptors, and their derivatives; nucleic acid inhibitors such as siRNAs or antisense RNAs, and their derivatives; genetically modified forms of soluble factors such as forms having altered activity; as well as naturally occurring and synthetic soluble factor antagonists; and small molecule compounds, but are not limited thereto.

In one embodiment, a VEGF inhibitor is one or more VEGF inhibitors selected from an anti-VEGF antigen-binding molecule, an anti-VEGFR1 antigen-binding molecule, an anti-VEGFR2 antigen-binding molecule, a fusion protein comprising a VEGF receptor or a fragment thereof, and a tyrosine kinase inhibitor. In one embodiment the VEGF inhibitor is an antibody, an antibody derivative, or an antibody fragment that binds to one or more signaling factors selected from an anti-VEGF antigen-binding molecule, an anti-VEGFR1 antigen-binding molecule, an anti-VEGFR2 antigen-binding molecule, a fusion protein comprising a VEGF receptor or a fragment thereof, and a tyrosine kinase inhibitor, or to the receptors thereof.

### VEGF signaling inhibitors

In one embodiment, a VEGF inhibitor is an inhibitor of VEGF signaling, such as an inhibitor of VEGF or VEGF receptor. In one embodiment, an inhibitor may be an anti-VEGF antigen-binding molecule, an anti-VEGF antibody (including a chimeric, humanized, or human anti-VEGF antibody), or an antigen-binding fragment thereof or an antibody derivative thereof. In another embodiment, an inhibitor may be an anti-VEGF receptor (VEGFR) antigen-binding molecule, an anti-VEGFR antibody (including a chimeric, humanized, or human anti-VEGFR antibody), or an antigen-binding fragment thereof or an antibody derivative thereof. In one embodiment, these inhibitors may be a soluble VEGF receptor or a fragment thereof that can block VEGF signaling. In a further embodiment, the VEGF receptor or a fragment thereof may be a fusion protein fused with a heterologous domain such as an Fc domain, for example, a VEGFR-Fc fusion protein.

In one embodiment, an anti-VEGF antibody includes, for example, Bevacizumab and Ranibizumab. In another embodiment, an anti-VEGF receptor antibody includes, for example, Ramucirumab. In one embodiment, an inhibitor of VEGF signaling includes recombinant fusion proteins such as Aflibercept.

### Bevacizumab

Bevacizumab is a humanized anti-VEGF antibody. Bevacizumab is an antibody indicated with the Japanese Accepted Names for Pharmaceuticals (JAN) of Bevacizumab, the CAS Registry No. of 216974-75-3, or the INN Name of Bevacizumab. Bevacizumab is also a recombinant humanized monoclonal antibody, which is composed of complementaritydetermining regions of a mouse anti-human vascular endothelial growth factor (VEGF) monoclonal antibody, human framework regions, and human IgG1 constant regions. Bevacizumab is produced in Chinese hamster ovary cells. Bevacizumab is a glycoprotein (molecular weight: approximately 149,000) composed of two H chains (γ1 chains) consisting of 453 amino acid residues and two L chains (κ chains) consisting of 214 amino acid residues. "Bevacizumab" also includes biosimilar Bevacizumabs.

### Pharmaceutical compositions comprising a VEGF inhibitor

From another viewpoint, the present disclosure provides pharmaceutical compositions comprising as an active ingredient a VEGF inhibitor, preferably an antibody against VEGF or its receptors, and more preferably Bevacizumab or Ramucirumab. On another hand, the present disclosure provides pharmaceutical compositions comprising as an active ingredient a fusion protein comprising the extracellular domain of a VEGF receptor, or preferably Aflibercept. Furthermore, the present disclosure relates to pharmaceutical compositions comprising the VEGF inhibitor as the active ingredient, which are for the purpose of any one of, or a combination selected from, preventing, alleviating, and treating symptoms of either, or both, of cytokine release syndrome (CRS) and cytokine release. The pharmaceutical compositions of the present disclosure are preferably administered to an individual who may develop CRS or cytokine release, and/or an individual who has developed CRS or signs of CRS and needs treatment. In certain embodiments of the present disclosure, the mechanism that brought about (or will bring about) CRS or cytokine release in subjects may be any mechanism. That is, regardless of the cause, the VEGF inhibitor can be administered to subjects observed to have CRS or show cytokine release or anticipated to develop CRS or cytokine release. For example, if administration of a given pharmaceutical agent may bring about CRS or cytokine release, subjects administered (or who will be administered) with that pharmaceutical agent can be selected, and the VEGF inhibitor can be administered to them. For example, a subject in whom CRS or cytokine release resulting from administration of a given pharmaceutical agent has been observed in the past may develop CRS or cytokine release if the same pharmaceutical agent is administered again.

In certain embodiments of the present disclosure, there is provided a step of selecting, from subjects in whom CRS or cytokine release has been observed in the past due to administration of a given pharmaceutical agent, a subject who is to be administered or who has been administered with the same pharmaceutical agent, and a VEGF inhibitor for administration along with the pharmaceutical agent to the subject. Alternatively, the present disclosure provides, so as to administer to a subject in whom CRS or cytokine release has been observed in the past due to administration of a given pharmaceutical agent the same pharmaceutical agent, a use of a VEGF inhibitor for administration to the subject along with the pharmaceutical agent. Furthermore, the present disclosure provides a method comprising selecting, from subjects in whom CRS or cytokine release has been observed in the past due to administration of a given pharmaceutical agent, a subject who will be administered with the same pharmaceutical agent, and administering a VEGF inhibitor together with the pharmaceutical agent to the subject, which is a method for the purpose of any one or a combination selected from preventing, alleviating, and treating at least one symptom selected from CRS and cytokine release resulting from administration of the same pharmaceutical agent. According to the present disclosure, an example of a treatment that may bring about CRS or cytokine release in a subject includes administration to the subject of an anti-T cell antigen-binding molecule, such as a bispecific antibody, or preferably a bispecific antibody that binds to a cancer antigen and CD3.

Without being limited to the following, a mechanism that enables a use of a pharmaceutical composition comprising as the active ingredient a VEGF inhibitor of the present disclosure for the purpose of any one or a combination selected from preventing, alleviating, and treating either, or both, of CRS and cytokine release, is a mechanism that can suppress IL-6 release by inhibiting VEGF signaling. Without being limited to the following, another mechanism that enables a use of a pharmaceutical composition comprising as the active ingredient a VEGF inhibitor of the present disclosure for the purpose of any one or a combination selected from preventing, alleviating, and treating CRS, is a mechanism in which vascular permeability is suppressed by the VEGF inhibitor and permeation of cytokines from the blood vessels into tissues can be suppressed.

Therefore, in the present disclosure, pharmaceutical compositions comprising as the active ingredient a VEGF inhibitor may also be expressed as a method for the purpose of any one, or a combination, selected from preventing, alleviating, and treating either or both of CRS and cytokine release associated with administration of an anti-T cell antigen-binding molecule, wherein the method comprises administering the VEGF inhibitor to an individual; or it can be expressed as a use of the VEGF inhibitor in the manufacture of a pharmaceutical for the purpose of any one, or a combination, selected from preventing, alleviating, and treating either or both of CRS and cytokine release associated with administration of an anti-T cell antigen-binding molecule.

In the present disclosure, "comprising a VEGF inhibitor as an active ingredient" means that the VEGF inhibitor is comprised as a major active ingredient, without limitation of the content ratio of the VEGF inhibitor.

If necessary, VEGF inhibitors of the present invention may be encapsulated in microcapsules (e.g., those made of hydroxymethylcellulose, gelatin, and poly(methylmetacrylate)), or incorporated as components of a colloidal drug delivery system (e.g., liposomes, albumin microspheres, microemulsion, nanoparticles, and nanocapsules) (see, for example, "Remington's Pharmaceutical Science 16th edition", Oslo Ed. (1980)). Methods for preparing the pharmaceutical agents as controlled-release pharmaceutical agents are also well known, and such methods may be applied to the VEGF inhibitors of the present invention (J. Biomed. Mater. Res. (1981) 15: 267-277; Chemtech. (1982) 12: 98-105; U.S. Patent No. 3,773,719; European Patent Application Publication Nos. EP 58,481 and EP 133,988; Biopolymers (1983) 22: 547-556).

The pharmaceutical compositions comprising the VEGF inhibitor of the present disclosure may be administered to patients by oral or parenteral administration, and parenteral administration is preferred. Specific examples of the administration method include administration by injection, transnasal administration, transpulmonary administration, and transdermal administration. Examples of administration by injection include intravenous injection, intramuscular injection, intraperitoneal injection, and subcutaneous injection. A pharmaceutical composition of the present invention or a cytotoxicity-inducing agent and a cell growth-inhibiting agent can be administered systemically or locally, for example, through administration by injection. The method of administration can be selected appropriately according to the age and symptoms of the patient. The dose can be selected from the range of 0.0001 mg to 1000 mg per kilogram body weight for a single administration. Alternatively, for example, the dose may be selected from the range of 0.001 mg/body to 100000 mg/body per patient. However, the pharmaceutical compositions of the present invention are not limited to these doses.

In one embodiment, when the VEGF inhibitor is Bevacizumab, the active ingredient is administered at approximately 5 to 15 mg/kg per dose, such as a dose selected from the doses of 5 mg/kg or less, 7.5 mg/kg or less, 10 mg/kg or less, and 15 mg/kg or less per dose for a patient weighing 30 kg or more, for example by intravenous injection over a course of 0.5 hours, 1 hour, 2 hours, or 3 hours. In one embodiment, when the VEGF inhibitor is Ramucirumab, the active ingredient is administered at approximately 4 to 15 mg/kg per dose, such as 8 mg/kg or less and 10 mg/kg or less per dose for a patient weighing 30 kg or more, for example by intravenous injection over a course of 0.5 hours, 1 hour, 2 hours, or 3 hours. In a different embodiment, when Bevacizumab or Ramucirumab is administered for purposes of treating CRS, and the symptoms of CRS are not improved after the first administration of Bevacizumab or Ramucirumab to a patient, administrations of Bevacizumab or Ramucirumab can be added up to three times. In one example, when Bevacizumab or Ramucirumab is sequentially administered in this manner, the interval must be 8 hours or more from the previous administration.

The pharmaceutical compositions comprising the VEGF inhibitor of the present disclosure can be formulated according to conventional methods (for example, Remington's Pharmaceutical Science, latest edition, Mark Publishing Company, Easton, U.S.A), and may also contain pharmaceutically acceptable carriers and additives. Examples include, but are not limited to surfactants, excipients, coloring agents, perfumes, preservatives, stabilizers, buffers, suspending agents, isotonization agents, binders, disintegrants, lubricants, fluidity promoting agents, and flavoring agents; and other commonly used carriers can be suitably used. Specific examples of the carriers include light anhydrous silicic acid, lactose, crystalline cellulose, mannitol, starch, carmellose calcium, carmellose sodium, hydroxypropyl cellulose, hydroxypropyl methyl cellulose, polyvinylacetal diethylaminoacetate, polyvinylpyrrolidone, gelatin, medium chain fatty acid triglyceride, polyoxyethylene hardened castor oil 60, saccharose, carboxymethyl cellulose, corn starch, inorganic salt, and such.

In one embodiment, the present disclosure relates to pharmaceutical compositions comprising a VEGF inhibitor, preferably a VEGF inhibitor, more preferably an antibody against VEGF or its receptor, and most preferably Bevacizumab or Ramucirumab, wherein the pharmaceutical composition is for use in its combination therapy with an anti-T cell antigen binding molecule. Further, the present disclosure relates to pharmaceutical compositions comprising as an active ingredient a VEGF inhibitor consisting of a fusion protein comprising the ligand-binding site of the VEGF receptor, preferably Aflibercept, wherein the pharmaceutical composition is for use in its combination therapy with an anti-T cell antigen binding molecule. In one embodiment, the anti-T cell antigen-binding molecule is for example, a bispecific antibody, or preferably a bispecific antibody that binds to a cancer antigen and CD3. Details of the combination therapy are disclosed in "V. Combination therapies" herein.

### IV. Cytokine Release Syndrome (CRS)

In one embodiment, cytokine release syndrome (CRS) is a serious and potentially life-threatening side effect that can occur when a pharmaceutical agent, for example, an antibody pharmaceutical (such as, an anti-T cell antibody) or a T cell pharmaceutical (such as, a chimeric antigen receptor (CAR) T cell (CAR-T cell)) is administered. Administration of antibody pharmaceuticals or T cell pharmaceuticals activates the immune response in the body more than necessary and releases inflammatory cytokines and such, which result in various symptoms such as chills, nausea, malaise, headache, fever, tachycardia, and blood pressure fluctuations. Severe cases, in particular, are sometimes referred to as cytokine storms. CRS results from high levels of immune activation when large numbers of lymphocytes and/or myeloid cells release inflammatory cytokines upon activation. The severity of CRS and the timing of onset of symptoms may vary depending on the magnitude of immune cell activation, the type of pharmaceutical agent administered, and/or the amount of systemic tumor tissue in the individual. In the case of T cell therapy for cancer, symptom onset is typically days to weeks after administration of the T cell therapy, for example, when there is a peak of T cell proliferation *in vivo.* See, for example, Lee et al. Blood. 124.2 (2014): 188-95.

In one embodiment, the symptoms of CRS may include neurologic toxicity, disseminated intravascular coagulation, cardiac dysfunction, adult respiratory distress syndrome, renal failure, and/or hepatic failure. For example, the symptoms of CRS may include fever/high fever with or without chills, fatigue, discomfort, muscle pain, vomiting, headache, nausea, loss of appetite, joint pain, diarrhea, rash, hypoxia, tachypnea, hypotension, widened pulse pressure, potentially diminished cardiac output (late), increased cardiac output (early), azotemia, hypofibrinogenemia with or without bleeding, elevated D-dimer, hyperbilirubinemia, transaminitis, confusion, delirium, changes in mental status, hallucinations, tremor (trembling), seizures, altered gait, word finding difficulty, obvious aphasia, or dementia.

In one embodiment, CRS is characterized by increased concentrations of several cytokines in an individual. In one embodiment, the cytokines include IL-1β, IL-2, IL-4, IL-5, IL-6, IL-8, IL-10, IL-12, IL-13, IL-15, IL-17, IL-1Ra, IL-2R, IFN-α, IFN-y, MIP-1α, MIP-1β, MCP-1, TNFα, GM-CSF, G-CSF, CXCL9, CXCL10, CXCR factor, VEGF, RANTES, eotaxin, EGF, HGF, FGF-β, CD30, CD30L, CD40, CD40L, ferritin, and RAGE, but are not limited thereto. Preferably, the cytokine comprises IL-6, IL-1 beta, or TNF-alpha, or any combination thereof. Most preferably, the cytokine is IL-6. In one embodiment, patients with large tumor volumes have a higher incidence and severity of cytokine syndrome.

In one embodiment, those skilled in the art will understand that the term "cytokine concentration" includes a measured concentration value, magnitude of fold change, magnitude of percent (%) change, or magnitude of rate change. In addition, methods for measuring cytokines in blood, saliva, serum, urine, plasma, and/or serum are well known in the art.

### Severity (Grade) of CRS

In one embodiment, CRS can be classified by severity (Grade) from 1 to 5. In one embodiment, for Grade 1 CRS, only symptomatic treatment is needed (for example, nausea, fever, fatigue, muscle pain, malaise, and headache), and the symptoms are not life threatening. For Grade 2 CRS, the symptoms require moderate intervention and generally respond to moderate intervention. Individuals with Grade 2 CRS develop hypotension that is responsive to either fluids or one type of low-dose vasopressor; or they develop grade 2 organ toxicity or mild respiratory symptoms that are responsive to low flow oxygen (less than 40% oxygen). In individuals with Grade 3 CRS, hypotension generally cannot be reversed by fluid therapy or one type of low-dose vasopressor. These individuals generally require lower flow of oxygen and have grade 3 organ toxicity (for example, renal or cardiac dysfunction or blood coagulation disorder) and/or grade 4 transaminitis. Individuals with Grade 3 CRS require more aggressive intervention, such as oxygen of 40% or higher, a high-dose vasopressor, and/or multiple vasopressors. Individuals with Grade 4 CRS suffer from immediately life-threatening symptoms, including grade 4 organ toxicity or a need for mechanical artificial ventilation. Individuals with Grade 4 CRS generally do not have transaminitis. In individuals with Grade 5 CRS, the toxicity causes death.

In one embodiment, the CRS grading is based on the Common Terminology Criteria for Adverse Events (CTCAE) v4.03 shown in Table 6, the Common Terminology Criteria for Adverse Events (CTCAE) v5.0 shown in Table 7, Lee's criteria of 2014 (Lee DW, et al. Current concepts in the diagnosis and management of cytokine release syndrome. Blood, 124 (2014), pp. 188-195) shown in Table 8, the 2019 ASTCT CRS Consensus Grading (Lee DW, et al. ASTCT Consensus Grading for Cytokine Release Syndrome and Neurologic Toxicity Associated with Immune Effector Cells. Biol Blood Marrow Transplant. 2019 Apr;25(4):625-638) shown in Table 9, Penn's criteria, MSKCC's criteria, or CARTOX's criteria (Biol Blood Marrow Transplant. 2019 Apr;25(4):625-638 indicated above). Preferably, CRS grading is based on CTCAE v4.03, CTCAE v5.0, or Lee's criteria of 2014. Unless otherwise specified, CRS as used herein refers to CRS according to the criteria in Table 8 (Lee's criteria of 2014).

**[Table 6]**

| (CTCAE v 4.03) | |
|---|---|
| Grade I | Mild reaction; not requiring interruption of infusion; not requiring treatment |
| Grade II | Requiring interruption of treatment or infusion. However, responding quickly to symptomatic treatment (for example: antihistamines, NSAIDs, narcotic pharmaceutical agents, and intravenous infusion); requiring prophylactic medications in 24 hours or less |
| Grade III | Prolonged (for example: not rapidly responsive to symptomatic treatment and/or brief interruption of infusion); recurrence after initial improvement; requiring hospitalization due to sequela(e) (for example: renal dysfunction, and pulmonary infiltration) |
| Grade IV | Life-threatening; requiring positive-pressure breathing or mechanical ventilation |
| Grade V | Death |

**[Table 7]**

| (CTCAE v 5.0) | |
|---|---|
| Grade I | Fever with or without constitutional symptoms |
| Grade II | Hypotension responding to infusion; hypoxia responding to <40% oxygen administration |
| Grade III | Hypotension manageable by a single vasopressor; hypoxia requiring ≥40% oxygen administration |
| Grade IV | Life-threatening; requiring emergency treatment |
| Grade V | Death |

**[Table 8]**

| (Lee's criteria) | |
|---|---|
| Grade I | Not life-threatening; requiring symptomatic treatments only (fever, nausea, fatigue, headache, muscle pain, discomfort) |
| Grade II | Requiring moderate intervention; hypoxia responding to >40% oxygen administration; hypotension responding to intravenous infusion or low-dose of a single vasopressor; grade 2 or higher organ toxicity |
| Grade III | Requiring aggressive intervention; hypoxia responding to ≤40% oxygen administration; hypotension responding to high-dose or multiple vasopressors; grade 3 or higher organ toxicity or grade 4 or higher transaminitis |
| Grade IV | Life-threatening; requiring mechanical artificial ventilation; grade 4 or higher organ toxicity (except transaminitis) |
| Grade V | Death |

**[Table 9]**

| (ASTCT CRS Consensus Grading) | | | |
|---|---|---|---|
| | Fever | | Hypotension and/or hypoxia |
| Grade I | 38°c or higher | and | Hypotension: none, hypoxia: none |
| Grade II | 38°c or higher | and | Hypotension not requiring a vasopressor and/or hypoxia requiring a low-flow nasal cannula or blow-by |
| Grade III | 38°Corhigher | and | Hypotension requiring a single vasopressor (regardless of combined use of vasopressin), and/or hypoxia requiring high-flow nasal cannula, face mask, nonrebreathing mask, or Venturi mask |
| Grade IV | 38°C or higher | and | Hypotension requiring multiple vasopressors (excluding vasopressin), and/or hypoxia requiring positive pressure (for example, CPAP, BiPAP, intubation, and mechanical artificial ventilation) |

In one embodiment, the symptoms of CRS develop within minutes, hours, or days after starting to administer the pharmaceutical agent, but some symptoms are delayed. Preferably, the symptoms of CRS develop within about 96 hours, about 72 hours, or about 48 hours after starting to administer the anti-T cell antigen-binding molecule, and more preferably, the symptoms develop from the day of administration of the anti-T cell antigen-binding molecule to the next day. In another embodiment, the severity of CRS symptoms correlates with peak cytokine concentrations.

### Signs of CRS

In one embodiment, signs of CRS refer to CRS-like symptoms that are precursors to the above-mentioned CRS (regardless of Grade). Specific examples include, but are not limited to, fever or hypotension that initially develops after administration of the anti-T cell antigen binding molecule.

### Treatment of CRS

In one embodiment, examples of treatment methods for CRS include VEGF inhibitors, bazedoxifene, SGP130 blockers, vasoactive pharmaceutical agents, systemic adrenal cortex hormones (for example, corticosteroids), immunosuppressants, and mechanical artificial ventilation.

In one embodiment, the VEGF inhibitor is one or more VEGF inhibitors or antagonistic inhibitors selected from an anti-VEGF antigen-binding molecule, an anti-VEGFR1 antigen-binding molecule, an anti-VEGFR2 antigen-binding molecule, a fusion protein comprising a VEGF receptor or a fragment thereof, and a tyrosine kinase inhibitor. Preferred VEGF inhibitors are disclosed in "III. VEGF inhibitors" herein.

In one embodiment, when the VEGF inhibitor is Bevacizumab, the active ingredient is administered at approximately 5 to 15 mg/kg per dose, such as a dose selected from the doses of 5 mg/kg or less, 7.5 mg/kg or less, 10 mg/kg or less, and 15 mg/kg or less per dose for a patient weighing 30 kg or more, for example by intravenous injection over a course of 0.5 hours, 1 hour, 2 hours, or 3 hours. In one embodiment, when the VEGF inhibitor is Ramucirumab, the active ingredient is administered at approximately 4 to 15 mg/kg per dose, such as 8 mg/kg or less and 10 mg/kg or less per dose for a patient weighing 30 kg or more, for example by intravenous injection over a course of 0.5 hours, 1 hour, 2 hours, or 3 hours. In a different embodiment, when Bevacizumab (Avastin) or Ranibizumab (Lucentis) is administered for purposes of treating CRS, and the symptoms of CRS are not improved after the first administration, administrations of Bevacizumab (Avastin) or Ranibizumab (Lucentis) can be added up to three times. In one example, when Bevacizumab (Avastin) or Ranibizumab (Lucentis) is sequentially administered in this manner, the interval must be 8 hours or more from the previous administration.

Exemplary vasoactive pharmaceutical agents include but are not limited to angiotensin-11, endothelin-1, alpha adrenergic agonists, rostanoids, phosphodiesterase inhibitors, endothelin antagonists, circulatory agonists (for example, adrenaline, dobutamine, isoprenaline, and ephedrine), vasopressors (for example, noradrenaline, vasopressin, metaraminol, vasopressin, and methylene blue), inodilators (for example, milrinone and levosimendan), and dopamine.

Exemplary vasopressors include but are not limited to norepinephrine, dopamine, phenylephrine, epinephrine, and vasopressin. In one embodiment, vasopressors include high-dose vasopressors and low-dose vasopressors. In one embodiment, a high-dose vasopressor includes one or more of the following: norepinephrine monotherapy at 20 µg/min or more, dopamine monotherapy at 10 µg/kg/min or more, phenylephrine monotherapy at 200 µg/min or more, and/or epinephrine monotherapy at 10 µg/min or more. In some embodiments, if an individual is on vasopressin, a high-dose vasopressor includes vasopressin + norepinephrine equivalent at 10 µg/min or more (where the dose of norepinephrine equivalent = [norepinephrine (µg/min)] + [dopamine (µg/kg/min)/2] + [epinephrine (µg/min)] + [phenylephrine (µg/min)/10]). In some embodiments, if the individual is on combination vasopressors (not vasopressin), a high-dose vasopressor includes norepinephrine equivalent at 20 µg/min or more (where the dose of norepinephrine equivalent = [norepinephrine (µg/min)] + [dopamine (µg/kg/min)/2] + [epinephrine (µg/min)] + [phenylephrine (µg/min)/10]. See for example, *idem.*

In one embodiment, a low-dose vasopressor is a vasopressor administered at a dose less than one or more of the doses listed above for high-dose vasopressors.

Exemplary corticosteroids include but are not limited to dexamethasone, hydrocortisone, and methylprednisolone. In one embodiment, a dose of 0.5 mg/kg of dexamethasone is used by oral or intravenous administration. In one embodiment, 8 to 20 mg of dexamethasone per dose, a pharmaceutically acceptable salt thereof, or a derivative thereof is used by oral or intravenous administration. It will be understood by those skilled in the art that the dose of dexamethasone is not limited to the above and may be appropriately changed depending on the condition of the individual and the severity of CRS.

An exemplary immunosuppressant includes a TNF-alpha inhibitor or an IL-1 inhibitor. In one embodiment, TNF-alpha inhibitors include anti-TNF-alpha antibodies, for example, a monoclonal antibody, such as Infliximab. In one embodiment, TNF-alpha inhibitors include soluble TNF-alpha receptors (for example, Etanercept). In embodiments, IL-1 or IL-1R inhibitors include Anakinra.

### V. Combination therapies

In one embodiment, the present disclosure relates to a combined use of an arbitrary pharmaceutical agent and a VEGF inhibitor, wherein the pharmaceutical agent is a pharmaceutical agent that is the cause of either or both of CRS and cytokine release in a subject of administration, and wherein the combined use is for the purpose of any one, or a combination, selected from preventing, alleviating, and treating either or both of CRS and cytokine release resulting from administration of the pharmaceutical agent. A combination therapy of the present disclosure can also be defined as follows:
specifically, a combination therapy using pharmaceutical agent A and pharmaceutical agent B, wherein pharmaceutical agent A has an effect of potentially inducing either or both of CRS and cytokine release following administration to a subject and pharmaceutical agent B is a VEGF inhibitor, and wherein the combination therapy is for the purpose of any one or a combination selected from preventing, alleviating, and treating either or both of CRS and cytokine release induced by administration of pharmaceutical agent A.

In another embodiment, the present disclosure relates to a pharmaceutical composition which comprises an arbitrary pharmaceutical agent (pharmaceutical agent A) and which is for use in a combination therapy with a VEGF inhibitor (pharmaceutical agent B), wherein the pharmaceutical agent may induce either or both of CRS and cytokine release in a subject of administration, and wherein the pharmaceutical composition is for the purpose of any one or a combination selected from preventing, alleviating, and treating either or both of CRS and cytokine release resulting from administration of the pharmaceutical agent. In a further embodiment, the present disclosure relates to a pharmaceutical composition which comprises a VEGF inhibitor and which is for use in a combination therapy with an arbitrary pharmaceutical agent, wherein the pharmaceutical agent may induce either or both of CRS and cytokine release in a subject of administration, and the pharmaceutical composition is for the purpose of any one or a combination selected from preventing, alleviating, and treating either or both of CRS and cytokine release resulting from administration of the pharmaceutical agent. In the present disclosure, when the pharmaceutical agent which may induce either or both of CRS and cytokine release is an anti-T cell antigen-binding molecule, the present disclosure relates to a method for treating cancer by administration of a VEGF inhibitor, wherein the method is characterized by the purpose of any one or a combination selected from preventing, alleviating, and treating either or both of CRS and cytokine release resulting from the anti-T cell antigen-binding molecule.

In the present disclosure, a pharmaceutical agent which may induce either or both of CRS and cytokine release in a subject of administration indicates a pharmaceutical agent which may cause either or both of CRS and cytokine release. Specifically, examples include pharmaceutical agents reported to induce either or both of CRS and cytokine release as a side effect accompanying administration. For example, pharmaceutical agents that target lymphocytes are pharmaceutical agents whose stimuli may induce cytokine production. In other words, lymphocyte-stimulating pharmaceutical agents may become a cause of either or both of CRS and cytokine release. Examples of such pharmaceutical agents are pharmaceutical agents comprising as the active ingredient an anti-T cell antigen-binding molecule.

In one embodiment, administration of a VEGF inhibitor accomplishes the purpose of any one or a combination selected from preventing, alleviating, and treating either or both of CRS and cytokine release in an individual. Therefore, the present disclosure may be rephrased as a method for the purpose of any one or a combination selected from preventing, alleviating, and treating either or both of CRS and cytokine release, which comprises administering a VEGF inhibitor.

In one embodiment, a VEGF inhibitor is administered subcutaneously or intravenously to an individual before, simultaneously with, or after administration of an arbitrary pharmaceutical agent that will cause either or both of CRS and cytokine release. Preferably, a VEGF inhibitor is administered intravenously to an individual before, simultaneously with, or after administration of the pharmaceutical agent. Although not intended to be limiting, when a VEGF inhibitor is administered before or simultaneously with administration of the pharmaceutical agent, it has the effect of preventing or alleviating development of either or both of CRS and cytokine release associated with administration of the pharmaceutical agent. In one embodiment, a VEGF inhibitor is administered 6 days, 5 days, 4 days, 3 days, 2 days, or 1 day before administration of an arbitrary pharmaceutical agent that causes either or both of CRS and cytokine release, or on the same day as but before administration of the pharmaceutical agent. Alternatively, a VEGF inhibitor may be administered on the same day as and simultaneously with administration of the pharmaceutical agent (in the present disclosure, administration of a VEGF inhibitor before administration of an arbitrary pharmaceutical agent that will cause either or both of CRS and cytokine release, and administration of a VEGF inhibitor simultaneously with administration of the pharmaceutical agent are collectively referred to as "pre-administration of a VEGF inhibitor"). In one embodiment, pre-administration of the VEGF inhibitor prevents or alleviates development of CRS associated with administration of an arbitrary pharmaceutical agent that causes either or both of CRS and cytokine release, without significantly impairing the drug efficacy of the pharmaceutical agent (for example, T cell-dependent cellular cytotoxicity (TDCC) and antitumor effects).

Furthermore, when a VEGF inhibitor is administered after administration of the arbitrary pharmaceutical agent that causes either or both of CRS and cytokine release and before development of CRS, it has the effect of preventing or alleviating CRS that may develop thereafter. In addition, when a VEGF inhibitor is administered after administration of the pharmaceutical agent and after development of CRS or signs of CRS, it has the effect of treating CRS and alleviating its symptoms.

Therefore, the present disclosure provides a method for either or both of treating CRS and alleviating its symptoms, which comprises monitoring development of CRS after an arbitrary treatment that causes either or both of CRS and cytokine release, selecting a subject showing signs thereof, and administering a VEGF inhibitor to the subject. Methods for monitoring development of CRS and finding out the signs of CRS are known, as described previously.

Effects by the VEGF inhibitor for the purpose of any one or a combination selected from preventing, alleviating, and treating either or both of CRS and cytokine release are effects revealed for the first time in the present disclosure. Therefore, the present disclosure provides pharmaceutical compositions comprising a VEGF inhibitor, which are for the purpose of any one or a combination selected from preventing, alleviating, and treating either or both of CRS and cytokine release. Alternatively, the present disclosure provides use of a VEGF inhibitor in the manufacture of pharmaceutical compositions for the purpose of any one or a combination selected from preventing, alleviating, and treating either or both of CRS and cytokine release. Furthermore, the present disclosure provides use of a VEGF inhibitor for the purpose of any one or a combination selected from preventing, alleviating, and treating either or both of CRS and cytokine release. The present disclosure provides a method for the purpose of any one or a combination selected from preventing, alleviating, and treating either or both of CRS and cytokine release, wherein the method comprises selecting any subject selected from subjects who have developed either or both of CRS and cytokine release, subjects showing signs of development thereof, and subjects who will receive treatment that may involve either or both of CRS and cytokine release, and administering a VEGF inhibitor to the subject. In the present disclosure, treatment that may involve either or both of CRS and cytokine release includes, for example, administration of an anti-T cell antigen-binding molecule.

In one embodiment, the anti-T cell antigen-binding molecule used in the combination therapy is a bispecific antigen-binding molecule comprising "a domain comprising an antibody variable region having T cell receptor complex-binding activity" and "a domain comprising an antibody variable region having cancer antigen-binding activity", and is preferably a bispecific antibody. In one embodiment, the bispecific antibody may have the structure of a single chain antibody, for example, a structure in which antibody variable regions are linked by linkers. In one embodiment, the anti-T cell antigen-binding molecule further comprises an Fc region with reduced Fcγ receptor-binding activity. Preferably, the anti-T cell antigen-binding molecule is a bispecific antibody comprising (1) a domain comprising an antibody variable region having a glypican 3-binding activity, (2) a domain comprising an antibody variable region having T cell receptor complex-binding activity, and (3) a domain comprising an Fc region with reduced Fcγ receptor-binding activity.

In one embodiment, a VEGF inhibitor used in the combination therapy is a VEGF inhibitor, preferably an antibody against VEGF or its receptor, and most preferably Bevacizumab or Ramucirumab. In other embodiments, the VEGF inhibitor used in the combination therapy is a fusion protein comprising the ligand binding site of a VEGF receptor, and preferred examples include Aflibercept. In one embodiment, when Bevacizumab or Ramucirumab is administered before or simultaneously with administration of the anti-T cell antigen binding molecule, Bevacizumab or Ramucirumab is administered to human adults and children, for example, at a dose selected from 5 mg/kg to 100 mg/kg, for example, 5 mg/kg to 90 mg/kg, 5 mg/kg to 80 mg/kg, 5 mg/kg to 70 mg/kg, 5 mg/kg to 60 mg/kg, 5 mg/kg to 50 mg/kg, 5 mg/kg to 40 mg/kg, 5 mg/kg to 30 mg/kg, 5 mg/kg to 20 mg/kg, 5 mg/kg to 15 mg/kg, and for example, 10 mg/kg to 100 mg/kg, 20 mg/kg to 100 mg/kg, 30 mg/kg to 100 mg/kg, 40 mg/kg to 100 mg/kg, 50 mg/kg to 100 mg/kg, 60 mg/kg to 100 mg/kg, 70 mg/kg to 100 mg/kg, 80 mg/kg to 100 mg/kg, 90 mg/kg to 100 mg/kg, and the like, per dose. In addition, although it is specified to avoid doubt, for example, when described as 5 mg to 100 mg/kg, any dose included between 5 mg to 100 mg/kg varying by 0.1 mg/kg, such as 5.0 mg/kg, 5.1 mg/kg, 5.2 mg/kg, 5.3 mg/kg, 5.4 mg/kg, 49.9 mg, 50 mg/kg, 50.1 mg/kg, 50.2 mg/kg, ... 99.8 mg/kg, 99.9 mg/kg, and 100 mg/kg, is intended to be individually and specifically described herein. In one embodiment, Bevacizumab is administered before or simultaneously with administration of the anti-T cell antigen-binding molecule, at 15 mg/kg or less, 0.5 to 15 mg/kg, 1 to 15 mg/kg, 2 to 15 mg/kg, 3 to 15 mg/kg, or 4 to 15 mg/kg per dose for a patient weighing 30 kg or more. In one embodiment, Ramucirumab is administered before or simultaneously with administration of the anti-T cell antigen-binding molecule, at 10 mg/kg or less, 0.5 to 10 mg/kg, 1 to 10 mg/kg, 2 to 10 mg/kg, 3 to 10 mg/kg, or 4 to 10 mg/kg per dose for a patient weighing 30 kg or more.

In the present disclosure, in several embodiments, in addition to the combined use of an anti-T cell antigen-binding molecule and a VEGF inhibitor, an immune checkpoint inhibitor can be additionally used in combination. "Immune checkpoint" refers to a signaling molecule that is expressed on immunocompetent cells (including T cells) and inhibits immune response against the immunocompetent cells by binding to a ligand. The immune checkpoint and its ligand include, but are not limited to, molecules such as PD-1, CTLA-4, TIM3, LAG3, PD-L1, PD-L2, BTNL2, B7-H3, B7-H4, CD48, CD80, 2B4, BTLA, CD160, CD60, CD86, and VISTA. In several embodiments, an "immune checkpoint inhibitor" of the present invention refers to a pharmaceutical agent that inhibits signal transduction mediated by an immune checkpoint by inhibiting binding between an immune checkpoint and its ligand.

In a non-limiting embodiment of the present disclosure, preferred examples of an immune checkpoint inhibitor include, but are not limited to, PD1 antibodies, PDL1 antibodies, CTLA-4 antibodies, TIM3 antibodies, and LAG3 antibodies. Examples of a PD-1 antibody include Pembrolizumab (CAS Registry No. 1374853-91-4), Nivolumab (CAS Registry No. 946414-94-4), MEDI0680, PDR001, BGB-A317, REGN2810, SHR-1210, PF-06801591, and various known PD1 antibodies. Examples of a PD-L1 antibody include Atezolizumab (CAS Registry No. 1380723-44-3), Avelumab (CAS Registry No. 1537032-82-8), Durvalumab (CAS Registry No. 1428935-60-7), MDX-1105, and various known PD-L1 antibodies. Examples of a CTLA-4 antibody include Ipilimumab (CAS Registry No. 477202-00-9), Tremelimumab (CAS Registry No. 745013-59-6), and various known CTLA-4 antibodies. Examples of a TIM3 antibody include MBG452 and various known TIM3 antibodies. Examples of an LAG3 antibody include BMS-986016, LAG525, and various known LAG3 antibodies.

In a certain embodiment of the present disclosure, when the combined use of an anti-T cell antigen-binding molecule and a VEGF inhibitor is further additionally combined with the use of an immune checkpoint inhibitor, the immune checkpoint inhibitor can be administered to the subject at any time. Specifically, for example, the immune checkpoint inhibitor can be administered before or after the combined administration of the anti-T cell antigen-binding molecule and VEGF inhibitor. Therefore, for example, after evaluating cancer regression effects caused either by the combined use of the anti-T cell antigen-binding molecule and VEGF inhibitor or by the immune checkpoint inhibitor, the other unevaluated component can be additionally administered. Alternatively, these three agents can be administered simultaneously.

The combination therapies of the present disclosure may further additionally include administration of corticosteroids. In one embodiment, for the purpose of preventing or alleviating CRS associated with administration of the anti-T cell antigen-binding molecule, corticosteroids are orally or intravenously administered to an individual before administration of the anti-T cell antigen-binding molecule, 2 days, 1 day, or 0 days before (on the same day as) the administration of the anti-T cell antigen-binding molecule. Alternatively, the corticosteroid is orally or intravenously administered to an individual simultaneously with the administration of the anti-T cell antigen-binding molecule (these may be referred to as "pre-administration of a corticosteroid" or "pre-administration of a steroid"). Preferably, pre-administration of a corticosteroid is performed in addition to pre-administration of a VEGF inhibitor (in combination with pre-administration of a VEGF inhibitor). Although not intended to be limiting, examples of preferred corticosteroids include dexamethasone, hydrocortisone, and methylprednisolone. Preferably, the corticosteroid is dexamethasone, a pharmaceutically acceptable salt thereof, or a derivative thereof, which is administered orally or intravenously. It will be understood by those skilled in the art that the dose of dexamethasone is not limited to the above and may be appropriately changed depending on the condition of the individual, the development of CRS, and the like.

In one embodiment, the combination therapy is carried out on the same day as administration of an anti-T cell antigen-binding molecule in the following order: (1) pre-administration of a corticosteroid, (2) pre-administration of a VEGF inhibitor, and (3) administration of an anti-T cell antigen-binding molecule. In this case, for example, administration of a corticosteroid to an individual is completed 1 hour or more, 2 hours or more, 3 hours or more, 4 hours or more, or 5 hours or more, or preferably 2 hours or more before starting the administration of a VEGF inhibitor; furthermore, administration of a VEGF inhibitor to the individual is completed 1 hour or more, 2 hours or more, 3 hours or more, 4 hours or more, or 5 hours or more, or preferably 2 hours or more before starting the administration of the anti-T cell antigen-binding molecule. In another embodiment, administration is performed on the same day as administration of an anti-T cell antigen-binding molecule in the following order: (1) pre-administration of a VEGF inhibitor, (2) pre-administration of a corticosteroid, and (3) administration of an anti-T cell antigen-binding molecule. In this case, for example, administration of a VEGF inhibitor to an individual is completed 1 hour or more, 2 hours or more, 3 hours or more, 4 hours or more, or 5 hours or more, or preferably 2 hours or more before starting the administration of a corticosteroid; furthermore, administration of the corticosteroid to the individual is completed 1 hour or more, 2 hours or more, 3 hours or more, 4 hours or more, or 5 hours or more, or preferably 2 hours or more before starting the administration of the anti-T cell antigen-binding molecule.

In a different embodiment, the combination therapy of the present disclosure does not include pre-administration of corticosteroids. Although not intended to be limiting, pre-administration of a VEGF inhibitor prevents or alleviates development of CRS associated with administration of the anti-T cell antigen-binding molecule, and administration of corticosteroids for purposes of preventing or alleviating CRS becomes unnecessary.

The combination therapies of the present disclosure may further include administration of Tocilizumab. In one embodiment, for the purpose of preventing or alleviating CRS associated with administration of the anti-T cell antigen-binding molecule, Tocilizumab is intravenously administered to an individual before administration of the anti-T cell antigen-binding molecule, 2 days, 1 day, or 0 days before (on the same day as) the administration of the anti-T cell antigen-binding molecule. Alternatively, Tocilizumab is intravenously administered to an individual simultaneously with the administration of the anti-T cell antigen-binding molecule (these may be referred to as "pre-administration of Tocilizumab" or "pre-administration of Tocilizumab"). Preferably, pre-administration of Tocilizumab is performed in addition to pre-administration of a VEGF inhibitor (in combination with pre-administration of a VEGF inhibitor). It will be understood by those skilled in the art that the dose of Tocilizumab is not limited to the above and may be appropriately changed depending on the condition of the individual, the development of CRS, and the like.

In one embodiment, the combination therapy is carried out on the same day as administration of an anti-T cell antigen-binding molecule in the following order: (1) pre-administration of Tocilizumab, (2) pre-administration of a VEGF inhibitor, and (3) administration of an anti-T cell antigen-binding molecule. In this case, for example, administration of Tocilizumab to an individual is completed 1 hour or more, 2 hours or more, 3 hours or more, 4 hours or more, or 5 hours or more, or preferably 2 hours or more before starting the administration of a VEGF inhibitor; furthermore, administration of a VEGF inhibitor to the individual is completed 1 hour or more, 2 hours or more, 3 hours or more, 4 hours or more, or 5 hours or more, or preferably 2 hours or more before starting the administration of the anti-T cell antigen-binding molecule. In another embodiment, administration is performed on the same day as administration of an anti-T cell antigen-binding molecule in the following order: (1) pre-administration of a VEGF inhibitor, (2) pre-administration of Tocilizumab, and (3) administration of an anti-T cell antigen-binding molecule. In this case, for example, administration of a VEGF inhibitor to an individual is completed 1 hour or more, 2 hours or more, 3 hours or more, 4 hours or more, or 5 hours or more, or preferably 2 hours or more before starting the administration of Tocilizumab; furthermore, administration of Tocilizumab to the individual is completed 1 hour or more, 2 hours or more, 3 hours or more, 4 hours or more, or 5 hours or more, or preferably 2 hours or more before starting the administration of the anti-T cell antigen-binding molecule.

In a different embodiment, the combination therapy of the present disclosure does not include pre-administration of Tocilizumab. Although not intended to be limiting, pre-administration of a VEGF inhibitor prevents or alleviates development of CRS associated with administration of the anti-T cell antigen-binding molecule, and administration of Tocilizumab for purposes of preventing or alleviating CRS becomes unnecessary.

It is naturally understood by those skilled in the art that the anti-T cell antigen-binding molecules, VEGF inhibitors, and/or other pharmaceutical agents in the present disclosure may all have a given "permissible range" for the timing of administration, the dosing interval, and the dose, respectively, and those skilled in the art can appropriately determine the permissible range. For example, performing timely increase or decrease of the dosing interval or appropriate increase or decrease of the dose of anti-T cell antigen-binding molecules, VEGF inhibitors, and/or other pharmaceutical agents at the discretion of the doctor, based on symptoms and such of the individual, is within the above "permissible range".

Below, non-limiting specific examples of combination therapies comprising anti-T cell antigen-binding molecules and VEGF inhibitors are described.

### Administration of an anti-T cell antigen-binding molecule, accompanied with pre-administration of a VEGF inhibitor

In one embodiment, the present disclosure provides a pharmaceutical composition comprising an anti-T cell antigen-binding molecule, which is for use in its combination therapy with a VEGF inhibitor. In a different embodiment, the present disclosure provides a pharmaceutical composition comprising a VEGF inhibitor, which is for use in its combination therapy with an anti-T cell antigen binding molecule. In a further embodiment, a VEGF inhibitor is administered to an individual before or simultaneously with administration of the anti-T cell antigen binding molecule. Although not intended to be limiting, when a VEGF inhibitor is administered before or simultaneously with administration of the anti-T cell antigen-binding molecule, it has the effect of preventing or alleviating development of CRS associated with administration of the anti-T cell antigen-binding molecule. In one embodiment, a VEGF inhibitor is administered 6 days, 5 days, 4 days, 3 days, 2 days, or 1 day before administration of the anti-T cell antigen-binding molecule, or on the same day as but before the administration of the bispecific antibody. Alternatively, a VEGF inhibitor is administered simultaneously with the anti-T cell antigen-binding molecule on the same day as administration of the anti-T cell antigen-binding molecule. In one embodiment, pre-administration of a VEGF inhibitor prevents or alleviates development of CRS associated with administration of an anti-T cell antigen-binding molecule, without significantly impairing the efficacy of the anti-T cell antigen-binding molecule (for example, T cell-dependent cellular cytotoxicity (TDCC) and antitumor effect).

### VI. Combination therapies with an anti-glypican 3 (GPC3)/ T cell receptor complex bispecific antibody and a VEGF inhibitor

In one embodiment, the present disclosure provides a pharmaceutical composition comprising an anti-GPC3/ T cell receptor complex bispecific antibody, wherein the pharmaceutical composition is for use in its combination therapy with a VEGF inhibitor. In a different embodiment, the present disclosure provides a pharmaceutical composition comprising a VEGF inhibitor, wherein the pharmaceutical composition is for use in its combination therapy with an anti-glypican 3 (GPC3)/ T cell receptor complex bispecific antibody. In one embodiment, the VEGF inhibitor is administered to an individual before or simultaneously with administration of the anti-GPC3/ T cell receptor complex bispecific antibody.

Although not intended to be limiting, when a VEGF inhibitor is administered before or simultaneously with administration of an anti-GPC3/ T cell receptor complex bispecific antibody, the inhibitor has effects of preventing or alleviating the development of CRS associated with administration of the anti-GPC3/ T cell receptor complex bispecific antibody. In one embodiment, the VEGF inhibitor is administered 6 days, 5 days, 4 days, 3 days, 2 days, or 1 day before the administration of the anti-GPC3/ T cell receptor complex bispecific antibody, or on the same day as but before the administration of the bispecific antibody. Alternatively, the VEGF inhibitor is administered on the same day as administration of the anti-GPC3/T cell receptor complex bispecific antibody, and simultaneously with the bispecific antibody (pre-administration of the VEGF inhibitor). For specific examples of the combination therapy comprising pre-administration of the VEGF inhibitor, see the various embodiments described above. In one embodiment, pre-administration of the VEGF inhibitor prevents or alleviates development of CRS associated with administration of the anti-GPC3/ T cell receptor complex bispecific antibody without significantly compromising the drug efficacy of the anti-GPC3/ T cell receptor complex bispecific antibody (for example, T cell-dependent cellular cytotoxicity (TDCC) and antitumor effects).

### VII. Production Methods and Kits

The present invention also provides a kit for use in the method of the present invention, which comprises the anti-T cell antigen-binding molecule of the present invention or the anti-T cell antigen-binding molecule produced by the production method of the present invention. In addition, the kit may include in its package a pharmaceutically acceptable carrier, medium, instructions describing the method of use, and such.

The present invention also relates to an anti-T cell antigen-binding molecule of the present invention or an anti-T cell antigen-binding molecule produced by the production method of the present invention for use in the method of the present invention.

The present invention also provides a kit comprising a VEGF inhibitor, which is for use in the method of the present invention. In addition, the kit may include in its package a pharmaceutically acceptable carrier, medium, instructions describing the method of use, and such.

The present invention also relates to a VEGF inhibitor for use in the method of the present invention.

The present invention also relates to nucleic acids encoding these molecules, vectors into which the nucleic acids have been introduced, cells containing the nucleic acids or the vectors, methods for producing the molecules by culturing the cells, and anti-T cell antigen-binding molecules and VEGF inhibitors produced by the methods.

For example, the anti-T-cell antigen-binding molecule can be produced by introducing into an appropriate host cell, a nucleic acid encoding the anti-T cell antigen-binding molecule or a vector into which the nucleic acid has been introduced and culturing the host cell. When the anti-T cell antigen-binding molecule is secreted, the anti-T cell antigen-binding molecule of interest can be recovered from the culture supernatant. A method for purifying the anti-T cell antigen-binding molecule of interest from the culture is also known. On the other hand, if the VEGF inhibitor is a protein component such as an antibody, it can be produced in the same manner as the multispecific antigen-binding molecule by introducing into an appropriate host cell, the nucleic acid encoding the VEGF inhibitor molecule or the vector into which the nucleic acid is introduced, and culturing the host cell.

In the present disclosure, the anti-T cell antigen-binding molecule and the VEGF inhibitor can be independently packaged with a pharmaceutically acceptable carrier, medium, instructions describing the method of use, and such. Alternatively, both can be formulated and then combined to form a kit. Specifically, the kit can include, for example, a package containing a formulated anti-T cell antigen-binding molecule and a package containing a formulated VEGF inhibitor. When each of the formulations constituting the kit is a lyophilized one or a concentrated liquid formulation, a liquid medium for dissolving or diluting the formulation can be combined with the kit.

The present disclosure provides a pharmaceutical composition comprising the anti-T cell antigen-binding molecule described above, which is a pharmaceutical composition for administration with a VEGF inhibitor, for either or both of treating and preventing cancer, and which is for the purpose of any one or a combination selected from preventing, alleviating, and treating either or both of CRS and cytokine release resulting from administration of the pharmaceutical composition.

Herein, the pharmaceutical composition is not limited to a pharmaceutical composition comprising an anti-T cell antigen-binding molecule, and it may be a Chimeric Antigen Receptor (hereafter, also referred to as "CAR")-expressing T cell (hereafter, also simply referred to as "CAR-T cell") produced by introducing a gene encoding CAR to normal peripheral blood T cells (peripheral blood T lymphocytes). CAR is a chimeric protein formed by artificially fusing an antibody that recognizes a cell surface antigen of cancer cells and such with a signal transduction region that induces T cell activation. CAR-T cells are produced by introducing a CAR-encoding gene into peripheral blood T lymphocytes. The CAR-expressing T cells produced by such an approach are used in treatment of diseases such as cancers by adoptive immunotherapy. These CAR-T cells are reactive to antigen-expressing target cells, and become capable of damaging the target cells without depending on interaction with major histocompatibility complexes (MHCs).

Clinical trials are ongoing worldwide on cancer immunotherapy by administration of CAR-T cells, more specifically, therapy which involves collecting T cells from patients, and introducing a gene encoding CAR to these T cells, which are then cultured and expanded, and transferred to the patients again (NPL 1). Results showing that cancer immunotherapy by administration of CAR-T cells shows efficacy on, for example, hematopoietic malignant tumor such as leukemia or lymphoma have been obtained. In 2017, Kymriah (registered trademark) (Novartis, tisagenlecleucel, CTL-019, CD3 zeta-CD137) and Yescarta (registered trademark) (KiTE, axicabtagene ciloleucel, CD3 zeta-CD28), which are CAR-T against CD19 as an antigen, were approved as pharmaceuticals in the USA.

CRS and cytokine release resulting from cancer immunotherapy by administration of CAR-T cells have been reported (Lee DW, et al. Current concepts in the diagnosis and management of cytokine release syndrome. Blood. 2014 Jul 10; 124(2): 188-95). Since it is suggested that the same CRS mechanism is initiated when a pharmaceutical composition comprising an anti-T cell antigen-binding molecule is administered and when CAR-T cells are administered, it is understood that administration of a VEGF inhibitor can prevent, alleviate, or treat cytokine release syndrome (CRS) associated with administration of CAR-T cells.

It should be noted that all prior art documents cited herein are incorporated herein by reference.

### [Example]

### [Example 1]

### Effect of combined use of the anti-mouse VEGFR2 antibody (DC101) on the drug efficacy of the ERY974 surrogate antibody (GC33/2C11)

A test using syngeneic mouse models to evaluate the effect of combined use of the anti-mouse VEGFR2 antibody DC101 (manufactured by Bio X Cell (US)) on the drug efficacy of the ERY974 surrogate antibody was carried out as follows. GC33/2C11 (an antibody comprising the amino acid sequence of SEQ ID NO: 433 as the GPC3-side heavy chain, the amino acid sequence of SEQ ID NO: 434 as the GPC3-side light chain, the amino acid sequence of SEQ ID NO: 435 as the CD3-side heavy chain, and the amino acid sequence of SEQ ID NO: 436 as the CD3-side light chain) was used as the ERY974 surrogate antibody.

MC38/hGPC3 cancer cells, which are mouse cancer cell line MC38 made to highly express human GPC3, were transplanted subcutaneously in C57BL/6J mice. The day of transplantation was set to day 0. On day 14, the mice were divided into four groups according to tumor size. The groups of mice were subjected to administration of (1) the vehicle, (2) GC33/2C11 (5 mg/kg), (3) the anti-mouse VEGFR2 antibody (DC101) (10 mg/kg), or (4) GC33/2C11 (5 mg/kg) and the anti-mouse VEGFR2 antibody (10 mg/kg), respectively, into the tail vein on day 14 and on day 18.

As a result, the combined use of GC33/2C1 and the anti-mouse VEGFR2 antibody enhanced the drug efficacy compared to when each antibody was used as a single drug (Fig. 1).

### [Example 2]

### Effect of combined use of the anti-mouse VEGFR2 antibody (DC101) on tumor-derived cytokine production resulting from the ERY974 surrogate antibody (GC33/2C11)

A test using syngeneic mouse models to evaluate the effect of combined use of the anti-mouse VEGFR2 antibody (DC101) on tumor tissue-derived cytokine production resulting from the ERY974 surrogate antibody was carried out as follows.

MC38/hGPC3 cancer cells, which are mouse cancer cell line MC38 made to highly express human GPC3, were transplanted subcutaneously in C57BL/6J mice. The day of transplantation was set to day 0.

On day 13, the mice were divided into four groups according to tumor size. The groups of mice were subjected to administration of (1) the vehicle, (2) GC33/2C11 (5 mg/kg), the anti-mouse VEGFR2 antibody (10 mg/kg), or (4) GC33/2C11 (5 mg/kg) and the anti-mouse VEGFR2 antibody (10 mg/kg) into the tail vein on day 13. Blood was collected from the mice before, and 2 hours, 6 hours, and 24 hours after the antibody administration, plasma components were recovered, and cytokines in the plasma were measured. For the cytokine measurements, a Mouse Cytokine/Chemokine Magnetic Bead Panel 1 (Millipore) reagent was used, and measurements were taken using the MAGPIX^{®} xPONT 4.2 system (Merck).

As a result, the amount of production of tumor-derived cytokines tended to be decreased in the group using GC33/2C11 and the anti-mouse VEGFR2 antibody in combination, compared to the group in which GC33/2C11 alone was administered. Therefore, this suggested that use of an angiogenesis inhibitor in combination decreases the amount of tumor-derived cytokine production due to ERY974 (Figs. 2A to 2E).

### [Example 3]

### Effect of combined use of the anti-mouse VEGFR2 antibody (DC101) on normal tissue-derived cytokine production resulting from the ERY974 surrogate antibody (GC33/2C11)

A test using human GPC3 knock-in mice, to evaluate the effect of the combined use of the anti-mouse VEGFR2 antibody (DC101) on normal tissue-derived cytokine production resulting from the ERY974 surrogate antibody (GC33/2C11) was carried out as follows.

Human GPC3-expressing human GPC3 knock-in mice express human GPC3 under expression control by the mouse GPC3 promoter (WO2018/038046). Meanwhile, mice and humans have been reported to have common GPC3-positive tissues. Observation of slight expression of GPC3 in the lungs is common to both humans and mice (Clin. Cancer. Res (2004) 10, 8630-40). Therefore, administering GC33/2C11 to the human GPC3 knock-in mice described above and measuring the amount of cytokine produced will enable prediction and evaluation of the mechanism of normal tissue-derived cytokine production when ERY974 is administered to humans.

The human GPC3-expressing human GPC3 knock-in mice were divided into four groups, and they were subjected to administration into the tail vein of (1) the vehicle, (2) GC33/2C11 (5 mg/kg), (3) the anti-mouse VEGFR2 antibody (10 mg/kg), or (4) GC33/2C11 (5 mg/kg) and the anti-mouse VEGFR2 antibody (10 mg/kg). Blood was collected from the mice before and 2 hours after the antibody administration, plasma components were recovered, and cytokines in the plasma were measured. For the cytokine measurements, a Mouse Cytokine/Chemokine Magnetic Bead Panel 1 (Millipore) reagent was used, and measurements were taken using a MAGPIX^{®} xPONT 4.2 system (Merck).

As a result, the amount of production of normal tissue-derived cytokines tended to be decreased in the group using GC33/2C11 and the anti-mouse VEGFR2 antibody in combination, compared to the group in which GC33/2C11 alone was administered. Therefore, this suggested that use of an angiogenesis inhibitor in combination decreases the amount of normal tissue-derived cytokine production due to ERY974 (Fig. 3).

### [Industrial Applicability]

The present disclosure is useful for preventing, alleviating, or treating adverse reactions caused by cytokine production associated with administration of anti-T cell antigen-binding molecules and the like. Anti-T cell antigen-binding molecules are drawing attention as means for treating cancer. Therefore, in some embodiments, the present disclosure is useful in treatment of cancer with anti-T cell antigen-binding molecules.

## Claims

1. A pharmaceutical composition comprising an anti-T cell antigen-binding molecule, wherein its use in combination with a vascular epithelial cell growth factor (VEGF) inhibitor prevents and/or alleviates and/or treats cytokine release syndrome and/or cytokine release.

2. The pharmaceutical composition of claim 1, wherein the VEGF inhibitor is administered before or simultaneously with administration of the pharmaceutical composition.

3. The pharmaceutical composition of claim 1 or 2, wherein the VEGF inhibitor is administered 6 days, 5 days, 4 days, 3 days, 2 days, or 1 day before the administration of the pharmaceutical composition, or on the same day as but before administration of the pharmaceutical composition.

4. The pharmaceutical composition of any one of claims 1 to 3, wherein the VEGF inhibitor is selected from the group consisting of an anti-VEGF antigen-binding molecule, an anti-VEGFR1 antigen-binding molecule, an anti-VEGFR2 antigen-binding molecule, a fusion protein comprising a VEGF receptor or a fragment thereof, and a tyrosine kinase inhibitor.

5. The pharmaceutical composition of any one of claims 1 to 4, wherein the VEGF inhibitor is selected from the group consisting of Bevacizumab, Ramucirumab, and Aflibercept.

6. The pharmaceutical composition of any one of claims 1 to 5, wherein a corticosteroid is not administered before or simultaneously with the administration of the pharmaceutical composition.

7. The pharmaceutical composition of any one of claims 1 to 5, wherein a corticosteroid is further administered before, simultaneously with, or after the administration of the pharmaceutical composition.

8. The pharmaceutical composition of claim 6 or 7, wherein the corticosteroid is dexamethasone, a pharmaceutically acceptable salt thereof, or a derivative thereof.

9. The pharmaceutical composition of any one of claims 1 to 8, wherein the anti-T cell antigen-binding molecule is a bispecific antigen-binding molecule comprising:
(1) a domain comprising an antibody variable region having T cell receptor complex-binding activity; and
(2) a domain comprising an antibody variable region having cancer antigen-binding activity.

10. The pharmaceutical composition of any one of claims 1 to 9, wherein the anti-T cell antigen-binding molecule is a bispecific antibody comprising:
(1) a domain comprising an antibody variable region having T cell receptor complex-binding activity;
(2) a domain comprising an antibody variable region having glypican 3-binding activity; and
(3) a domain comprising an Fc region with reduced binding activity towards an Fcγ receptor.

11. The pharmaceutical composition of any one of claims 1 to 10, which is for treatment of cancer.

12. The pharmaceutical composition of claim 11, wherein the antitumor effect is enhanced by the combined use as compared to when the pharmaceutical composition or the VEGF inhibitor is used as a single drug.

13. The pharmaceutical composition of any one of claims 1 to 11, wherein the cytokine release syndrome is caused by cytokine release from either or both of a non-tumor tissue and a tumor tissue.

14. The pharmaceutical composition of claim 13, wherein the tumor tissue comprises GPC3-expressing cells.

15. A VEGF inhibitor for preventing and/or alleviating and/or treating cytokine release syndrome and/or cytokine release.
